# EUROPEAN PATENT SPECIFICATION

(11) **EP 4 045 493 B1**
(45) Date of publication and mention of the grant of the patent: **13.08.2025**
(21) Application number: 20792421.8
(22) Date of filing: 16.10.2020
(51) Int. Cl.: C07D 401/14, A61K 31/4545, A61P 35/00

(54) **COMPOUNDS FOR TARGETED DEGRADATION OF CARRIER PROTEINS AND USES THEREOF**
VERBINDUNGEN FÜR DEN GEZIELTEN ABBAU VON TRÄGERPROTEINEN UND VERWENDUNGEN DAVON
COMPOSÉS POUR LA DÉGRADATION CIBLÉE DE PROTÉINES PORTEUSES ET LEURS UTILISATIONS

(30) Priority: 16.10.2019 EP 19203700
(43) Date of publication of application: 24.08.2022
(73) Proprietor: CeMM - Forschungszentrum für Molekulare Medizin GmbH, 1090 Wien (AT)
(72) Inventor: BENSIMON, Ariel, 1190 Wien (AT); WINTER, Georg, 1140 Wien (AT); SUPERTI-FURGA, Gulio, 1070 Wien (AT)
(74) Representative: Vossius & Partner Patentanwälte Rechtsanwälte mbB
(86) International application number: PCT/EP2020/079281
(87) International publication number: WO 2021/074429

(56) References cited:
- DANIEL P BONDESON ET AL: "Catalytic in vivo protein knockdown by small-molecule PROTACs", NATURE CHEMICAL BIOLOGY, vol. 11, no. 8, 10 June 2015 (2015-06-10), Basingstoke, pages 611 - 617, XP055279063, ISSN: 1552-4450, DOI: 10.1038/nchembio.1858
- ROSIE A. JOHNSTON ET AL: "Selective Inhibition of Human Solute Carrier Transporters by Multikinase Inhibitors", DRUG METABOLISM AND DISPOSITION, vol. 42, no. 11, 27 August 2014 (2014-08-27), US, pages 1851 - 1857, XP055693799, ISSN: 0090-9556, DOI: 10.1124/dmd.114.059097
- S. F. PEDERSEN ET AL: "The SLC9A-C Mammalian Na + /H + Exchanger Family: Molecules, Mechanisms, and Physiology", PHYSIOLOGICAL REVIEWS., vol. 99, no. 4, 1 October 2019 (2019-10-01), US, pages 2015 - 2113, XP055693807, ISSN: 0031-9333, DOI: 10.1152/physrev.00028.2018

## Description

### FIELD OF THE INVENTION

The present invention relates to compounds of formula (I) with the ability to modulate, in particular to induce ubiquitination of a transmembrane protein/transmembrane proteins, such as SLCs/solute carrier proteins. Particularly, the present invention relates to compounds with the ability to induce degradation of SLCs/solute carrier proteins via the ubiquitin-proteasome pathway. The invention also relates to the compounds and composition for use as medicaments as well as pharmaceutical compositions comprising these compounds, particularly for use as degraders of transmembrane proteins, such as SLCs/solute carrier proteins. Furthermore, the present invention relates to the compounds of the present invention for use intreating a disease or condition, such as cancer, wherein the compound(s) of the present invention is/are to be administered.

### BACKGROUND OF THE INVENTION

Several families of transmembrane transporters including solute carrier (SLC) proteins, ion channels, water channels and ATP-driven pumps, enable the exchange of nutrients, ions and metabolic products across cellular membranes (Hediger, M. A., Clémençon, B., Burrier, R. E. & Bruford, E. A. The ABCs of membrane transporters in health and disease (SLC series): Introduction. Molecular Aspects of Medicine 34, 95-107 (2013)). The more than 450 members of the solute carrier (SLC) proteins represent the largest class of transmembrane transporters encoded in the human genome (Lin, L. Nature Publishing Group 14, 543-560 (2015)). Their several-pass transmembrane domain structure and hydrophobicity contributed to the orphan status of many SLCs, devoid of both natural cargos and functional inhibitors. 65 families are identified on the basis of sequence similarities. For example, amino acid accumulation is mediated by members of the SLC1, SLC3/7, SLC6, SLC15, SLC16, SLC17, SLC32, SLC36, SLC38 and SLC43. Further members of the SLC superfamily, such as the SLC9 family of sodium/hydrogen exchangers, regulate ion fluxes at the plasma membrane, or solute transport into and out of cellular organelles. In particular, sodium/hydrogen exchangers or sodium/proton antiports are a family of transporters that maintain cellular pH by utilising the sodium gradient across the membrane, such as the plasma membrane, to extrude protons produced by metabolism, in a stoichiometry of 1 Na⁺ (in): 1 H⁺ (out). For example, NHE1 is (also known as SLC9A1) is an electroneutral and reversible ion transporter that exchanges one Na⁺ ion for one H⁺ ion, contributing to both cytoplasmic alkalinization, and acidification of the microenvironment (Parks et al. Nature Reviews Cancer 13, 611-623 (2013)).

NHE3, which is also known as SLC9A3, is highly expressed in the intestine and kidneys and regulate sodium movements in those tissues. NHE1, also denoted SLC9A1 is considered to be a ubiquitously-expressed transporter. NHE3 is highly expressed in the intestine and kidneys and regulate sodium movements in those tissues.

In recent years, evidence of direct roles for some SLCs in key tumorigenic processes has been accumulating, demonstrating that SLCs may be therapeutically attractive for targeted drug development in cancer (El-gebali, S., Bentz, S., Hediger, M. A. & Anderle, P. Molecular Aspects of Medicine Solute carriers ( SLCs ) in cancer. Molecular Aspects of Medicine 34, 719-734 (2013); Qing and Shu, Mol Cell Ther. 2, 15 (2014)). Moreover, SLC9A1 was a promising target in cardiovascular disease but clinical development of specific inhibitors was abandoned due to poor response and adverse side effects. Further, SLC9A1 has been investigated in a variety of cancer models, but most prominently in glioma and breast cancer ( Amith SR, Fong S, Baksh S, Fliegel L. Na (+)/H (+)exchange in the tumour microenvironment: does NHE1 drive breast cancer carcinogenesis?, Int J Dev Biol. 2015;59(7-9):367-77; Cong D, Zhu W, Kuo JS, Hu S, Sun D, Ion transporters in brain tumors, Curr Med Chem. 2015;22(10):1171-81).

Most of SLC-targeting small molecule development has been oriented towards inhibition (Lin, L., Yee, S. W., Kim, R. B. & Giacomini, K. M. SLC Transporters as Therapeutic Targets: Emerging Opportunities. Nat Rev Drug Discov 14, 543-560 (2015)). However, in this approach only the transport function is regulated, and it does not enable the proteostatic regulation of the transmembrane proteins such as SLCs (such as degradation) or the regulation of transport-independent functions of transmembrane proteins such as SLCs. Thus, novel paradigms in drug design are highly needed.

Several techniques developed in recent years to control the targeted degradation of specific proteins by a new generation of small-molecule degraders or "PROTAC^{™}" (proteolysis targeting chimeras) now enables the design of small-molecules that induce the selective and immediate degradation of target protein (Winter, G. E. et al. DRUG DEVELOPMENT. Phthalimide conjugation as a strategy for in vivo target protein degradation. Science 348, 1376-1381 (2015), Bondeson, D. P. et al. Catalytic in vivo protein knockdown by small-molecule PROTACs. Nat. Chem. Biol. 11, 611-617 (2015)). Johnston et al. reported on the selective inhibition of human solute carrier transporters by multikinase inhibitors" in Drug Metabolism and Disposition, 2014, 42(11,) pages 1851-1857. Pedersen et al. referred to the SLC9A-C mammalian Na+ /H+ exchanger family, molecules, mechanisms, and physiology in Physiological Reviews, 2019, 99(4), pages 2015-2113.

The terms "PROTAC^{®}", "PROTAC^{™}", "PROTAC", "PROTAC^{®}s'', "PROTAC^{™}s", "PROTACs" or "proteolysis targeting chimera" are used interchangeably and refer in particular to heterobifunctional compounds as illustrated in formula (I).

PROTACs operate by inducing molecular proximity between the protein of interest (POI) and a cellular E3 ligase substrate receptor by binding simultaneously to both proteins. This induced proximity leads to ubiquitination and proteasomal degradation of the POI. Of note, the modular design consisting of a warhead binding to the POI, a flexible linker, and a defined E3 ligase ligand renders PROTAC development very flexible. The list of proteins permissive to targeted degradation now contains a large number of protein kinases, including one instance of a single-pass transmembrane receptor tyrosine kinase. Some proteins with one (1) transmembrane region, like EGFR, HER2, c-Met, ALK and FLT-3 (Cell Chem Biol. 2018 Jan 18;25(1):67-77. The Advantages of Targeted Protein Degradation Over Inhibition: An RTK Case Study. Burslem GM, Smith BE, Lai AC, Jaime-Figueroa S, McQuaid DC, Bondeson DP, Toure M, Dong H, Qian Y, Wang J, Crew AP, Hines J, Crews CM. / Eur J Med Chem. 2018 May 10;151:304-314. Proteolysis Targeting Chimeras (PROTACs) of Anaplastic Lymphoma Kinase (ALK). Zhang C, Han XR, Yang X, Jiang B, Liu J, Xiong Y, Jin J. J Am Chem Soc. 2018 Dec 5;140(48):16428-16432/ Enhancing Antiproliferative Activity and Selectivity of a FLT-3 Inhibitor by Proteolysis Targeting Chimera Conversion.Burslem GM, Song J, Chen X, Hines J, Crews CM) have been shown to be degradable by "PROTAC" induced degradation. However, many proteins, such as SLCs/solute carrier transporters, may have more than ten (10) transmembrane regions which is challenging for the potential degradation of these proteins by PROTAC. Moreover, several of the main drug targets and disease-associated genes have a multi-pass transmembrane domain topology and are located at different subcellular locations. Prominent examples are G-protein-coupled receptors (GPCRs), ABC-transporters, such as the cystic fibrosis transmembrane conductance regulator (CFTR), and solute carrier transporter proteins (SLCs). Consequently, compounds that are able to effectively target such disease associated transmembrane proteins are not known.

Hence, in view of the above, the technical problem underlying the present invention is the provision of chemical compounds which can induce degradation of transmembrane proteins, such as multi-pass transmembrane domain proteins.

The solution to this technical problem is provided by the embodiments as defined herein below and as characterized in the claims. In the context of the present invention novel compounds are provided that alter protein function or protein abundance (for instance via induced targeted protein degradation) by altering the function of or binding to ubiquitin E3 ligases, including ligases of the cullin RING E3 ligase family.

In this context, transmembrane proteins, particularly solute carriers, are increasingly attracting the interest of chemical biologists and drug discoverers as they are involved in a variety of diseases and there are some prominent drug targets. Thus, the invention described herein represents a breakthrough in terms of chemical strategy, in particular because it has been shown that the degradation machinery required for proteolysis, such as, inter alia, ligand induced proteolysis and/or proteolysis induced by binding of a moiety of a compound to a transmembrane protein, is accessible to transmembrane proteins such as solute carriers.

The invention is as defined in the claims.

### DESCRIPTION OF THE INVENTION

Described herein is a compound having the following formula (I):

Transmembrane Protein
Binding Moiety
(TMPBM) | Linker (L) | E3 Ligase Binding Moiety
(EBM)

In formula (I), TMPBM is a moiety binding to a transmembrane protein, in particular to a solute carrier (SLC) protein, L is a linker moiety; and EBM is a moiety modifying the function of the E3 ligase and/or binding to at least one member of the E3 ligase complex. TMPBM represents a moiety having the partial formula (II) shown below, including any stereoisomers, tautomers, pharmaceutically acceptable salts and, solvates and prodrugs thereof.

The compounds of formula (I) and other compounds disclosed herein are highly potent PROTACs for the degradation of transmembrane proteins, such as SLCs. By the use of these compounds in particular multi-pass transmembrane proteins of different topology and structural folds are amenable to the proximity-induced proteolysis. This is an important breakthrough with manifold opportunities for the treatment of a wide variety of diseases.

In the following, examples of compounds of formula (I) are presented. It is to be understood that these also encompass any stereoisomers, tautomers, pharmaceutically acceptable salts and solvates (in particular stereoisomers, pharmaceutically acceptable salts and solvates) of the compounds presented as Markush formulae or specific formulae. It is to be understood, that this also applies to any partial structures of compound of formula (I), such as partial formula (II), and partial formula (III).

TMPBM represents a moiety having the following partial formula (II): 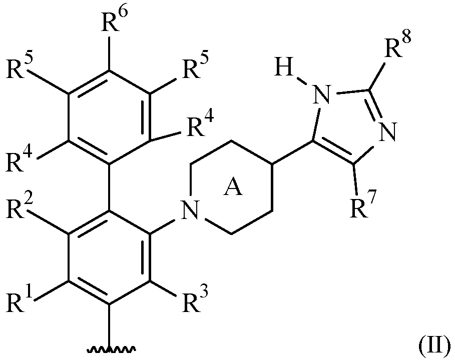 including any stereoisomers, tautomers, pharmaceutically acceptable salts and solvates thereof.

In Formula (II), 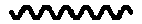 indicates the position of attachment of the partial formula (II) to the linker (L).

R¹, R², R³, R⁴, R⁵, R⁶, R⁷ and R⁸ in Formula (II) are each independently selected from -H, - halogen, -NO₂, -CN, -C₁₋₃ alkyl, -OH, -O-C₁₋₂ alkyl, NH₂, -NH(C₁₋₂ alkyl), -N(C₁₋₂ alkyl)₂, -CHO, -CO(C₁₋₂ alkyl), COOH, COO(C₁₋₂ alkyl), CONH₂, CONH(C₁₋₂ alkyl), and CON(-C₁₋₂ alkyl)₂, wherein each alkyl is optionally substituted with one or more F.

In addition, Ring A in formula (II) is optionally substituted with one or more F.

It is more preferred that in the moiety of partial formula (II) R¹, R², R³ and each R⁴ are each hydrogen, R⁷ is methyl, R⁸ is H or NH₂, and Ring A is not substituted with any F.

In addition, it is preferred that in the moiety of partial formula (II) one R⁵ is selected from H, F, Cl, Me, CF₃, CF₂H and CH₂F; while the other R⁵ is H, and R⁶ is F or Cl.

A particularly preferred example of the moiety of partial formula (II) has the following formula (IIa):

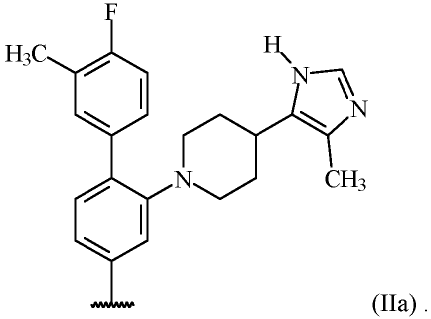

The present inventors have found that in particular compounds of formula (I) in which the linker has a certain length, such as about 14 to about 24 atoms in length, show highly surprisingly good suitability as PROTACs for the degradation of transmembrane proteins, such as SLCs.

In the compounds according to the present invention, L is preferably selected from C₁₄₋₂₄ alkylene, C₁₄₋₂₄ alkenylene, and C₁₄₋₂₄ alkynylene, wherein said alkylene, said alkenylene and said alkynylene are each optionally substituted with one or more groups independently selected from halogen, C₁₋₅ haloalkyl, -O(C₁₋₅ haloalkyl), -CN, -OR²¹, -NR²¹R²¹, -NR²¹OR²¹, -COR²¹, -COOR²¹, -OCOR²¹, -CONR²¹R²¹, -N R²¹COR²¹, -NR²¹COOR²¹, -OCONR²¹R²¹, -SR²¹, -SOR²¹, -SO₂R²¹, -SO₂NR²¹R²¹, -NR²¹SO₂R ²¹, -SO₃R²¹, and -NO₂, and further wherein one or more -CH₂- units comprised in said alkylene, said alkenylene or said alkynylene are each optionally replaced by a group independently selected from -O-, -NR²¹-, -CO-, -S-, -SO-, and -SO₂-;
each R²¹ is independently selected from hydrogen, C₁₋₅ alkyl, C₂₋₅ alkenyl, C₂₋₅ alkynyl, carbocyclyl, and heterocyclyl, wherein said alkyl, said alkenyl and said alkynyl are each optionally substituted with one or more groups R^{Alk}, and further wherein said carbocyclyl and said heterocyclyl are each optionally substituted with one or more groups R^{Cyc};
any two R²¹ are optionally linked to form a ring;
each R^{Alk} is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl);
each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl).

In L, the C₁₄₋₂₄ alkylene, C₁₄₋₂₄ alkenylene, and C₁₄₋₂₄ alkynylene are preferably C₁₅₋₂₂ alkylene, C₁₅₋₂₂ alkenylene, and C₁₅₋₂₂ alkynylene, respectively, preferably C₁₆₋₂₀ alkylene, C₁₆₋₂₀ alkenylene, and C₁₆₋₂₀ alkynylene respectively wherein each of the alkylenes, alkenylenes and alkynylenes may be modified as set out above. It is further preferred that L is selected from C₁₄₋₂₄ alkylene and C₁₄₋₂₄ alkenylene, more preferably C₁₅₋₂₂ alkylene, C₁₅₋₂₂ alkenylene, even more preferably C₁₆₋₂₀ alkylene, still more preferably C₁₇₋₁₉ alkylene, most preferably C₁₇ alkylene, wherein each of the alkylenes and alkenylenes may be modified as set out above.

Particularly preferred examples of L include one of the following structures:

-O-L¹-NH-C(=O)- and -NH-L¹-NH-C(=O)-,

preferably

-NH-L¹-NH-C(=O)-

-wherein L¹ is selected from C₁₃₋₁₇ alkylene and C₁₃₋₁₇ alkenylene, wherein said alkylene and said alkenylene are each optionally substituted with one or more groups independently selected from halogen, C₁₋₃ alkyl and C₁₋₃ haloalkyl, and further wherein one or more -CH₂- units comprised in said alkylene or said alkenylene is/are replaced by a group independently selected from -O-, -NR²¹-, -CO-, -S-, -SO-, and -SO₂-.

It is preferred that L¹ is selected from C₁₃₋₁₇ alkylene, wherein said alkylene is optionally substituted with one or more groups independently selected from halogen, C₁₋₃ haloalkyl and C₁₋₃ haloalkyl, and further wherein one or more -CH₂- units comprised in said alkylene or said alkenylene is/are replaced by a group independently selected from -O-, -NR²¹- and -CO-.

Specific examples of L¹ include:

-CH₂-CH₂-O-CH₂-CH₂-O-CH₂-CH₂-O-CH₂-CH₂-O-CH₂-CH₂-,

and

-CH₂-C(=O)-NH-CH₂-CH₂-O-CH₂-CH₂-CH₂-O-CH₂-CH₂-CH₂-O-CH₂-CH₂-.

Specific examples of L include:

-NH-CH₂-CH₂-O-CH₂-CH₂-O-CH₂-CH₂-O-CH₂-CH₂-O-CH₂-CH₂-NH-C(=O)-,

and

-O-CH₂-C(=O)-NH-CH₂-CH₂-O-CH₂-CH₂-CH₂-O-CH₂-CH₂-CH₂-O-CH₂-CH₂-NH-C(=O)-.

More preferred is that the -O- or -NH- on the left hand side of the above examples of L is bound to EBM as defined herein and the NH-C(=O)- on the right hand side is bound to the TMPBM as defined herein.

EBM is preferably a moiety binding to at least one member of the E3 ligase complex. EBM in the compound of formula (I) is more preferably a moiety binding to at least one member of the E3 ligase complex with a Kd of 10 µM or less, as determined by the assay set out in the present application.

In particular, the binding of the EBM as disclosed herein and used in context of the invention to at least one member of the E3 ligase complex, such as the substrate receptor CRBN, can be measured by using assays based on the NanoBRET^{™} technology, according to the manufacturer's protocol and recommendations (Promega; NanoBRET^{™} assay).

Likewise, the interaction between a transmembrane protein, such as SLC9A1, as disclosed herein and used in context of the invention and at least one member of the E3 ligase complex, such as the substrate receptor CRBN, can be measured by using assays based on the NanoBRET^{™} technology, according to the manufacturer's protocol and recommendations (Promega; NanoBRET^{™} assay).

Likewise, the interaction between a compound, such as a PROTAC as disclosed herein and used in context of the invention, and at least one member of the E3 ligase complex, such as the substrate receptor CRBN, can be measured by using assays based on the NanoBRET^{™} technology, according to the manufacturer's protocol and recommendations (Promega; NanoBRET^{™} assay).

Thereby, the NanoBRET^{™} assay allows measuring proximity of an EBM, a compound, such as a PROTAC or a transmembrane protein, to at least one member of the E3 ligase complex, such as a substrate receptor, like CRBN or VHL, by determining the energy transfer from a luminescent donor to a fluorescent acceptor (see, e.g., Riching et al. ACS Chem. Biol. 2018, 13, 2758-2770). In this context, the ternary complex formation of a compound comprising an EBM, such as a PROTAC, an E3 ligase member, such as a substrate receptor, and a target protein, such as a transmembrane protein/transmembrane proteins, can be measured.

For the expression of a donor in HEK293 cells, a transmembrane protein can be cloned into a plasmid comprising a luciferase, such as NanoLuc^{®} luciferase. For example, the transmembrane protein, such as SLC9A1, can be cloned into the NanoLuc donor plasmid (Promega; NanoLuc^{®} luciferase) at the C-terminus. Alternatively, a transmembrane protein used as a donor on this assay can also be tagged at another position and/or site, for example on the N-terminus or a loop of the transmembrane protein that is directed to the cytoplasm. The person skilled in the art knows which positions and sites can be used for tagging a transmembrane protein and is aware which transmembrane proteins are feasible to be tagged at a particular position or site. The donor may alternatively be tagged, for example with HiBiT using CRISPR-Cas9. As another alternative, the assay can be adapted to become a TR-FRET assay by changing NanoLuc to a tag that enables labeling with fluorophore (for example but not limited to SNAP-tag, followed by Tb3+ cryptate labelling). Therefore, it can be generalized that the nanoBRET assay can also be transferred to TR-FRET. The donor may also comprise a compound comprising an EBM, such as a PROTAC, as disclosed herein and used in context of the invention.

For expression of an acceptor in HEK293 cells, a HaloTag can be employed that is attached to a E3 ligase member, such as the substrate receptor CRBN (Promega; HaloTag^{®}). Alternatively, any other tag can be used that enables labeling with an acceptor fluorophore. Examples of such tags include but are not limited to SNAPtag, CLIPtag, biotin and streptavidin. The donor and/or acceptor can be transiently or stably expressed in these cells.

For example, and according to the manufacturer's protocol and recommendations (Promega; NanoBRET^{™} assay), HEK293 cells are seeded in 6-well plates, at 800k cells per well. The two plasmids expressing the acceptor and donor are transiently transfected in HEK293 at a ratio 1: 1, using FuGene. The next day, the transfected cells are trypsinized, washed, mixed with nanoBRET 618 ligand and re-seeded in a 96-well white plate. The next day, cells are treated with 10uM MG132 for 1 hour and then treated for 6 hours with 7 doses of a compound, such as the compound denoted as PROTAC d9A1-2 (in uM:0.000256, 0.00128, 0.0064, 0.032, 0.16, 0.8, 4, 20). NanoBRET Nano-Glo Substrate are prepared in Opti-MEM, and dispensed (50ul) in each well, followed by 30sec shaking in the plate reader (Molecular Devices). Donor emission (460nm) and acceptor emission (618nm) are measured within 10 minutes of substrate addition on the plate reader (Molecular Devices). The BRET ratio is calculated as the ratio between acceptor emission and donor emission. The raw BRET ratio is normalized to maximum, from which the Kd value are derived.

In view of the above, using assays based on the NanoBRET^{™} technology, the interaction between transmembrane proteins, such as SLCs, or a compound comprising an EBM, such as a PROTAC, as disclosed herein and in context of the invention and a member of the E3 ligase, such as the substrate receptor of the E3 ligase, such as CRBN or VHL, can be measured. In this context, the Kd values that can be determined by applying this technology comprise Kd values of a transmembrane protein, such as a SLC as provided herein, or a compound comprising an EBM, such as a PROTAC, as disclosed herein and in context of the invention and at least one member of the E3 ligase, such as a substrate receptor. Determination of the Kd by using this assay can also be employed to SLCs from the SLC9 family or other families.

Preferably, EBM is a moiety of partial formula (III): 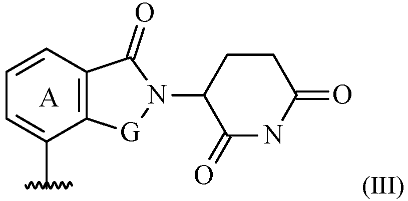 including any stereoisomers, tautomers, pharmaceutically acceptable salts and solvates thereof, in particular stereoisomers, pharmaceutically acceptable salts and solvates thereof, wherein 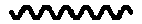 indicates the position of attachment of the partial formula (III) to the linker (L).

G is selected from -C(=O)- and CH₂. Preferably, G is -C(=O)-.

Ring A is optionally substituted with one or more F. More preferably, Ring A is not substituted with one or more F.

It is to be understood that partial compounds of formula (III) may be present in the form of the following two enantiomers:

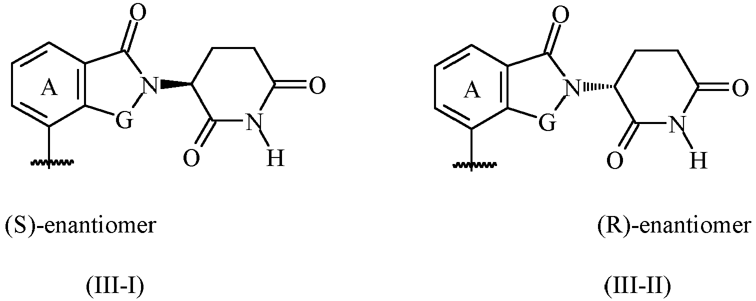

It is preferred that the partial compounds of formula (III) are selected from partial compounds of formula (III-I), in other words, the (S) enantiomer is preferred.

Particularly preferred examples of the compounds of formula (I) include the following: 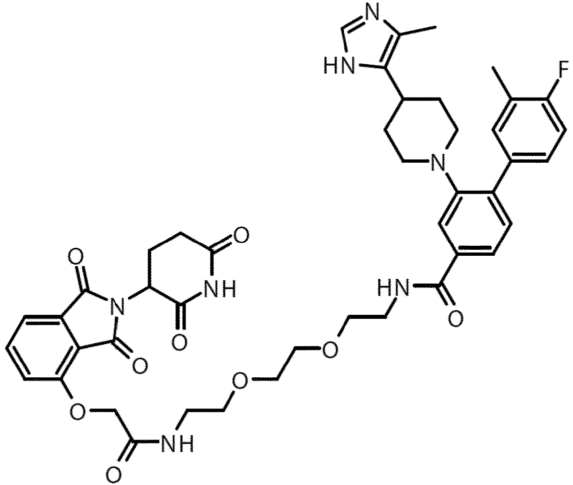 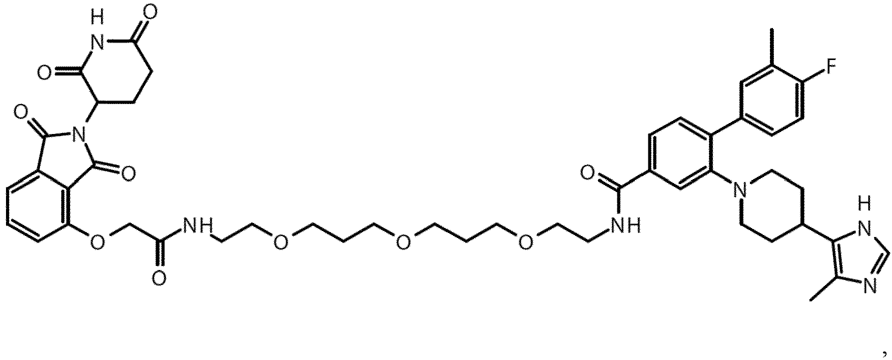 and

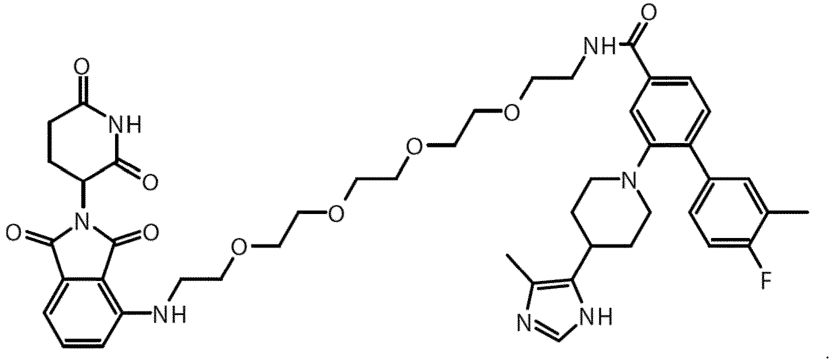

In each of these, the left-hand side part of the molecule preferably has an (S) stereochemistry in the part corresponding to the partial compound of formula (III). It is to be understood that these also encompass any stereoisomers, tautomers, pharmaceutically acceptable salts and solvates of the compounds presented above.

Until now, compounds capable of degrading membrane proteins with several transmembrane regions via degradation by the proteasome system have not been available. The inventors identified compounds with the ability to degrade membrane proteins with several transmembrane regions by proteasome mediated degradation. Thereby, it was shown in the appended Examples that SLCs, particularly from the SLC9 family such as SLC9A1 which has twelve transmembrane regions, are amenable to proteasome mediated degradation. As shown in the appended Examples, SLCs have been ectopically expressed as proteins tagged with a mutated FKBP domain, which made these SLCs amenable to degradation by specific degrader molecules (e.g. dTAG7/dTAG13) published recently (Nabet, B. et al.. Nat Chem Biol 14, 431-441 (2018). To test targeted degradation of endogenous SLCs, heterobifunctional molecules, also denoted PROTACs were designed in which the chemical structure of warhead binding an SLC of interest and the linker connecting this warhead to the thalidomide derivative were varied. It was found that such compounds, for example, those binding SLC9A1, led to efficient degradation of the target

Furthermore, as shown in the appended Examples and as a proof of concept, various human SLCs, localized to different subcellular membrane locations (cell surface, endoplasmic reticulum, golgi and lysosome), are amenable to degradation by heterobifunctional ligands when fused to an appropriate degradation domain, for example when the chromosomal locus is engineered to express a fusion protein of the SLC with the degradation-mediating tag. Yet, the compounds of the invention are particularly useful in therapy and/or in the pharmaceutical field since the development/design of an additional degradation tag is not necessary for degradation (as illustrated in the appended Examples). Accordingly, degradation of endogenous SLCs modulated/induced by the compounds of the invention can bind to SLCs per se and do not, like the above described known compounds such as dTAG7 or dTAG13, only bind indirectly to SLC via a tag. As illustrated in the appended Examples, this is particularly advantageous for compounds used in therapeutics since compounds of this invention can degrade SLCs in cell(s) without the need of applying any modification(s) and/or manipulation(s) to said cell(s), such as by inserting a tag to the SLC.

It was further shown that the compound of the invention can lead to the degradation of an SLC protein. Moreover, as documented in the appended examples, the compounds of the present invention are efficiently able to engage and degrade endogenous members of the SLC9 family, a family of sodium/proton exchangers.

Transmembrane proteins with several transmembrane domains, such as SLCs, are attracting the attention of the scientific community but there are few tools to study their function. Particularly, SLCs are increasingly attracting the interest of chemical biologists and drug discoverers as they are involved in a variety of diseases and there are some prominent drug targets (SSRIs, glifozins).

Hence, the inventors found for the first-time compounds of new molecular entity targeting multi-pass transmembrane domain proteins. Since the inventors identified compounds which have the ability to degrade multi-pass transmembrane proteins and since several drug targets are multi-pass transmembrane proteins, such as SLCs, the newly identified compounds of the as disclosed herein represent valuable candidate drugs for such targets such as drugs for treating cancer, metabolic, inflammatory or cardiological diseases. Such compounds may further be able to bind to more than one transmembrane protein, in particular more than one multi-pass transmembrane domain protein. For example, the compound as disclosed herein and in context of the invention may bind to one or more transmembrane proteins of the SLC9A family. For example, the compound as disclosed herein and in context of the invention may bind to at least 2, preferably to at least 5, more preferably to at least 10 transmembrane proteins such as from the SLC9A family.

Further, the compounds as disclosed herein and in context of the invention may be able to target transmembrane proteins within specific tissues or within specific locations of the cells, such as the cell surface, the lysosome or the endoplasmatic reticulum, or any vesicle en route. Thus, documenended Examples, the present invention provides for compounds which function to recruit transmembrane proteins to an E3 ubiquitin ligase, thereby degrading transmembrane proteins in a tissue-specific, cell-specific and/or intracellular-specific manner. This, in turn, is particularly useful in therapeutics and/or in the pharmaceutical field, wherein transmembrane proteins of disease-associated tissue(s), cell(s) and/or can specifically be degraded by the compounds of the present invention.

Accordingly, and disclosed herein, the compounds comprise a E3 ligase binding moiety/ EBM, a linker/L and a transmembrane protein binding moiety/TMPBM and are able of stimulating/inducing ubiquitination of a target protein/target proteins, e.g. via degradation of a transmembrane protein/ transmembrane proteins by the ubiquitination system. The terms "E3 ligase binding moiety" and "EBM" or are used interchangeably and means that the E3 ligase binding moiety/ EBM is moiety modifying the function of the E3 ligase and/or binding to at least one regulator or member of the E3 ligase complex/cullin-RING ubiquitin ligase complex/CRL complex. "Modifying the function of the E3 ligase" as used in context of the invention means that the cullin-RING ubiquitin ligase activity/CRL activity is enhanced by the E3 ligase binding moiety/ EBM, for example by binding of the E3 ligase binding moiety/ EBM to the E3 ligase/cullin-RING ubiquitin ligase/CRL or by modifying the function of the E3 ligase complex/cullin-RING ubiquitin ligase complex/CRL complex. The term "cullin RING ubiquitin E3 ligase" or "CRL" are used interchangeably and refer to an ubiquitin ligase in a complex in which the catalytic core consists of a member of the cullin family and a RING domain protein; the core is associated with one or more additional proteins that confer substrate specificity. The RING domain proteins of the CRL mediate the transfer of ubiquitin from the E2 to the E3-bound substrate. In particular, the cullin RING ubiquitin E3 ligase (CRL) are modular multi-subunit complexes that all contain a common core comprising a cullin subunit and a zinc-binding RING domain subunit. In particular, the cullin subunit folds into an extended structure that forms the backbone of CRLs. The C-terminal region of the cullin subunit forms a globular domain that wraps itself around the RING protein, which in turn recruits the E2 conjugating enzyme to form the enzymatic core. The N-terminal region of the cullin subunit, which resides at the opposite end of the elongated cullin structure, recruits substrate receptors via adapter proteins.

In context of the invention, the compound has the capacity of stimulating/inducing ubiquitination of a transmembrane protein/transmembrane proteins by enhancing the cullin-RING ubiquitin ligase activity/CRL activity. As disclosed herein and in context of the invention, the E3 ligase binding moiety/EBM may be pomalidomide, thalidomide and lenalidomide. For examples, the EBM may be pomalidomide or thalidomide. As another example, the E3 ligase binding moiety/EBM may be pomalidomide. Particularly, the E3 ligase binding moiety/EBM may be pomalidomide (see, e.g., Winter, G. E. et al. DRUG DEVELOPMENT.. Science 348, 1376-1381 (2015)).

Said "enhanced cullin-RING ubiquitin ligase activity"/"enhanced CRL activity" means that said cullin-RING ubiquitin ligase activity/CRL activity is enhanced in the presence of the compound of the present invention compared to the cullin-RING ubiquitin ligase activity/CRL activity in the absence of said compound. Accordingly, the present invention relates to a compound with the capacity to induce and/or stimulate the ubiquitination of a transmembrane protein/ transmembrane proteins via enhancing the CRL activity. As disclosed herein and as illustrated in the appended Examples, said enhanced activity of the E3 ligase complex/cullin-RING ubiquitin ligase complex/CRL complex may be determined by the measurement of the level/amount of transmembrane protein/transmembrane proteins in a cell expressing the transmembrane protein/transmembrane proteins in the presence of the compound and compared to the level in the absence of said compound, wherein a decreased level of the transmembrane protein/transmembrane proteins in the presence of said compound compared to the level in the absence of said compound indicates the capacity of said compound to induce and/or stimulate the ubiquitination of a transmembrane protein/ transmembrane proteins, for example via enhancing the CRL activity.

The enhanced CRL activity may be induced by the presence of said compound. Said compound may be able to induce molecular proximity between a component of a E3 ligase complex/cullin-RING ubiquitin ligase complex/CRL complex and a transmembrane protein/transmembrane proteins which may be bound to the compound or which may be part of a ternary complex comprising the E3 ligase complex/cullin-RING ubiquitin ligase complex/CRL complex, the target protein/target proteins and the compound. Means and methods known in the art of how to determine the binding of the E3 ligase binding moiety/ EBM may to the E3 ligase complex/cullin-RING ubiquitin ligase complex/CRL complex comprise, inter alia, immunoassays (like Western blots, ELISA tests and the like) and/or reporter assay (like luciferase assays and the like). Such means and methods to determine the binding of a compound to the E3 ubiquitin ligase can be determined, for example, by immunoassays as for instance but not limited to radioimmunoassays, chemiluminescence- and fluorescence-immunoassays, Enzyme-linked immunoassays (ELISA), Luminex-based bead arrays, protein microarray assays, assays suitable for point-of-care testing and rapid test formats such as for instance immune-chromatographic strip tests. Suitable immunoassays may be selected from the group of immunoprecipitation, enzyme immunoassay (EIA)), enzyme-linked immunosorbenassays (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or Luminex ^{®} Assays. An immunoassay is a biochemical test that measures the presence or concentration of a macromolecule/polypeptide in a solution through the use of an antibody or immunoglobulin as a binding agent. In principle, all labeling techniques which can be applied in assays of said type can be used, such as labeling with radioisotopes, enzymes, fluorescence-, chemoluminescence- or bioluminescence labels and directly optically detectable color labels, such as gold atoms and dye particles.

The enhanced activity of the E3 ligase complex/cullin-RING ubiquitin ligase complex/CRL complex may also be determined by methods known in the art, such as surface plasmon resonance spectroscopy (SPR) or Förster resonance energy transfer (FRET), and methods as described herein and as illustrated in the appended examples. For example, such methods may include, but are not limited to FRET (Förster Resonance Energy Transfer) analysis. The theory of FRET (Förster Resonance Energy Transfer) defines a distance dependent, non-radiative transfer of energy from an excited donor (D) to an acceptor molecule (A). The relationship between easily accessible spectroscopic data and theoretical equations was the achievement of Theodor Förster, thereby enabling the possibility of many FRET applications in all kinds of natural sciences. FRET has been used in biochemical applications within the 1 to 10 nm scale (K. E. Sapsford et al., Angew. Chem. Int. Ed., 45, 4562, 2006) (e.g. protein-protein binding, protein folding, molecular interactions at and in cell membranes, DNA hybridization and sequencing, immunoreactions of antigens and antibodies). Details of the theory of FRET are well known. Further examples include protein complementation assay (PCA). Protein complementation assays (PCA) provide a means to detect the interaction of two biomolecules, e.g., polypeptides. PCA utilizes two fragments of the same protein, e.g., enzyme, that when brought into close proximity with each other can reconstitute into a functional, active protein. Further, binding of a compound to the E3 ubiquitin ligase may be detected, for example, in a Western Blot. Western blotting involves application of a protein sample (lysate) onto a polyacrylamide gel, subsequent separation of said complex mixture by electrophoresis, and transferal or "electro-blotting" of separated proteins onto a second matrix, generally a nitrocellulose or polyvinylidene fluoride (PVDF) membrane. Following the transfer, the membrane is "blocked" to prevent nonspecific binding of antibodies to the membrane surface. Many antibody labeling or tagging strategies are known to those skilled in the art. In the simplest protocols, the transferred proteins are incubated or complexed with a primary enzyme-labeled antibody that serves as a probe. After blocking non-specific binding sites a suitable substrate is added to complex with the enzyme, and together they react to form chromogenic, chemiluminescent, or fluorogenic detectable products that allow for visual, chemiluminescence, or fluorescence detection, respectively. This procedure is described by Gordon et al., U.S. Patent 4,452,901 issued June 15, 1984.

As is evident from the appended Examples, the compound of the present invention may bind a transmembrane protein/transmembrane proteins via the transmembrane protein binding moiety/TMPBM of the compound and bind or modify the function of the E3 ligase complex/cullin-RING ubiquitin ligase complex/CRL complex, for example by recruiting the transmembrane protein/transmembrane proteins bound to the transmembrane protein binding moiety/TMPBM of the compound to the E3 ligase complex/cullin-RING ubiquitin ligase complex/CRL complex. As another example, the compound in context of the invention may alter the function of a transmembrane protein/transmembrane proteins, for example by modifying posttranslational changes of a transmembrane protein/transmembrane proteins. A posttranslational modification may include but is not limited to the phosphorylation status of a transmembrane protein, e.g. a tyrosine kinase phosphorylating a transmembrane protein. Thus, the compound may induce ubiquitination of a transmembrane protein/transmembrane proteins, e.g., by modifying a transmembrane protein/transmembrane proteins in that the target protein becomes accessible for a E3 ligase complex/cullin-RING ubiquitin ligase complex/CRL complex, thereby the compound may not associate with a transmembrane protein/transmembrane proteins and/or E3 ligase complex/cullin-RING ubiquitin ligase complex/CRL complex.

As described herein and in context of the invention, the TMPBM as defined in claim 1 may bind to a transmembrane protein/transmembrane proteins. In one embodiment, the TMPBM as defined in claim 1 may bind to a SLC having between 3 and 17 transmembrane domains, such as between 3 and 14 transmembrane domains or 3 and 13 transmembrane domains, particularly between 6 and 14 transmembrane domains, more particularly between 8 and 14 transmembrane domains, even more particularly between 8 and 12 transmembrane domains. As used herein, the term "transmembrane protein" or "transmembrane protein(s)", as used herein, refers to a protein that is attached to or associated with a membrane of a cell or an organelle. They are often subdivided into several categories including integral membrane proteins, peripheral membrane proteins and lipid-anchored proteins. For example, the membrane protein is an integral membrane protein that is permanently bound to the lipid bilayer and which requires a detergent or another apolar solvent to be removed. Integral membrane proteins include transmembrane proteins that are permanently attached to the lipid membrane and span across the membrane one or several times. Examples of suitable membrane proteins include receptors such as GPCRs and growth factor receptors; transmembrane ion channels such as ligand-gated and voltage gated ion channels; transmembrane transporters such as neurotransmitter transporters; enzymes; carrier proteins; ion pumps; viral membrane proteins and supramolecular complexes thereof, amongst others.

Specific non-limiting examples of transmembrane proteins as used herein are provided further in the specification. For example, transmembrane proteins may function as gateways to permit transport of specific substances across the membrane. They may undergo conformational changes to move a substance through the membrane. Typically, the peptide sequence spanning the membrane or the transmembrane domain, is mainly hydrophobic. Membrane proteins are involved in signal transduction, mass transportation, energy production, formation of cytoskeleton etc. at cell membranes. In addition, the membrane protein is very important as a potential drug target. Non-limiting examples of such membrane transport proteins may be carriers and/or channels. Particularly non-limiting examples of such membrane transport proteins may be carriers. As used herein, the term "membrane transport proteins", "carrier" or "transporter" refers to transmembrane proteins which are embedded (singly or in complexes) in the cellular membrane (reviewed by Oh and Amidon (1999) in Membrane Transporters as Drug Targets, ed. Amidon and Sadee, Kluwer Academic/Plenum Publishers, New York, Chapter 1). There are two general classes of carriers/membrane transport proteins: channels or pores, and transporters (also known as carriers or permeases). Channels and transporters differ in their translocation mechanisms. Channels are hydrophilic group-lined protein tunnels whose opening by a regulatory event allow free, rapid passage of their charge-, size-, and geometry-selected small ions down their concentration gradients. Transporters specifically and selectively bind the molecules they move, some with and some against their concentration gradients, across membranes.

Carriers/membrane transporters are critical facilitators of the uptake (e.g. solute carrier family (SLC) transporters) and export (e.g. ABC transporters) of drugs. Such carriers/membrane transporters can alter drug disposition and distribution in several important ways. Many of these carriers/membrane transporters play key roles in pharmacology. As such, these carriers/membrane transporters play critical roles in mediating both the chemo-sensitivity and chemo-resistance of cancer cells to cancer chemotherapeutics. ABC transporters are frequently associated with decreased intracellular concentration of chemotherapeutic agents and acquired multi-drug resistance of tumor cells. SLC transporters, including anion, cation, nucleoside and amino acid transporters, are associated with increased sensitivity of tumor cells to chemotherapeutic agents since these transporters facilitate the cellular uptake of hydrophilic compounds. Carriers/membrane transporters can be classified as either passive or active transporters. The active transporters can be further divided into primary or secondary active transporters based on the process of energy coupling and facilitated transport. The ABC transporters are primary active transporters which export compounds against a chemical gradient driven by ATP and an inherent ATPase activity. The majority of passive transporters, which permit compounds to equilibrate along a concentration gradient, ion pumps, secondary active transporters and exchangers belong to the SLC transporter family. Transporters can move molecules by two types of processes. In one process, "facilitated diffusion" transporters move molecules with their concentration gradients. In the other process, "active transport," transporters move molecules against their concentration gradients. Active transport to move a molecule against its gradient requires energy. Primary active transporters, such as Na⁺/K⁺ ATPases or ABC transporters use energy from ATP hydrolysis or light, and establish ion gradients and membrane potential energy. Secondary active transporters, such as the H⁺/peptide transporter, use the pH or ion gradients established by primary active transporters to transport other molecules. In secondary active transport, the transporter uses two separate binding sites to move the primary ion down its concentration gradient to produce the energy to move the secondary solute against its gradient. The coupled solute either travels in the same direction as the primary solute (symport) or in the opposite direction (antiport).

The term "transport proteins" and "transport molecules" are used interchangeably and refer to transport proteins that are specific for a particular target solute or class of solutes, and are also present in one or more specific membranes. Transport molecules localized to the plasma membrane permit an exchange of solutes with the surrounding environment, while transport molecules localized to intracellular membranes (e.g., golgi apparatus, vesicles, membranes of the mitochondrion, peroxisome, lysosome, endoplasmic reticulum, nucleus, or vacuole) permit import and export of molecules from organelle to organelle or to the cytoplasm. For example, in the case of the mitochondrion, transporters in the inner and outer mitochondrial membranes permit the import of sugar molecules, calcium ions, and water (among other molecules) into the organelle and the export of newly synthesized ATP to the cytosol.

Transporters play important roles in the ability of the cell to regulate homeostasis, to grow and divide, and to communicate with other cells, e.g., to transport signaling molecules, such as hormones, reactive oxygen species, ions, neurotransmitters or vitamins. A wide variety of human diseases and disorders are associated with defects in transporter or other membrane transport molecules, including certain types of liver disorders (e.g., due to defects in transport of long-chain fatty acids (Al Odaib et al. (1998) New Eng. J. Med. 339:1752-1757), hyperlysinemia (mitochondrial lysine transport defect (Oyanagi et al. (1986) Inherit. Metab. Dis. 9:313-316)), and cataract (Wintour (1997) Clin. Exp. Pharmacol. Physiol. 24(1):1-9).

As described herein and in context of the invention, the TMPBM as defined in claim 1 may bind to a solute carrier (SLC) protein, for example but not limited to a TMPBM binding to a SLC selected from a SLC of the SLC family selected from the group consisting of SLC1 (high affinity glutamate and neutral amino acid transporter), SLC2 (facilitative GLUT transporter), SLC3 (heavy subunits of the heteromeric amino acid transporters), SLC4 (bicarbonate transporter), SLC5 (sodium glucose cotransporter), SLC6 (sodium- and chloride-dependent neurotransmitter transporter), SLC7 (cationic amino acid transporter/glycoprotein-associated amino-acid transporter), SLC8 (Na+/Ca2+ exchanger), SLC9 (Na+/ H+ exchanger), SLC10 (sodium bile salt cotransport), SLC11 (proton coupled metal ion transporter), SLC12 (electroneutral cation-Cl cotransporter), SLC13 (human Na+-sulfate/carboxylate cotransporter), SLC14 (urea transporter), SLC15 (proton oligopeptide cotransporter), SLC16 (monocarboxylate transporter), SLC17 (vesicular glutamate transporter), SLC18 (vesicular amine transporter), SLC19 (folate/thiamine transporter), SLC20 (type III Na+-phosphate cotransporter), SLC21/SLCO (organic anion transporting), SLC22 (organic cation/anion/zwitterion transporter), SLC23 (Na+-dependent ascorbic acid transporter), SLC24 (Na+/(Ca2+-K+) exchanger), SLC25 (mitochondrial carrier), SLC26 (multifunctional anion exchanger), SLC27 (fatty acid transport protein), SLC28 (Na+-coupled nucleoside transport), SLC29 (facilitative nucleoside transporter), SLC30 (zinc efflux), SLC31 (copper transporter), SLC32 (vesicular inhibitory amino acid transporter), SLC33 (Acetyl-CoA transporter), SLC34 (type II Na+-phosphate cotransporter), SLC35 (nucleoside-sugar transporter), SLC36 (proton-coupled amino acid transporter), SLC37 (sugar-phosphate/phosphate exchanger), SLC38 (System A & N, sodium-coupled neutral amino acid transporter), SLC39 (metal ion transporter), SLC40 (basolateral iron transporter), SLC41 (MgtE-like magnesium transporter), SLC42 (Rh ammonium transporter family (pending)), SLC43 (Na+-independent, system-L like amino acid transporter), SLC44 (Choline-like transporter), SLC45 (Putative sugar transporter), SLC46 (Folate transporter), SLC47 (Multidrug and Toxin Extrusion (MATE)), SLC48 (Heme transporter), SLC49 (FLVCR-related transporter), SLC50 (Sugar efflux transporters), SLC51 (Transporters of steroid-derived molecules), and SLC52 (Riboflavin transporter), SLC53 (Phosphate carriers), SLC54 (Mitochondrial pyruvate carriers), SLC 55 (Mitochondrial cation/proton exchangers), SLC56 (Sideroflexins), SLC57 (NiPA-like magnesium transporter family), SLC58 (MagT-like magnesium transporter family), SLC59 (Sodium-dependent lysophosphatidylcholine symporter family), SLC60 (Glucose transporters), SLC61 (Molybdate transporter family), SLC 62 (Pyrophosphate transporters), SLC63 (Sphingosine-phosphate transporters), SLC64 (Golgi Ca2+/H+ exchangers), SLC65 (NPC-type cholesterol transporters) proteins.

Further, the TMPBM as defined in claim 1 may bind to a solute carrier (SLC) protein, for example but not limited to a TMPBM binding to a member of the major facilitator family selected from the group consisting of CLN3, DIRC2, FLVCR1, FLVCR2, MFSD1, MFSD10, MFSD11, MFSD12, MFSD13A, MFSD14A, MFSD14B, MFSD2A, MFSD2B, MFSD3, MFSD4A, MFSD4B, MFSD5, MFSD6, MFSD6L, MFSD8, MFSD9, MPC1, MPC1L, MPC2, MTCH1, MTCH2, OCA2, RHAG, RHBG, RHCG, SPNS1, SPNS2, SPNS3, SV2A, SV2B, SV2C, SVOP, SVOPL, TMEM104, UCP1, UCP2, UCP3, UNC93A, UNC93B1, XPR1; see, e.g., Perland et al. Open Biol. 2017 Sep;7(9):1701422; PMID:28878041.

Particularly non-limiting examples the TMPBM may bind to SLC9 (Na+/ H+ exchanger) family proteins, even more preferably selected from SLC9A subfamily proteins.

There are over 30 families of secondary transporters, also known as solute carriers or SLC (reviewed by Berger, et al. (2000) in The Kidney: Physiology and Pathophysiology, eds. Seldin DW and Giebisch ., Lippincott, Williams & Wilkins, Philadelphia 1:107-138; see also the website maintained by the HUGO gene nomenclature committee, University College London, UK; in particular Gene Nomenclature Committee (HGNC); see also (http://slc. bi oparadigms.org/; https://www.ncbi.nlm.nih.gov/pmc/articles/PMC3853582; https://salilab.org/pdf/Schlessinger_ClinPharmacolTher_2013.pdf; https://www.tandfonline.com/doi/pdf/10.1080/09687688.2018.1448123; Perland et al. Open Biol. 2017 Sep;7(9):1701422; PMID:28878041; https://re-solute.eu/knowledgebase).

For example, sequences of the SLC9 family may comprise the sequences as provided by https://www.uniprot.org. Such sequences of the SLC9 family include but are not limited to SLC9A1, also denoted as P19634, and having SEQ ID NO.1; SLC9A2, also denoted as Q9UBYO, and having the SEQ ID NO.2; SLC9A3, also denoted as P48764, and having SEQ ID NO.3; SLC9A4, also denoted as Q6AI14 and having SEQ ID NO.4; SLC9A4, also denoted as Q6AI14 and having SEQ ID NO.4; SLC9A5, also denoted as Q14940 and having SEQ ID NO.5; SLC9A6, also denoted as Q92581 and having SEQ ID NO.6; SLC9A7, also denoted as Q96T83; SLC9A8, also denoted as Q9Y2E8 and having SEQ ID NO.7; SLC9A9, also denoted as Q8IVB4 and having SEQ ID NO.8; SLC9B1, also denoted as Q4ZJI4; SLC9B2, also denoted as Q86UD5 and having SEQ ID NO.9; SLC9C1, also denoted as Q4G0N8; and SLC9C2, also denoted as Q5TAH2.

As used herein, the term "SLC", "solute carrier" or "SLC transporter" are used interchangeably and refer to a protein encoded by the corresponding SLC gene, e.g. by the term "SLC9A1 polypeptide" is meant a polypeptide encoded by SLC9A1 gene. The SLCs are also known in the art by other names recognized by the skilled person, e.g. SLC9A1 is also known in the art as NHE-1 .

"SLC", "solute carrier" or "SLC transporter" as used herein may belong to different members such as for example but not limited to amino acid transporter. The following members of the solute carrier families are:
(A) high affinity glutamate and neutral amino acid transporters including SLClAl, SLC1A2, SLC1A3, SLC1A4, SLC1A5, SLC1A6, SLC1A7;
(B) facilitative GLUT transporter including, SLC2A1, SLC2A2, SLC2A3, SLC2A4, SLC2A5, SLC2A6, SLC2A7, SLC2A8, SLC2A9, SLC2A10, SLC2A11, SLC2A12, SLC2A13, SLC2A14;
(C) heavy subunits of heteromeric amino acid transporters including SLC3A1, SLC3A2;
(D) bicarbonate transporters including SLC4A1, SLC4A1, SLC4A2, SLC4A3, SLC4A4, SLC4A5, SLC4A6, SLC4A7, SLC4A8, SLC4A9, SLC4A10, SLC4A11;
(E) sodium glucose cotransporters SLC5A1, SLC5A2, SLC5A3, SLC5A4, SLC5A5, SLC5A6, SLC5A7, SLC5A8, SLC5A9, SLC5A10, SLC5A11, SLC5A12; (F) sodium- and chloride-dependent neurotransmitter transporters including
   SLC6A1, SLC6A2, SLC6A3, SLC6A4, SLC6A5, SLC6A6, SLC6A7, SLC6A8, SLC6A9, SLC6A10, SLC6A11, SLC6A12, SLC6A13, SLC6A14, SLC6A15, SLC6A16, SLC6A17, SLC6A18, SLC6A19, SLC6A20;
(G) cationic amino acid transporter/glycoprotein-associated including SLC7A1, SLC7A2, SLC7A3, SLC7A4, SLC7A5, SLC7A6, SLC7A7, SLC7A8, SLC7A9, SLC7A10, SLC7A11, SLC7A13, SLC7A14;
(H) Na+/Ca2+ exchangers including SLC8A1, SLC8A2, SLC8A3;
(I) Na+/H+ exchangers including SLC9A1, SLC9A2, SLC9A3, SLC9A4, SLC9A5, SLC9A6, SLC9A7, SLC9A8, SLC9A9, SLC9A10, SLC9A11;
(J) organic cation/anion/zwitterion transporters including SLC22A1, SLC22A2, SLC22A3, SLC22A4, SLC22A5, SLC22A6, SLC22A7, SLC22A8, SLC22A9, SLC22A10, SLC22A11, SLC22A12, SLC22A13, SLC22A14, SLC22A15, SLC22A16, SLC22A17, SLC22A18, SLC22A19, SLC22A20;
(K) Na+/(Ca2+-K+) exchangers including SLC24A1, SLC24A2, SLC24A3, SLC24A4, SLC24A5, SLC24A6;
(L) multifunctional anion exchangers including SLC26A1, SLC26A2, SLC26A3, SLC26A4, SLC26A5, SLC26A6, SLC26A7, SLC26A8, SLC26A9, SLC26A10, SLC26A11;
(M) H+/amino acid symporters including SLC36A1, SLC36A2, SLC36A3, SLC36A4;
(N) System A & N, sodium-coupled neutral amino acid transporters including SLC38A1, SLC38A2, SLC38A3, SLC38A4, SLC38A5, SLC38A6; and (O) Na+-independent, system-L like amino acid transporters including SLC43A1, SLC43A2, SLC43A3;
(O) Na+-independent, system-L like amino acid transporters including SLC43A1, SLC43A2, SLC43A3.

The transmembrane protein/transmembrane proteins may be ubiquitinated by the E3 ligase complex/cullin-RING ubiquitin ligase complex/CRL complex. Particularly, the inventors found that transmembrane protein/transmembrane proteins can be recognized by the compounds of the present invention. Such transmembrane protein/transmembrane proteins may further include proteins which are not recognized by E3 ligase complex/cullin-RING ubiquitin ligase complex/CRL complex in the absence of the compound of the present invention. Thus, it has been surprisingly found that the compounds of the present invention are able to induce ubiquitination of the transmembrane protein/transmembrane proteins, i.e. leading to degradation of the transmembrane protein/transmembrane proteins by the ubiquitination/proteasome system.

In one particular embodiment, the TMPBM as defined in claim 1 may bind to a solute carrier (SLC) protein;
(i) wherein the SLC (i) may be a SLC located on the cell surface, the endoplasmatic reticulum (ER), the Golgi apparatus, the mitochondria, the intracellular vesicles and/or the lysosomes; for example wherein the SLC located on the cell surface may be selected from the group consisting of SLC38A1, SLC38A2, SLC2A1, SLC2A3, SLC16A1; SLC1A5; SLC4A4 and SLC7A3; wherein the SLC located at the ER may be selected from the group consisting SLC30A9 and SLC39A7, wherein the SLC on the Golgi apparatus may be SLC35B2 and SLC33A1, wherein the SLC on the mitochondria may be SLC25A50 (also denoted as Mitochondrial carrier homolog 2(MTCH2)) and wherein the SLC located in the lysosomes may be SLC38A9. More particularly, the SLC located on the cell surface may be SLC38A1, SLC38A2, SLC2A3, SLC1A5, SLC16A1, SLC4A4, SLC7A3, SLC1A5 and SLC2A1, even more particularly SLC38A1, SLC38A2, SLC2A3, SLC1A5, SLC16A1, SLC4A4 and SLC7A3;
   and/or
(ii) wherein the SLC may be a member of the SLC9 family: for example wherein the SLC may be SLC9A1, SLC9A2, SLC9A3, SLC9A4, SLC9A5, SLC9A6, SLC9A7, SLC9A8, SLC9A9, SLC9B1, SLC9B2, SLC9C1 and SLC9C2. Particularly, the SLC may be SLC9A1, SLC9A2, SLC9A3, SLC9A4, SLC9A5, SLC9A6, SLC9A8, SLC9A9 and SLC9B2. Even more particularly, the SLC may be SLC9A1, SLC9A2 and SLC9A4 and even more particularly the SLC may be SLC9A1.In one embodiment, the TMPBM as defined in claim 1 may bind to SLC9A1.

In another particular embodiment, the TMPBM as defined in claim 1 may bind to a solute carrier (SLC) protein;
(i) wherein the SLC (i) may be a SLC located on the cell surface, the endoplasmatic reticulum (ER), the Golgi apparatus, the mitochondria, the intracellular vesicles and/or the lysosomes; for example wherein the SLC located on the cell surface may be selected from the group consisting of SLC38A1, SLC38A2, SLC2A1, SLC2A3, SLC16A1; SLC4A4 and SLC7A3; wherein the SLC located at the ER may be selected from the group consisting SLC30A9 and SLC39A7, wherein the SLC on the Golgi apparatus may be SLC35B2 and SLC33A1, wherein the SLC on the mitochondria may be SLC25A50 (also denoted as Mitochondrial carrier homolog 2(MTCH2)) and wherein the SLC located in the lysosomes may be SLC38A9. More particularly, the SLC located on the cell surface may be SLC38A1, SLC38A2, SLC2A3, SLC1A5, SLC16A1, SLC4A4, SLC7A3, SLC1A5 and SLC2A1, even more particularly SLC38A1, SLC38A2, SLC2A3, SLC1A5, SLC16A1, SLC4A4 and SLC7A3;
   and/or
(ii) wherein the SLC may be a member of the SLC9 family: for example wherein the SLC may be SLC9A1, SLC9A2, SLC9A3, SLC9A4, SLC9A5, SLC9A6, SLC9A7, SLC9A8, SLC9A9, SLC9B1, SLC9B2, SLC9C1 and SLC9C2. Particularly, the SLC may be SLC9A1, SLC9A2, SLC9A3, SLC9A4, SLC9A5, SLC9A6, SLC9A7, SLC9A8, SLC9A9, SLC9B1 and SLC9B2. Even more particularly, the SLC may be SLC9A1, SLC9A2 and SLC9A4 and even more particularly the SLC may be SLC9A1.In one embodiment, the TMPBM as defined in claim 1 may bind to SLC9A1.

The intracellular vesicles as disclosed herein and in context of the invention may be for example, synaptic vesicles, insulin granules or phagosomes. The person skilled in the art can clearly identify intracellular vesicles carrying a transmembrane protein, such as an SLC protein and can further identify subtypes of transmembrane proteins such as from the SLC family on such an intracellular vesicle.

In one embodiment, the TMPBM as defined in claim 1 may bind onany binding site of a transmembrane protein. For example, the binding site of a transmembrane protein may be without a functional effect, such as without an inhibitory, allosteric and/or activating effect. In one embodiment, the TMPBM as defined in claim 1 may bind to an inhibitory site, an allosteric binding site of the transmembrane protein such as SLC.

Non-limiting examples of a binding site is an activatory site, an inhibitory site or an allosteric binding site of the transmembrane protein such as SLC. As used herein, the term "orthosteric binding site" refers to the site within a transmembrane protein that binds an endogenous compound. By contrast, the term "allosteric binding site" refers to a site other than an orthosteric binding site to which compounds bind. Accordingly, allosteric compounds bind to a transmembrane protein through a site that is spatially distinct from the orthosteric binding site. For example, binding of a compound as disclosed herein and as used in context of the invention to an allosteric binding site of the transmembrane protein may maintain the functionality of said transmembrane protein. As another example, binding of a compound as disclosed herein and as used in context of the invention to an allosteric binding site of the transmembrane protein may result in a conformational change of said transmembrane protein which may alter the functionality of said transmembrane protein. The term "inhibitory binding site" refers to the site within a transmembrane protein that binds an inhibitory compound. In other words, a compound as disclosed herein and as used in context of the invention bound to an inhibitory binding site of the transmembrane protein/transmembrane proteins may decrease and/or deplete the function of said transmembrane protein/transmembrane proteins.

In one embodiment, the present invention provides compounds that stimulate/induce ubiquitination of a target protein/target proteins, i.e. transmembrane protein/transmembrane protein/transmembrane degradation by the cullin RING E3 ligase, wherein the compound is a compound of compounds of formulae (I) as disclosed herein and illustrated in the appended examples. A corresponding read-out whether the compound to be identified in the methods of this invention is able and/or capable to stimulate/induce ubiquitination of a transmembrane protein/transmembrane proteins may be the measurement of the level of the transmembrane protein/transmembrane proteins. When said "level" of the target transmembrane protein/transmembrane proteins is decreased in the presence of the compound compared to the "level" determined in the absence of the compound, said compound is able to stimulate/induce the activity of the E3 ubiquitin ligase. For example, the prediction whether a compound is able to degrade a transmembrane protein/transmembrane proteins, in particular by inducing ubiquitination of a transmembrane protein/transmembrane proteins can be assessed, e.g., by the determination of a fold change in transmembrane protein/transmembrane proteins level. Hereby, the respective fold change value for identifying a compound to degrade a protein refers to measurements of the level of a transmembrane protein/transmembrane proteins. Fold change values may be determined by previously described methods known in the art. For example, methods are known to a skilled person for using the Coefficient of variation in assessing variability of quantitative assays in order to establish fold change values (Reed et al., Clin Diagn Lab Immunol.2002; 9(6):1235-1239).

In one embodiment, the present invention relates to a compound for use in medicine. The term "medicine" as used herein is intended to be a generic term inclusive of prescription and non-prescription medications. The compound for use in medicine should be understood as being useful in maintaining health or promoting recovery from a disease, preferably cancer. Further, the term "medicine" includes medicine in any form, including, without limitation, e.g., pills, salves, creams, powders, ointments, capsules, injectable medications, drops, vitamins and suppositories. The scope of this invention is not limited by the type, form or dosage of the medicine. **In** one aspect of the present invention, the chemical compound or agent is for use in the treatment of cancer.

**In** context of the invention, the compounds are particularly useful as medicaments, for example in the treatment of diseases and/or disorders wherein it is desired to degrade target protein/ target proteins via ubiquitination. Thus, in one embodiment, the invention refers to a composition comprising a compound as disclosed herein and in context of the invention. The term "composition", "pharmaceutical composition" or "pharmaceutical formulation" refers to a preparation which is in such form as to permit the biological activity of an active ingredient contained therein to be effective, and which contains no additional components which are unacceptably toxic to a subject to which the pharmaceutical composition would be administered. As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, decreasing the rate of disease progression, amelioration or palliation of the disease state, and remission or improved prognosis. "Alleviation," "alleviating," or equivalents thereof, refer to both therapeutic treatment and prophylactic or preventative measures, wherein the object is to ameliorate, prevent, slow down (lessen), decrease or inhibit a disease or condition, e.g., the formation of atherosclerotic plaques. Those in need of treatment include those already with the disease or condition as well as those prone to having the disease or condition or those in whom the disease or condition is to be prevented.

The present invention also provides the compound of the present invention for use in treating such diseases or disorders, wherein the compound of the present invention is to be administered to an individual in need of such a treatment with the compound of the invention, i.e. the compound that can stimulating/induce ubiquitination of a target protein/target proteins. A "disorder," a "disease," or a "condition," as used interchangeably herein, is any condition that would benefit from treatment with a composition (e.g., a pharmaceutical composition) described herein. This includes chronic and acute disorders or diseases including those pathological conditions which predispose the mammal to the disorder in question.

A large number of diseases may be treated or prevented by the compounds of the present invention. These include 2p21 microdeletion syndrome, abnormal coordination, abnormality of the cornea, absence seizures, ache, type ib (disorder) achondrogenesis, acidosis, acrodermatitis, acrodermatitis enteropathica, acute cerebrovascular accidents, acute confusional senile dementia, acute kidney injury, acute kidney insufficiency, acute lung injury, adenocarcinoma of lung (disorder), adenoid cystic carcinoma, adenoma, basal cell adenoma, microcystic adenoma, monomorphic adenoma, trabecular adenoma, adult fanconi syndrome, adult learning disorders, adult neuronal ceroid lipofuscinosis, adult-onset citrullinemia type 2, adverse reaction to drug, psychotic affective disorders, age related macular degeneration, age-related memory disorders, akinetic-rigid variant of Huntington disease, ocular albinism, alcohol abuse, alcohol sensitivity, alcohol use disorder, alcohol withdrawal seizures, alcohol withdrawal-induced major motor seizure, alcoholic intoxication, chronic alcoholic intoxication, Allan-Herndon-Dudley syndrome (ahds), allodynia, alloxan diabetes, alternating hemiplegia of childhood, early onset Alzheimer disease, late onset Alzheimer disease, Alzheimer's disease, focal onset Alzheimer's disease, amelogenesis imperfecta, amelogenesis imperfecta hypomaturation type, hypomaturation type iia5 amelogenesis imperfecta, type iii amelogenesis imperfecta, inborn errors amino acid metabolism, inherited disorders amino acid metabolism, amphetamine abuse, amphetamine addiction, amphetamine-related disorders, amyotrophic lateral sclerosis, amyotrophic lateral sclerosis with dementia, guam form amyotrophic lateral sclerosis, anaplastic carcinoma, anasarca, anemia, hemolytic anemia, acquired hemolytic anemia, idiopathic acquired hemolytic anemia, hereditary spherocytic hemolytic anemia, hypochromic microcytic anemia with iron overload, hypochromic microcytic anemia with iron overload 1, microangiopathic anemia, pyridoxine-refractory sideroblastic anemia 2, pyridoxine-refractory autosomal recessive sideroblastic anemia, anhedonia, animal mammary neoplasms, anxiety disorders, anxiety neurosis (finding), neurotic anxiety states, thoracic aortic aneurysm, aortic valve calcification, apnea, aprosodia, arterial tortuosity, arterial tortuosity syndrome, arthrogryposis, arthrogryposis, mental retardation and seizures, as if personality, asperger syndrome, ataxia, appendicular ataxia, motor ataxia, sensory ataxia, truncal ataxia, hereditary ataxias, atelosteogenesis type 2, atherogenesis, atherosclerosis, atonic absence seizures, atrophic, attention deficit disorder, attention deficit hyperactivity disorder, attention deficit hyperactivity disorder (ADHD), atypical ductal breast hyperplasia, aura, autism spectrum disorders, autistic disorder, autoimmune hemolytic anemia, autosomal dominant juvenile parkinson disease, autosomal dominant parkinsonism, autosomal recessive parkinsonism, autosomal recessive polycystic kidney disease, autosomal recessive primary microcephaly, autosomal recessive sideroblastic anemia, avoidant personality disorder, awakening epilepsy, nonobstructive azoospermia, Barrett epithelium, Barrett esophagus, Bartter disease, antenatal type 1 Bartter syndrome, basal cell carcinoma, idiopathic basal ganglia calcification 6, biotin-responsive basal ganglia disease, basal ganglia diseases, benign neoplasm, bilateral deafness, bile acid coa ligase deficiency and defective amidation, primary bile acid malabsorption, biliary cirrhosis, secondary biliary cirrhosis, bipolar disorder, bipolar i disorder, Birk-Landau-Perez syndrome, bladder neoplasm, blastocyst disintegration, body mass index quantitative trait locus 11, body mass index quantitative trait locus 4 (disorder), bone diseases, developmental bone diseases, brain diseases, brain dopamine-serotonin vesicular transport disease, brain hemorrhage, brain injuries, focal brain injuries, brain ischemia, brain lacerations, breast cancer, familial breast cancer, breast carcinoma, brittle diabetes, bronchioloalveolar adenocarcinoma, brown oculocutaneous albinism, Brown-Vialetto-van Laere syndrome, Brown-Vialetto-van Laere syndrome 2, brucellosis, pulmonary brucellosis, bulbar palsy, burn induced multiple organ injury, buruli ulcer, calcinosis, calcium pyrophosphate arthropathy, calcium pyrophosphate deposition disease, embryonal cancer, embryonal and mixed cancer, cannabis use, carbohydrate deficient glycoprotein syndrome type 2k, carcinoma, carcinoma of lung, spindle-cell carcinoma, carcinomatosis, cardiac arrhythmia, cardiac hypertrophy, cardiac ischaemic reperfusion, cardiomegaly, dilated cardiomyopathy, familial idiopathic cardiomyopathy, cardiovascular diseases, carditis, carnitine-acylcarnitine translocase deficiency, cataract, cataract 47, cataract and cardiomyopathy, juvenile cataract with microcornea and glucosuria, celiac disease, central hypothyroidism, cerebral injury, cerebral ischemia, cerebrovascular accident, cerebrovascular disorders, neuronal ceroid lipofuscinosis 7, parry type neuronal ceroid lipofuscinosis, ceruloplasmin deficiency, cervical cancer, Charcot-Marie-Tooth disease, type ia (disorder) Charcot-Marie-Tooth disease, type ib Charcot-Marie-Tooth disease, chemical and drug induced liver injury, chemically-induced liver toxicity, child development deviations, specific child development disorders, childhood acute lymphoblastic leukemia, childhood progressive bulbar palsy, childhood tic disorders, cholangiocarcinoma, choledochal cyst, type i choledochal cyst, type ii choledochal cyst, type iii choledochal cyst, type iv choledochal cyst, type v choledochal cyst, cholestasis, cholestasis in newborn, episodic choreoathetosis/spasticity, chromophobe renal cell carcinoma, chronic depression, chronic motor or vocal tic disorder, cirrhosis, type ii neonatal-onset citrullinemia, cleft palate, isolated cleft palate, cleft upper lip, cluttering, cocaine abuse, cocaine dependence, cocaine-induced mood disorder, cocaine-related disorders, cognition disorders, cognitive impairment, cold hemagglutinin disease, collecting duct carcinoma of the kidney, collodion fetus, colon cancer, colonic neoplasms, colorectal cancer, colorectal carcinoma, colorectal neoplasms, combined d-2- and l-2-hydroxyglutaric aciduria, combined oxidative phosphorylation deficiency 18, combined oxidative phosphorylation deficiency 28, complete hearing loss, complex partial seizures, complex partial status epilepticus, preneoplastic condition, conduction disorder of the heart, congenital nonprogressive cone-rod synaptic disorder, congenital abnormality, congenital anemia, congenital aneurysm of ascending aorta, congenital cataracts, hearing loss and neurodegeneration, congenital chloride diarrhea, type 2c congenital disorder of glycosylation, type iif congenital disorder of glycosylation, type iim congenital disorder of glycosylation, type iin congenital disorder of glycosylation, congenital disorders of glycosylation, congenital glucose-galactose malabsorption, congenital heart defects, congenital hypothyroidism, congenital ichthyosis, postsynaptic congenital myasthenic syndromes, presynaptic congenital myasthenic syndromes, congenital myopathy (disorder), congenital nonbullous ichthyosiform erythroderma, sodium type (disorder) congenital secretory diarrhea, congestive heart failure, connective tissue diseases, constipation, constipation-predominant irritable bowel syndrome, contact dermatitis, contact hypersensitivity, conventional (clear cell) renal cell carcinoma, convulsions, convulsive seizures, corneal dystrophy and perceptive deafness, fuchs endothelial corneal dystrophy 4, corneal endothelial dystrophy 2, corneal opacity, corpus callosum agenesis neuronopathy, cortical dysplasia, coxsackievirus infections, craniofacial abnormalities, autosomal dominant craniometaphyseal dysplasia, cranioosteoarthropathy, craniosynostosis, x-linked creatine deficiency, crohn disease, Crohn's disease of large bowel, Crohn's disease of the ileum, stomatin-deficient cryohydrocytosis with mental retardation, seizures, cataracts, and massive hepatosplenomegaly, cystic fibrosis, cystic fibrosis modifier 1, cystinuria, type a cystinuria, type a-b cystinuria, type b cystinuria, cytopenia, de la chapelle dysplasia, de Toni-Debre-Fanconi syndrome, deaf mutism, deafness, acquired deafness, autosomal dominant 25 (disorder) deafness, autosomal dominant 72 deafness, autosomal recessive 4 deafness with enlarged vestibular aqueduct, autosomal recessive 61 deafness, deficiency of glutamate decarboxylase, degenerative polyarthritis, delirium, dementia, depressed mood, depression, bipolar depression, neurotic depression, postpartum depression, depressive disorder, treatment-resistant depressive disorder, depressive symptoms, depressive syndrome, allergic contact dermatitis, atopic dermatitis, developmental academic disorder, developmental disabilities, diabetes mellitus, experimental diabetes mellitus, insulin-dependent diabetes mellitus, ketosis-prone diabetes mellitus, non-insulin-dependent diabetes mellitus, diabetes mellitus type 2, autoimmune diabetes, diabetic cardiomyopathies, diabetic cataract, diarrhea, diastrophic dysplasia, dicarboxylicaminoaciduria, disease exacerbation, distal renal tubular acidosis, down syndrome, partial trisomy 21 down syndrome, drug abuse, drug dependence, drug habituation, drug toxicity, drug use disorders, drug withdrawal symptoms, drug-induced acute liver injury, drug-induced depressive state, drug-induced liver disease, ductal carcinoma, dysarthria, flaccid dysarthria, guttural dysarthria, mixed dysarthria, scanning dysarthria, spastic dysarthria, dysglossia, dyslalia, dysosteosclerosis, dysphoric mood, dysthymic disorder, dystonia, dystonia 18 (disorder), diurnal dystonia, limb dystonia, paroxysmal dystonia, ear diseases, early infantile epileptic encephalopathy with suppression bursts, early myoclonic encephalopathy, infantile eczema, edema, Ehlers-Danlos syndrome, type iv Ehlers-Danlos syndrome, elliptocytosis 4, hereditary elliptocytosis, embryo disintegration, embryo loss, embryonal neoplasm, embryonal rhabdomyosarcoma, encephaloclastic proliferative vasculopathy, encephalopathies, endogenous depression, endometrioma, endometriosis, enterovirus infections, epilepsy, anterior fronto-polar epilepsy, benign psychomotor childhood epilepsy, childhood absence epilepsy, cingulate epilepsy, cryptogenic epilepsy, frontal lobe epilepsy, susceptibility to idiopathic generalized epilepsy 12, susceptibility to idiopathic generalized epilepsy 14, lateral temporal epilepsy, opercular epilepsy, orbito-frontal epilepsy, pyridoxine-dependent epilepsy, supplementary motor epilepsy, temporal lobe epilepsy, epileptic drop attack, epileptic encephalopathy, early infantile epileptic encephalopathy 25, early infantile epileptic encephalopathy 34, early infantile epileptic encephalopathy 41, epileptic seizures, multiple epiphyseal dysplasia 4, type 6 (disorder) episodic ataxia, erythrocyte lactate transporter defect, erythrocytosis, esophageal neoplasms, etat marbre, riboflavin-responsive exercise intolerance, experimental hepatoma, external ophthalmoplegia, extrahepatic cholangiocarcinoma, extrapyramidal disorders, extravascular hemolysis, fahr's syndrome (disorder), familial Alzheimer disease (fad), familial apoceruloplasmin deficiency, familial dementia, familial juvenile parkinsonism, familial ménière disease, familial renal glucosuria, fanconi syndrome, fanconi-bickel syndrome, fatty liver, fazio-londe syndrome, fibrosis, liver fibrosis, flaccid muscle tone, floppy muscles, hereditary folate malabsorption, follicular adenoma, food allergy, foveal hypoplasia 2, foveal hypoplasia and anterior segment dysgenesis, fowler syndrome, fracture, spiral fracture, benign childhood frontal epilepsy, fuchs endothelial dystrophy, gastric cancer, gastrointestinal dysfunction, gastrointestinal neoplasms, gastrointestinal stromal sarcoma, gastrointestinal stromal tumors, generalized absence seizures, generalized pustular psoriasis, generalized seizures, germ cell cancer, germ cell tumor, gianotti-crosti syndrome, gitelman syndrome, glaucoma, glioma, glomerular filtration rate, glomerulosclerosis, glucose-6-phosphate transport defect, glut1 deficiency syndrome, autosomal recessive glut1 deficiency syndrome 1, glycine encephalopathy with normal serum glycine, glycogen storage disease ic, glycogen storage disease type id, renal glycosuria, goiter, gorlin chaudhry moss syndrome, gout, gout susceptibility 2, grand mal status epilepticus, gustatory seizure, harlequin fetus, hartnup disease, head and neck squamous cell carcinoma, hearing impairment, extreme hearing loss, heart decompensation, heart diseases, heart failure, right-sided heart failure, hematological disease, hemochromatosis, type 4 hemochromatosis, hemolysis (disorder), nonalcoholic fatty liver disease hepatic steatosis, hepatitis b, drug-induced hepatitis, toxic hepatitis, morris hepatoma, novikoff hepatoma, hepatomegaly, hereditary clear cell renal cell carcinoma, hereditary clubbing, hereditary diffuse gastric cancer, hereditary hemochromatosis, hereditary hyperexplexia, hereditary motor and sensory neuropathy type i, hereditary motor and sensory neuropathy with optic atrophy (disorder), hereditary motor and sensory-neuropathy type ii, hereditary spherocytosis, herpes encephalitis, herpetic acute necrotizing encephalitis, herpetic meningoencephalitis, hhh syndrome, histiocytosis, histiocytosis with joint contractures and sensorineural deafness, hiv coinfection, hiv infections, Huntington disease, late onset Huntington disease, hydrocephalus, hydrops fetalis, hyperactive behavior, hyperalgesia, primary hyperalgesia, secondary hyperalgesia, thermal hyperalgesia, hyperammonaemia, hyperammonemia, hypercalcemia, idiopathic hypercalcemia of infancy, infantile hypercalcemia, infantile hypercalcemia 1, infantile hypercalcemia 2, hypercalciuria, familial hypercholesterolemia, hyperekplexia 3, hyperglycinuria (disorder), hyperhomocysteinemia, familial hyperinsulinemic hypoglycemia 7, hyperinsulinism due to uncoupling protein 2 deficiency, generalized hyperkinesia, hypermanganesemia with dystonia 2, hypermanganesemia with dystonia polycythemia and cirrhosis, hyperoxaluria, hyperphosphatemia, hyperpigmentation, hypertensive disease, hypertrichosis, hypertrophic cardiomyopathy, primary autosomal dominant hypertrophic osteoarthropathy, primary autosomal recessive hypertrophic osteoarthropathy 2, hyperuricemia, hypogonadism, isolated hypogonadotropic hypogonadism, hypogonadotropic hypogonadism, global cerebral hypomyelination, hypophosphatemia, hereditary hypophosphatemic rickets with hypercalciuria, hypotension, hypothyroidism, hypotonia-cystinuria syndrome, renal hypouricemia 2, ichthyosis congenita ii, ichthyosis prematurity syndrome, congenital autosomal recessive ichthyosis 6, idiopathic autoimmune hemolytic anemia, idiopathic generalized epilepsy, idiopathic hypogonadotropic hypogonadism, iga glomerulonephritis, iieocolitis, iminoglycinuria, immersion related epilepsy, impaired glucose tolerance, impulse-ridden personality, inadequate personality, inborn errors of metabolism, infantile free sialic acid storage disease, infantile neuronal ceroid lipofuscinosis, infantile nystagmus, infantile sialic acid storage disease, inflammation, inflammatory bowel disease, inflammatory bowel diseases, inflammatory responses in cerebral ischemia and traumatic brain injury, influenza, acute brain injuries, insulin resistance, susceptibility to insulin resistance, insulin sensitivity, intellectual developmental disorder with neuropsychiatric features, intellectual disability, internal ophthalmoplegia, intestinal obstruction, intracranial hemorrhages, intracranial hypertension, intrahepatic cholangiocarcinoma, intravascular hemolysis, involutional depression, involutional paraphrenia, iron deficiency anemia, iron overload, irritable heart, jacksonian seizure, juvenile arthritis, juvenile Huntington disease, juvenile neuronal ceroid lipofuscinosis, juvenile psoriatic arthritis, juvenile-onset still disease, ketotic hypoglycemia, kidney calculi, kidney diseases, acute kidney failure, kidney injury, kidney neoplasm, kohlschutter tonz syndrome, kyphosis deformity of spine, lactic acidosis induced pulmonary vein arrhythmogenesis, lactic acidosis induced sepsis, late-infantile neuronal ceroid lipfuscinosis, learning disabilities, learning disorders, learning disturbance, left-sided heart failure, visceral leishmaniasis, lens opacities, lenticulostriate disorders, leprosy, leukemia, acute lymphocytic leukemia l2, leukodystrophy, libman-sacks disease, lichtenstein-knorr syndrome, lipoidosis, liver cancer, liver carcinoma, liver cirrhosis, experimental liver cirrhosis, liver diseases, liver dysfunction, liver neoplasms, experimental liver neoplasms, long sleeper syndrome, low tension glaucoma, lung cancer, lung neoplasms, systemic lupus erythematosus, lysinuric protein intolerance, macular dystrophy with central cone involvement, major depressive disorder, malabsorption syndrome, malaria, malformations of cortical development, malignant glioma, malignant mesothelioma, malignant neoplasm of breast, malignant neoplasm of esophagus, malignant neoplasm of kidney, malignant neoplasm of liver, malignant neoplasm of lung, malignant neoplasm of ovary, malignant neoplasm of pancreas, malignant neoplasm of prostate, malignant neoplasm of salivary gland, malignant neoplasm of stomach, malignant neoplasm of urinary bladder, malignant neoplasms, malignant tumor of colon, animal mammary carcinoma, mammary neoplasms, experimental mammary neoplasms, human mammary neoplasms, manganese poisoning, manic, manic disorder, marfan syndrome, marijuana abuse, mechanical allodynia, susceptibility to meconium ileus in cystic fibrosis, megacolon, melancholia, melanoma, semantic memory disorder, spatial memory disorder, memory disorders, memory impairment, memory loss, meningococcal meningitis of serogroup a, meningococcal meningitis of serogroup b, meningococcal meningitis of serogroup c, meningococcal meningitis of serogroup w-135, meningococcal meningitis of serogroup y, meningococcal meningitis, mental deficiency, mental depression, south african type mental retardation x-linked, autosomal recessive 48 mental retardation, autosomal recessive 7 mental retardation, psychosocial mental retardation, x-linked mental retardation, x-linked syndromic christianson type mental retardation, mesothelioma, metabolic acidosis, metabolic bone disorder, microangiopathic hemolytic anemia, microcalcification, microcephaly, primary autosomal recessive microcephaly 15, amish type (disorder) microcephaly, microlissencephaly, milk-alkali syndrome, minimal brain dysfunction, mitochondrial diseases, autosomal dominant mitochondrial dna depletion syndrome 12a (cardiomyopathic type) autosomal recessive mitochondrial dna depletion syndrome 12b (cardiomyopathic type), mitochondrial phosphate carrier deficiency, mitochondrial pyruvate carrier deficiency, mitral valve prolapse syndrome, mixed gliomas, monocarboxylate transporter 1 deficiency, mood disorders, motor tic disorders, movement disorders, multiple epiphyseal dysplasia, multiple sclerosis, acute fulminating multiple sclerosis, muscle hypotonia, muscle tone atonic, presynaptic congenital myasthenic syndrome 20, presynaptic congenital myasthenic syndrome 21, congenital myasthenic syndromes, congenital slow channel myasthenic syndromes, protection against mycobacterium tuberculosis, susceptibility to (finding) mycobacterium tuberculosis, myocardial failure, myocardial infraction, myocardial ischemia, myocardial reperfusion injury, myocarditis, myoclonic astatic epilepsy, myoclonic seizures, myoclonic-atonic epilepsy, myopathy, autosomal dominant myopia 24, n-acetylneuraminic acid storage disease, narcissistic personality disorder, necrosis, neonatal hypotonia, neoplasm invasiveness, neoplasm metastasis, neoplasms, embryonal and mixed neoplasms, germ cell and embryonal neoplasms, neoplastic cell transformation, nephrocalcinosis, calcium oxalate nephrolithiasis, hypophosphatemic nephrolithiasis-osteoporosis 1, nerve degeneration, nervous system disorder, neurocirculatory asthenia, neurodevelopmental disorders, neuronal ceroid-lipofuscinoses, neuropathic pain, distal hereditary motor type viia neuropathy, hereditary motor and sensory type vibneuropathy, nova scotian type niemann-pick disease, type c niemann-pick disease, type c1 niemann-pick disease, type d niemann-pick disease, congenital stationary night blindness, congenital stationary type 1 night blindness, congenital stationary type 1a night blindness, congenital stationary type 1b night blindness, congenital stationary type 2a night blindness, congenital stationary type 2b (disorder) night blindness, non-convulsive status epilepticus, non-epileptic convulsion, nonepileptic seizures, noninfiltrating intraductal carcinoma, nonorganic psychosis, non-small cell lung carcinoma, obesity, obsessive-compulsive disorder, oculocutaneous albinism type 2, oculocutaneous albinism type 6, type iv oculocutaneous albinism, odontome, olfactory seizure, ophthalmoparesis, ophthalmoplegia, optic atrophy, substance-induced organic mental disorders, orthostatic intolerance, primary hypertrophic osteoarthropathy, osteoarthrosis deformans, osteogenesis imperfecta, osteopenia, malaysian-melanesian-filipino type ovalocytosis, ovarian cancer, ovarian neoplasm, oxalosis, pain, burning pain, crushing pain, migratory pain, splitting pain, pancreatic cancer, pancreatic neoplasm, pancytopenia, papillary adenoma, papillary renal cell carcinoma, paranoia, parkinson disease, autosomal recessive juvenile parkinson disease 2, parkinsonian disorders, experimental parkinsonism, juvenile parkinsonism, infantile parkinsonism-dystonia, pediatric autoimmune disease, pendred's syndrome, peripheral diabetic neuropathy, peripheral neuropathy, peritoneal neoplasms, peritoneal surface malignancy, personality disorders, petit mal status, petty laxova wiedemann syndrome, physiological wound healing, pigmented basal cell carcinoma, pitt-rogers-danks syndrome, poisoning, polycystic kidney disease, polycythemia, disseminated superficial actinic type porokeratosis 8, disseminated superficial actinic porokeratosis, posterior column ataxia with retinitis pigmentosa, posterior fossa hemorrhage, post-traumatic tic disorder, precancerous conditions, precursor cell lymphoblastic leukemia lymphoma, prelingual deafness, prenatal injuries, prescription drug abuse, presenile dementia, primary biliary cirrhosis, primary hypogonadism, primary hypothyroidism, primary microcephaly, profound mental retardation, progressive bulbar palsy, autosomal dominant progressive external ophthalmoplegia with mitochondrial dna deletions 1, autosomal dominant progressive external ophthalmoplegia with mitochondrial dna deletions 2, prostate cancer, prostatic neoplasms, pseudoaphakia, pseudohyperkalemia cardiff, drug psychoses, substance-induced psychoses, involutional psychosis, psychotic disorders, pulmonary alveolar microlithiasis, pulmonary hypertension, radiating pain, ramsay hunt paralysis syndrome, recurrent depression, regional enteritis, renal carnitine transport defect, renal cell carcinoma, renal hypouricemia, renal tubular acidosis, distal renal tubular acidosis with hemolytic anemia (disorder), distal renal tubular acidosis with normal red cell morphology, proximal renal tubular acidosis with ocular abnormalities and mental retardation, type ii renal tubular acidosis, reperfusion injury, respiratory depression, adult respiratory distress syndrome, respiratory failure, respiratory hypersensitivity, respiratory insufficiency, retinal degeneration, retinitis pigmentosa, retinitis pigmentosa 68, rh deficiency syndrome, rhabdomyolysis, rhabdomyosarcoma, rhabdomyosarcoma 1, rheumatoid arthritis, rhinolalia, regulator type rh-null, riboflavin deficiency, rotor syndrome, roussy-levy syndrome (disorder), salivary gland neoplasms, sarcoidosis, sarcomatoid renal cell carcinoma, schizophrenia, catatonic schizophrenia, schneckenbecken dysplasia, schwartz-lelek syndrome, seasonal affective disorder, secondary hypothyroidism, seizures, auditory seizures, clonic seizures, focal seizures, sensory seizures, somatosensory seizures, senile paranoid dementia, sensorineural hearing loss (disorder), sensory hearing loss, severe congenital microcephaly, severe depression, severe major depression with psychotic features, hemorrhagic shock, short sleeper syndrome, finnish type (disorder) sialic acid storage disease, sialuria, sideroblastic anemia, simple partial status epilepticus, single seizure, sinus histiocytosis, variation in skin-hair-eye pigmentation 4, sleep disorders, sleep quality, sleep wake disorders, sleep-related neurogenic tachypnea, southeast asian ovalocytosis, autosomal dominant spastic paraplegia 42, autosomal dominant (disorder) spastic paraplegia 6, hereditary spastic paraplegia, thin corpus callosum spastic tetraplegia and progressive microcephaly, speech disorders, type 4 spherocytosis, autosomal recessive 3 spinocerebellar ataxia, Ehlers-Danlos syndrome-like spondylocheirodysplasia, squamous cell carcinoma, squamous cell carcinoma of esophagus, status epilepticus, alcohol withdrawal-induced status epilepticus, subclinical status epilepticus, steatohepatitis, stomach carcinoma, stomach neoplasms, stomatocytosis i, streptozotocin diabetes, stroke, substance abuse problem, substance dependence, substance use disorders, substance withdrawal syndrome, substance-related disorders, subwakefullness syndrome, sudden infant death syndrome, physical suffering, tactile allodynia, testicular microlithiasis, thiamine metabolism dysfunction syndrome 4 (bilateral striatal degeneration and progressive polyneuropathy type), thiamine responsive megaloblastic anemia syndrome, thinness, thyroid agenesis, thyroid carcinoma, thyroid dyshormonogenesis 1, thyroid gland follicular adenoma, thyroid hormone resistance syndrome, thyroid hypoplasia, thyroid neoplasm, tic disorder, vocal tic disorders, tissue fibrosis, susceptibility to (finding) tobacco addiction, tonic - clonic seizures, tonic seizures, tooth abnormalities, transient tic disorder, rubral tremor, meiotic nondisjunction trisomy 21, mitotic nondisjunction trisomy 21, tuberculosis, tumoral calcinosis, ulcerative colitis, uncinate epilepsy, undifferentiated carcinoma, unilateral hypotonia, unipolar depression, serum quantitative trait locus uric acid concentration 2, serum quantitative trait locus uric acid concentration 4, urolithiasis, vascular diseases, vascular hypertrophy, verbal fluency disorders, vertiginous seizure, visual seizure, withdrawal symptoms, wolf-hirschhorn syndrome, xerocytosis, x-linked csnb, epstein-barr virus infection and neoplasia x-linked immunodeficiency with magnesium defect and neonatal zinc deficiency due to low breast milk zinc.

Preferred diseases include abnormal coordination, abnormality of the cornea, absence seizures, ache, achondrogenesis of type ib (disorder), acidosis, acute lung injury, adenocarcinoma of lung (disorder), age related macular degeneration, Allan-Herndon-Dudley syndrome (ahds), alloxan diabetes, Alzheimer's disease, anasarca, hemolytic anemia, hemolytic acquired anemia, hereditary spherocytic hemolytic anemia, microangiopathic anemia, arthrogryposis, ataxia, appendicular ataxia, motor ataxia, sensory ataxia, truncal ataxia, hereditary ataxias, atelosteogenesis type 2, atherosclerosis, atonic absence seizures, attention deficit hyperactivity disorder (ADHD), atypical ductal breast hyperplasia, aura, autism spectrum disorders, autistic disorder, autosomal recessive polycystic kidney disease, awakening epilepsy, nonobstructive azoospermia, Barrett epithelium, Barrett esophagus, bilateral deafness, biliary cirrhosis, secondary biliary cirrhosis, Birk-Landau-Perez syndrome, developmental bone diseases, breast cancer, breast carcinoma, burn induced multiple organ injury, embryonal cancer, embryonal and mixed cancer, cardiac arrhythmia, cardiac ischaemic reperfusion, cardiomegaly, cataract, cataract 47, juvenile cataract with microcornea and glucosuria, celiac disease, cerebral injury, cervical cancer, child development deviations, specific child development disorders, choledochal cyst, choledochal cyst of type i, choledochal cyst of type ii, choledochal cyst of type iii, choledochal cyst of type iv, choledochal cyst of type v, cholestasis, episodic choreoathetosis/spasticity, chromophobe renal cell carcinoma, cognition disorders, cognitive impairment, collecting duct carcinoma of the kidney, colon cancer, colonic neoplasms, colorectal cancer, complete hearing loss, complex partial seizures, congenital anemia, hearing loss and neurodegeneration congenital cataracts, congenital chloride diarrhea, congenital hypothyroidism, sodium type (disorder) congenital secretory diarrhea, congestive heart failure, constipation, constipation-predominant irritable bowel syndrome, conventional (clear cell) renal cell carcinoma, convulsions, convulsive seizures, corneal dystrophy and perceptive deafness, fuchs endothelial corneal dystrophy 4, corneal endothelial dystrophy 2, corneal opacity, craniofacial abnormalities, stomatin-deficient cryohydrocytosis, cystic fibrosis, cystic fibrosis modifier 1, cytopenia, de la chapelle dysplasia, deaf mutism, deafness, acquired deafness, autosomal recessive deafness 4 with enlarged vestibular aqueduct, autosomal recessive 61 deafness, degenerative polyarthritis, dementia, depression, allergic contact dermatitis, developmental disabilities, diabetes mellitus, experimental diabetes mellitus, non-insulin-dependent diabetes mellitus, diabetic cataract, diarrhea, diastrophic dysplasia, distal renal tubular acidosis, ductal carcinoma, dystonia, dystonia 18 (disorder), ear diseases, edema, elliptocytosis 4, hereditary elliptocytosis, embryonal neoplasm, endometrioma, endometriosis, epilepsy, anterior fronto-polar epilepsy, childhood absence epilepsy 1, cingulate epilepsy, cryptogenic epilepsy, frontal lobe epilepsy, susceptibility to idiopathic generalized epilepsy 12, opercular epilepsy, orbito-frontal epilepsy, supplementary motor epilepsy, epileptic drop attack, epileptic encephalopathy, epileptic seizures, multiple epiphyseal dysplasia 4, erythrocyte lactate transporter defect, familial dementia, familial ménière disease, benign childhood frontal epilepsy, fuchs endothelial dystrophy, gastric cancer, gastrointestinal dysfunction, gastrointestinal neoplasms, generalized absence seizures, generalized seizures, germ cell cancer, germ cell tumor, glaucoma, glioma, glomerular filtration rate, glomerulosclerosis, glut1 deficiency syndrome, autosomal recessive glut1 deficiency syndrome 1, goiter, gustatory seizure, head and neck squamous cell carcinoma, hearing impairment, extreme hearing loss, heart decompensation, heart diseases, heart failure, right-sided heart failure, hematological disease, nonalcoholic fatty liver disease hepatic steatosis, hereditary spherocytosis, Huntington disease, hyperammonaemia, familial hyperinsulinemic hypoglycemia 7, hyperoxaluria, hyperphosphatemia, hypertrophic cardiomyopathy, hypotension, inborn errors of metabolism, inflammatory bowel disease, inflammatory responses in cerebral ischemia and traumatic brain injury, intellectual disability, intestinal obstruction, intracranial hypertension, jacksonian seizure, juvenile arthritis, juvenile psoriatic arthritis, juvenile-onset still disease, kidney calculi, kidney injury, kyphosis deformity of spine, lactic acidosis induced pulmonary vein arrhythmogenesis, lactic acidosis induced sepsis, left-sided heart failure, lens opacities, leukemia, leukodystrophy, lichtenstein-knorr syndrome, lipoidosis, liver cancer, liver carcinoma, experimental liver cirrhosis, liver neoplasms, lung cancer, malaria, malignant mesothelioma, malignant neoplasm of breast, malignant neoplasm of liver, malignant neoplasm of ovary, malignant neoplasm of prostate, malignant tumor of colon, mammary neoplasms, human mammary neoplasms, susceptibility to meconium ileus in cystic fibrosis, melanoma, mental deficiency, south african type mental retardation x-linked, psychosocial mental retardation, x-linked syndromic christianson type mental retardation, metabolic acidosis, microangiopathic hemolytic anemia, microcephaly, microlissencephaly, monocarboxylate transporter 1 deficiency, multiple epiphyseal dysplasia, multiple sclerosis, myocardial failure, myocardial infraction, myoclonic seizures, na,neoplasm metastasis, embryonal and mixed neoplasms, germ cell and embryonal neoplasms, neoplastic cell transformation, nephrocalcinosis, calcium oxalate nephrolithiasis, nervous system disorder, neuropathic pain, non-epileptic convulsion, nonepileptic seizures, noninfiltrating intraductal carcinoma, obesity, olfactory seizure, osteoarthrosis deformans, osteogenesis imperfecta, malaysian-melanesian-filipino type ovalocytosis, ovarian cancer, ovarian neoplasm, oxalosis, pain, burning pain, crushing pain, migratory pain, splitting pain, pancreatic cancer, papillary renal cell carcinoma, pediatric autoimmune disease, pendred's syndrome, peripheral diabetic neuropathy, peritoneal neoplasms, peritoneal surface malignancy, physiological wound healing, polycystic kidney disease, prelingual deafness, primary biliary cirrhosis, primary microcephaly, profound mental retardation, prostate cancer, prostatic neoplasms, pseudoaphakia, pseudohyperkalemia cardiff, radiating pain, renal cell carcinoma, renal tubular acidosis, distal renal tubular acidosis with hemolytic anemia, distal renal tubular acidosis with normal red cell morphology, proximal renal tubular acidosis with ocular abnormalities and mental retardation, renal tubular acidosis of type ii, reperfusion injury, sarcomatoid renal cell carcinoma, schizophrenia, seizures, auditory seizures, clonic seizures, focal seizures, sensory seizures, somatosensory seizures, senile paranoid dementia, sensorineural hearing loss (disorder), sensory hearing loss, severe congenital microcephaly, hemorrhagic shock, single seizure, sleep quality, southeast asian ovalocytosis, autosomal dominant spastic paraplegia 42, hereditary spastic paraplegia, type 4 spherocytosis, stomach carcinoma, streptozotocin diabetes, stroke, physical suffering, thyroid agenesis, thyroid hormone resistance syndrome, thyroid hypoplasia, tissue fibrosis, tonic - clonic seizures, tonic seizures, rubral tremor, ulcerative colitis, urolithiasis, vascular hypertrophy, vertiginous seizure, visual seizure and xerocytosis.

Even more preferred diseases include abnormal coordination, absence seizures, ache, acidosis, acute lung injury, adenocarcinoma of lung (disorder), alloxan diabetes, Alzheimer's disease, anasarca, arthrogryposis, ataxia, appendicular ataxia, motor ataxia, sensory ataxia, truncal ataxia, hereditary ataxias, atherosclerosis, atonic absence seizures, attention deficit hyperactivity disorder (ADHD), atypical ductal breast hyperplasia, autistic disorder, Barrett esophagus, Birk-Landau-Perez syndrome, breast cancer, burn induced multiple organ injury, embryonal cancer, embryonal and mixed cancer, cardiac arrhythmia, cardiac ischaemic reperfusion, cardiomegaly, cataract, celiac disease, cerebral injury, cervical cancer, child development deviations, specific child development disorders, chromophobe renal cell carcinoma, cognitive impairment, collecting duct carcinoma of the kidney, colon cancer, colonic neoplasms, complex partial seizures, congenital anemia, congenital cataracts, hearing loss, and neurodegeneration, sodium type (disorder) congenital secretory diarrhea, congestive heart failure, constipation, constipation-predominant irritable bowel syndrome, conventional (clear cell) renal cell carcinoma, convulsions, convulsive seizures, corneal opacity, cryohydrocytosis, stomatin-deficient, with mental retardation, seizures, cataracts, and massive hepatosplenomegaly, cystic fibrosis, cytopenia, degenerative polyarthritis, dementia, depression, allergic contact dermatitis, developmental disabilities, diabetes mellitus, diabetic cataract, diarrhea, distal renal tubular acidosis, ductal carcinoma, dystonia, edema, embryonal neoplasm, epilepsy, epileptic drop attack, epileptic encephalopathy, epileptic seizures, erythrocyte lactate transporter defect, familial dementia, gastric cancer, gastrointestinal dysfunction, generalized absence seizures, generalized seizures, germ cell cancer, germ cell tumor, glaucoma, glioma, glomerular filtration rate, glomerulosclerosis, glut1 deficiency syndrome, gustatory seizure, head and neck squamous cell carcinoma, hearing impairment, heart decompensation, heart failure, right-sided heart failure, nonalcoholic fatty liver disease hepatic steatosis, Huntington disease, hyperammonaemia, hyperinsulinemic hypoglycemia, familial, 7, hyperphosphatemia, hypertrophic cardiomyopathy, hypotension, inborn errors of metabolism, inflammatory bowel disease, inflammatory responses in cerebral ischemia and traumatic brain injury, intellectual disability, intestinal obstruction, jacksonian seizure, juvenile arthritis, juvenile psoriatic arthritis, juvenile-onset still disease, kidney injury, lactic acidosis induced pulmonary vein arrhythmogenesis, lactic acidosis induced sepsis, left-sided heart failure, lens opacities, leukemia, lichtenstein-knorr syndrome, lipoidosis, liver cancer, experimental liver cirrhosis, liver neoplasms, lung cancer, malignant mesothelioma, malignant neoplasm of breast, malignant neoplasm of liver, malignant neoplasm of ovary, malignant tumor of colon, mammary neoplasms, human mammary neoplasms, susceptibility to meconium ileus in cystic fibrosis, melanoma, mental deficiency, south african type mental retardation x-linked, psychosocial mental retardation, mental retardation, x-linked, syndromic, christianson type, metabolic acidosis, microcephaly, microlissencephaly, monocarboxylate transporter 1 deficiency, multiple sclerosis, myocardial failure, myocardial infraction, myoclonic seizures, neoplasm metastasis, embryonal and mixed neoplasms, germ cell and embryonal neoplasms, neoplastic cell transformation, nervous system disorder, neuropathic pain, non-epileptic convulsion, nonepileptic seizures, noninfiltrating intraductal carcinoma, obesity, olfactory seizure, osteoarthrosis deformans, ovarian cancer, ovarian neoplasm, pain, burning pain, crushing pain, migratory pain, splitting pain, pancreatic cancer, papillary renal cell carcinoma, pediatric autoimmune disease, peripheral diabetic neuropathy, peritoneal neoplasms, peritoneal surface malignancy, physiological wound healing, polycystic kidney disease, primary microcephaly, profound mental retardation, prostate cancer, pseudoaphakia, radiating pain, renal cell carcinoma, renal cell carcinoma, renal tubular acidosis, renal tubular acidosis, proximal, with ocular abnormalities and mental retardation, type ii renal tubular acidosis, reperfusion injury, sarcomatoid renal cell carcinoma, schizophrenia, seizures, auditory seizures, clonic seizures, focal seizures, sensory seizures, somatosensory seizures, senile paranoid dementia, severe congenital microcephaly, hemorrhagic shock, single seizure, sleep quality, autosomal dominant spastic paraplegia 42, hereditary spastic paraplegia, stomach carcinoma, streptozotocin diabetes, stroke, physical suffering, tissue fibrosis, tonic - clonic seizures, tonic seizures, rubral tremor, vascular hypertrophy, vertiginous seizure and visual seizure.

Diseases related to SLC9 proteins include in particular attention deficit hyperactivity disorder (ADHD), acute lung injury, Alzheimer's disease, atherosclerosis, autism, Barrett's oesophagus, brain cancer (e.g. glioma), breast cancer, burn induced multiple organ injury, cardiac arrhythmia, cardiac ischaemic reperfusion, cardiomegaly, celiac disease, cerebral injury, cervical cancer, cognitive impairment, colon cancer, constipation, constipation-predominant irritable bowel syndrome, cystic fibrosis, depression, diabetes, diabetic cataract, epilepsy, gastric cancer, gastrointestinal dysfunction, glomerular filtration rate, glomerulosclerosis, head and neck squamous cell carcinoma, nonalcoholic fatty liver disease hepatic steatosis, hyperammonaemia, hyperphosphatemia, inflammatory bowel disease, inflammatory responses in cerebral ischemia and traumatic brain injury, kidney injury, lactic acidosis induced pulmonary vein arrhythmogenesis, lactic acidosis induced sepsis, leukemia, liver cancer, melanoma, multiple sclerosis, myocardial infraction, neoplasm metastasis, neuropathic pain, ovarian cancer, pediatric autoimmune disease, peripheral diabetic neuropathy, physiological wound healing, polycystic kidney disease, prostate cancer, seizures, sleep quality, stomach carcinoma, stroke, tissue fibrosis, vascular hypertrophy, intellectual disability, ataxia, microcephaly, lung cancer, renal cell carcinoma and pancreatic cancer.

Preferred examples of diseases related to SLC9 proteins include Alzheimer's disease, atherosclerosis, autism, brain cancer (e.g. glioma), breast cancer, cardiac arrhythmia, cardiac ischaemic reperfusion, celiac disease, cervical cancer, colon cancer, diabetes, epilepsy, gastric cancer, gastrointestinal dysfunction, head and neck squamous cell carcinoma, nonalcoholic fatty liver disease hepatic steatosis, inflammatory bowel disease, intellectual disability, lactic acidosis, leukemia, liver cancer, lung cancer, melanoma, myocardial infraction, neoplasm metastasis, ovarian cancer, pancreatic cancer, prostate cancer, renal cell carcinoma, seizures, stomach carcinoma, stroke, tissue fibrosis and vascular hypertrophy.

More preferred examples of diseases related to SLC9 proteins include atherosclerosis, breast cancer, cardiac arrhythmia, cardiac ischaemic reperfusion, cervical cancer, colon cancer, gastric cancer, glioma, head and neck squamous cell carcinoma, leukemia, liver cancer, lung cancer, melanoma, myocardial infraction, neoplasm metastasis, ovarian cancer, pancreatic cancer, prostate cancer, renal cell carcinoma, stomach carcinoma, stroke, tissue fibrosis and vascular hypertrophy.

In context of the invention, the composition may comprise a pharmaceutically acceptable diluent, excipient or carrier. The term "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the Federal or a state government or listed in the U.S. Pharmacopeia or other generally recognized pharmacopeia for use in mammals, and more particularly in humans. A "pharmaceutically acceptable carrier" refers to an ingredient in a pharmaceutical composition or formulation, other than an active ingredient, which is nontoxic to a subject. A pharmaceutically acceptable carrier includes, but is not limited to, a buffer, excipient, stabilizer, or preservative. Such pharmaceutically acceptable carriers may be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by A.R. Gennaro, 20th Edition.

In one embodiment, the composition may be for use in treating or preventing cancer. The term "cancer" as used herein refers to any malignant tumor in the aforementioned tissue and cell type. Examples of the cancer may include a cancer which can be caused by an abnormal adherent cell, or a cancer which can be caused by an abnormal blood cell (e.g., leukemia, lymphoma, multiple myeloma). Specifically, examples of the cancer which can be caused by the abnormal adherent cell may include a lung cancer (e.g. squamous cell carcinoma, non-small cell carcinoma such as adenocarcinoma and large cell carcinoma, and small cell carcinoma), a gastrointestinal cancer (e.g., stomach cancer, small intestine cancer, large intestine cancer, rectal cancer), a pancreatic cancer, a renal cancer, a hepatic cancer, a thymic cancer, a spleen cancer, a thyroid cancer, an adrenal cancer, a prostate cancer, an urinary bladder cancer, an ovarian cancer, an uterus cancer (e.g., endometrial carcinoma, cervical cancer), a bone cancer, a skin cancer, a brain tumor, a sarcoma, a melanoma, a blastoma (e.g., neuroblastoma), an adenocarcinoma, a planocellular cancer, a solid cancer, an epithelial cancer, and a mesothelioma. Particularly, the cancer may be leukemia, particularly acute myeloid leukemia (AML) and B-cell acute lymphoblastic leukemia (B-ALL) a chronic leukemia, such as chronic myeloid leukemia; adenoid cystic carcinoma; osteosarcoma; ovarian cancer; Ewings sarcoma; lung adenocarcinoma and prostate cancer; lymphoma, neuroblastoma, gastrointestinal cancers, endometrial cancers, medulloblastoma, prostate cancers, esophagus cancer, breast cancer, thyroid cancer, meningioma, liver cancer, colorectal cancer, pancreatic cancer, chondrosarcoma, osteosarcoma, kidney cancer, particularly the cancer may be leukemia. In one embodiment, the composition may be for use in treating or preventing cancer, particularly leukemia such as chronic myeloid leukemia. In one embodiment, the present invention further may relate to the compound of the present invention for use in treating cancer wherein the chemical compound or agent is to be administered to a patient having cancer.

The compound or composition of the invention may be used for the treatment or prevention of cancer. For example, the composition or compound of the present invention may be used for the treatment of cancer (without being limiting) in particular in the treatment of lymphoma, particularly Hodgkin's lymphoma and non-Hodgkin's lymphoma such as B cell lymphoma; leukemia such as acute myeloid leukemia (AML) or chronic lymphocytic leukemia (CLL); and/or myeloma such as multiple myeloma. This is also illustrated in the appended Examples.

A "patient" or "individual" or "subject" is a mammal. Mammals include, but are not limited to, domesticated animals *(e.g.,* cows, sheep, cats, dogs, and horses), primates *(e.g.,* humans and non-human primates such as monkeys), rabbits, and rodents (e.g., mice and rats). In certain aspects, the patient, individual, or subject is a human. In one embodiment, the patient may be a "cancer patient," *i.e.* one who is suffering or at risk for suffering from one or more symptoms of cancer.

It will be understood that the specific dose level for any particular patient will depend upon a variety of factors including the activity of the specific compound employed, the age, body weight, general health, sex, diet, time of administration, route of administration, rate of excretion, drug combination and the causative mechanism and severity of the particular disease undergoing therapy.

The compounds of formula compounds of formulae (I) can be prepared by methods known in the field of synthetic chemistry and as described in the experimental examples of the present application.

The scope of the invention embraces the compounds provided herein, particularly the compounds of formula compounds of formulae (I), in any solvated form, including, e.g., solvates with water (i.e., as a hydrate) or solvates with organic solvents such as, e.g., methanol, ethanol or acetonitrile (i.e., as a methanolate, ethanolate or acetonitrilate), or in any crystalline form (i.e., as any polymorph), or in amorphous form. It is to be understood that such solvates of the compounds provided herein, particularly the compounds of formula (I), also include solvates of pharmaceutically acceptable salts of the corresponding compounds.

Moreover, the compounds provided herein, particularly the compounds of formula compounds of formulae (I), may exist in the form of different isomers, in particular stereoisomers (including, e.g., geometric isomers (or cis/trans isomers), enantiomers and diastereomers) or tautomers. All such isomers of the compounds provided herein are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds provided herein.

The scope of the invention also embraces the compounds provided herein, particularly the compounds of formula compounds of formulae (I), in which one or more atoms are replaced by a specific isotope of the corresponding atom. For example, the invention encompasses compounds of formula compounds of formulae (I), in which one or more hydrogen atoms (or, e.g., all hydrogen atoms) are replaced by deuterium atoms (i.e., ²H; also referred to as "D"). Accordingly, the invention also embraces compounds of formula compounds of formulae (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula compounds of formulae (I) or a reactant or precursor to be used in the synthesis of the compound of formula compounds of formulae (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; or Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces the compounds provided herein, particularly the compounds of formula compounds of formula (I), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers or imaging probes in positron emission tomography (PET). The invention thus includes (i) compounds of formula compounds of formulae (I), in which one or more fluorine atoms (or, e.g., all fluorine atoms) are replaced by ¹⁸F atoms, (ii) compounds of formula compounds of formulae (I), in which one or more carbon atoms (or, e.g., all carbon atoms) are replaced by ¹¹C atoms, (iii) compounds of formula compounds of formulae (I), in which one or more nitrogen atoms (or, e.g., all nitrogen atoms) are replaced by ¹³N atoms, (iv) compounds of formula compounds of formulae (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula compounds of formulae (I), in which one or more bromine atoms (or, e.g., all bromine atoms) are replaced by ⁷⁷Br atoms, (vii) compounds of formula compounds of formulae (I), in which one or more iodine atoms (or, e.g., all iodine atoms) are replaced by ¹²⁰I atoms, and (viii) compounds of formula compounds of formulae (I), in which one or more iodine atoms (or, e.g., all iodine atoms) are replaced by ¹²⁴I atoms. **In** general, it is preferred that none of the atoms in the compounds of formula compounds of formulae (I) are replaced by specific isotopes.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₅ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₅ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more carbon-to-carbon double bonds. The term "C₂₋₅ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₅ alkylene" denotes an alkylene group having 1 to 5 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or - CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "alkoxy" refers to an -O-alkyl group, wherein the alkyl moiety comprised in this group is as defined above.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "cyclyl" refers to a carbocyclyl or a heterocyclyl, as defined herein above.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, the term "heteroaryl" particularly preferably refers to pyridinyl (e.g., 2-pyridyl, 3-pyridyl, or 4-pyridyl), imidazolyl, thiazolyl, 1H-tetrazolyl, 2H-tetrazolyl, thienyl (i.e., thiophenyl), or pyrimidinyl.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members. Moreover, unless defined otherwise, the term "cycloalkyl" even more preferably refers to cyclohexyl or cyclopropyl, and yet even more preferably refers to cyclohexyl.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, "heterocycloalkyl" even more preferably refers to tetrahydropyranyl, piperidinyl, piperazinyl, morpholinyl, pyrrolidinyl, or tetrahydrofuranyl.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-Cl), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of formula (I) may be attached to the remainder of the respective compound via a number of different positions of the corresponding specific substituent group. Unless defined otherwise, the preferred attachment positions for the various specific substituent groups are as illustrated in the examples.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor^{®} HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

A proposed, yet non-limiting dose of the compounds according to the invention for oral administration to a human (of approximately 70 kg body weight) may be 0.05 to 8000 mg, preferably 0.1 mg to 4000 mg, of the active ingredient per unit dose. The unit dose may be administered, e.g., 1 to 3 times per day. The unit dose may also be administered 1 to 7 times per week, e.g., with not more than one administration per day. A further exemplary dose of the compounds of formula (I) for oral administration to a human is 50 to 200 mg/kg bodyweight/day, particularly 100 mg/kg/day. It will be appreciated that it may be necessary to make routine variations to the dosage depending on the age and weight of the patient/subject as well as the severity of the condition to be treated. The precise dose and also the route of administration will ultimately be at the discretion of the attendant physician or veterinarian.

The compounds provided herein, particularly the compound of formula (I), or a pharmaceutical composition comprising such a compound can be administered in monotherapy (e.g., without concomitantly administering any further therapeutic agents, or without concomitantly administering any further therapeutic agents against the same disease that is to be treated or prevented with the compound of formula (I)). However, the compound of formula (I) or a pharmaceutical composition comprising the compound of formula (I) can also be administered in combination with one or more further therapeutic agents. If the compound of formula (I) is used in combination with a second therapeutic agent active against the same disease or condition, the dose of each compound may differ from that when the corresponding compound is used alone, in particular, a lower dose of each compound may be used. The combination of the compound of formula (I) with one or more further therapeutic agents (such as, e.g., a BRD4 inhibitor, preferably a direct BRD4 inhibitor) may comprise the simultaneous/concomitant administration of the compound of formula (I) and the further therapeutic agent(s) (either in a single pharmaceutical formulation or in separate pharmaceutical formulations), or the sequential/separate administration of the compound of formula (I) and the further therapeutic agent(s). If administration is sequential, either the compound of formula (I) according to the invention or the one or more further therapeutic agents may be administered first. If administration is simultaneous, the one or more further therapeutic agents may be included in the same pharmaceutical formulation as the compound of formula (I), or they may be administered in one or more different (separate) pharmaceutical formulations.

Preferably, the one or more further therapeutic agents to be administered in combination with a compound of the present invention are anticancer drugs. The anticancer drug(s) to be administered in combination with a compound of formula (I) according to the invention may, e.g., be selected from: a tumor angiogenesis inhibitor (e.g., a protease inhibitor, an epidermal growth factor receptor kinase inhibitor, or a vascular endothelial growth factor receptor kinase inhibitor); a cytotoxic drug (e.g., an antimetabolite, such as purine and pyrimidine analog antimetabolites); an antimitotic agent (e.g., a microtubule stabilizing drug or an antimitotic alkaloid); a platinum coordination complex; an anti-tumor antibiotic; an alkylating agent (e.g., a nitrogen mustard or a nitrosourea); an endocrine agent (e.g., an adrenocorticosteroid, an androgen, an anti-androgen, an estrogen, an anti-estrogen, an aromatase inhibitor, a gonadotropin-releasing hormone agonist, or a somatostatin analog); or a compound that targets an enzyme or receptor that is overexpressed and/or otherwise involved in a specific metabolic pathway that is misregulated in the tumor cell (e.g., ATP and GTP phosphodiesterase inhibitors, histone deacetylase inhibitors, protein kinase inhibitors (such as serine, threonine and tyrosine kinase inhibitors, e.g., Abelson protein tyrosine kinase inhibitors) and the various growth factors, their receptors and corresponding kinase inhibitors (such as epidermal growth factor receptor kinase inhibitors, vascular endothelial growth factor receptor kinase inhibitors, fibroblast growth factor inhibitors, insulin-like growth factor receptor inhibitors and platelet-derived growth factor receptor kinase inhibitors)); methionine, aminopeptidase inhibitors, proteasome inhibitors, cyclooxygenase inhibitors (e.g., cyclooxygenase-1 or cyclooxygenase-2 inhibitors), topoisomerase inhibitors (e.g., topoisomerase I inhibitors or topoisomerase II inhibitors), poly ADP ribose polymerase inhibitors (PARP inhibitors), and epidermal growth factor receptor (EGFR) inhibitors/antagonists.

An alkylating agent which can be used as an anticancer drug in combination with a compound of the present invention may be, for example, a nitrogen mustard (such as cyclophosphamide, mechlorethamine (chlormethine), uramustine, melphalan, chlorambucil, ifosfamide, bendamustine, or trofosfamide), a nitrosourea (such as carmustine, streptozocin, fotemustine, lomustine, nimustine, prednimustine, ranimustine, or semustine), an alkyl sulfonate (such as busulfan, mannosulfan, or treosulfan), an aziridine (such as hexamethylmelamine (altretamine), triethylenemelamine, ThioTEPA (N,N'N'-triethylenethiophosphoramide), carboquone, or triaziquone), a hydrazine (such as procarbazine), a triazene (such as dacarbazine), or an imidazotetrazine (such as temozolomide).

A platinum coordination complex which can be used as an anticancer drug in combination with a compound of the present invention may be, for example, cisplatin, carboplatin, nedaplatin, oxaliplatin, satraplatin, or triplatin tetranitrate.

A cytotoxic drug which can be used as an anticancer drug in combination with a compound of the present invention may be, for example, an antimetabolite, including folic acid analogue antimetabolites (such as aminopterin, methotrexate, pemetrexed, or raltitrexed), purine analogue antimetabolites (such as cladribine, clofarabine, fludarabine, 6-mercaptopurine (including its prodrug form azathioprine), pentostatin, or 6-thioguanine), and pyrimidine analogue antimetabolites (such as cytarabine, decitabine, 5-fluorouracil (including its prodrug forms capecitabine and tegafur), floxuridine, gemcitabine, enocitabine, or sapacitabine).

An antimitotic agent which can be used as an anticancer drug in combination with a compound of the present invention may be, for example, a taxane (such as docetaxel, larotaxel, ortataxel, paclitaxel/taxol, tesetaxel, or nab-paclitaxel (e.g., Abraxane^{®})), a Vinca alkaloid (such as vinblastine, vincristine, vinflunine, vindesine, or vinorelbine), an epothilone (such as epothilone A, epothilone B, epothilone C, epothilone D, epothilone E, or epothilone F) or an epothilone B analogue (such as ixabepilone/azaepothilone B).

An anti-tumor antibiotic which can be used as an anticancer drug in combination with a compound of the present invention may be, for example, an anthracycline (such as aclarubicin, daunorubicin, doxorubicin, epirubicin, idarubicin, amrubicin, pirarubicin, valrubicin, or zorubicin), an anthracenedione (such as mitoxantrone, or pixantrone) or an anti-tumor antibiotic isolated from Streptomyces (such as actinomycin (including actinomycin D), bleomycin, mitomycin (including mitomycin C), or plicamycin).

A tyrosine kinase inhibitor which can be used as an anticancer drug in combination with a compound of the present invention may be, for example, axitinib, bosutinib, cediranib, dasatinib, erlotinib, gefitinib, imatinib, lapatinib, lestaurtinib, nilotinib, semaxanib, sorafenib, sunitinib, axitinib, nintedanib, ponatinib, or vandetanib.

A topoisomerase inhibitor which can be used as an anticancer drug in combination with a compound of the present invention may be, for example, a topoisomerase I inhibitor (such as irinotecan, topotecan, camptothecin, belotecan, rubitecan, or lamellarin D) or a topoisomerase II inhibitor (such as amsacrine, etoposide, etoposide phosphate, teniposide, or doxorubicin).

A PARP inhibitor which can be used as an anticancer drug in combination with a compound of the present invention may be, for example, BMN-673, olaparib, rucaparib, veliparib, CEP 9722, MK 4827, BGB-290, or 3-aminobenzamide.

An EGFR inhibitor/antagonist which can be used as an anticancer drug in combination with a compound of the present invention may be, for example, gefitinib, erlotinib, lapatinib, afatinib, neratinib, ABT-414, dacomitinib, AV-412, PD 153035, vandetanib, PKI-166, pelitinib, canertinib, icotinib, poziotinib, BMS-690514, CUDC-101, AP26113, XL647, cetuximab, panitumumab, zalutumumab, nimotuzumab, or matuzumab.

Further anticancer drugs may also be used in combination with a compound of the present invention. The anticancer drugs may comprise biological or chemical molecules, like TNF-related apoptosis-inducing ligand (TRAIL), tamoxifen, amsacrine, bexarotene, estramustine, irofulven, trabectedin, cetuximab, panitumumab, tositumomab, alemtuzumab, bevacizumab, edrecolomab, gemtuzumab, alvocidib, seliciclib, aminolevulinic acid, methyl aminolevulinate, efaproxiral, porfimer sodium, talaporfin, temoporfin, verteporfin, alitretinoin, tretinoin, anagrelide, arsenic trioxide, atrasentan, bortezomib, carmofur, celecoxib, demecolcine, elesclomol, elsamitrucin, etoglucid, lonidamine, lucanthone, masoprocol, mitobronitol, mitoguazone, mitotane, oblimersen, omacetaxine, sitimagene, ceradenovec, tegafur, testolactone, tiazofurine, tipifarnib, vorinostat, or iniparib.

Also biological drugs, like antibodies, antibody fragments, antibody constructs (for example, single-chain constructs), and/or modified antibodies (like CDR-grafted antibodies, humanized antibodies, "full humanized" antibodies, etc.) directed against cancer or tumor markers/factors/cytokines involved in proliferative diseases can be employed in cotherapy approaches with the compounds of the invention. Examples of such biological molecules are anti-HER2 antibodies (e.g. trastuzumab, Herceptin^{®}), anti-CD20 antibodies (e.g. Rituximab, Rituxan^{®}, MabThera^{®}, Reditux^{®}), anti-CD19/CD3 constructs (see, e.g., EP1071752) and anti-TNF antibodies (see, e.g., Taylor PC. Antibody therapy for rheumatoid arthritis. Curr Opin Pharmacol. 2003. 3(3):323-328). Further antibodies, antibody fragments, antibody constructs and/or modified antibodies to be used in cotherapy approaches with the compounds of the invention can be found, e.g., in: Taylor PC. Curr Opin Pharmacol. 2003. 3(3):323-328; or Roxana A. Maedica. 2006. 1(1):63-65.

An anticancer drug which can be used in combination with a compound of the present invention may, in particular, be an immunooncology therapeutic (such as an antibody (e.g., a monoclonal antibody or a polyclonal antibody), an antibody fragment, an antibody construct (e.g., a single-chain construct), or a modified antibody (e.g., a CDR-grafted antibody, a humanized antibody, or a "full humanized" antibody) targeting any one of CTLA-4, PD-1/PD-L1, TIM3, LAG3, OX4, CSF1R, IDO, or CD40. Such immunooncology therapeutics include, e.g., an anti-CTLA-4 antibody (particularly an antagonistic or pathway-blocking anti-CTLA-4 antibody; e.g., ipilimumab or tremelimumab), an anti-PD-1 antibody (particularly an antagonistic or pathway-blocking anti-PD-1 antibody; e.g., nivolumab (BMS-936558), pembrolizumab (MK-3475), pidilizumab (CT-011), AMP-224, or APE02058), an anti-PD-L1 antibody (particularly a pathway-blocking anti-PD-L1 antibody; e.g., BMS-936559, MEDI4736, MPDL3280A (RG7446), MDX-1105, or MEDI6469), an anti-TIM3 antibody (particularly a pathway-blocking anti-TIM3 antibody), an anti-LAG3 antibody (particularly an antagonistic or pathway-blocking anti-LAG3 antibody; e.g., BMS-986016, IMP701, or IMP731), an anti-OX4 antibody (particularly an agonistic anti-OX4 antibody; e.g., MEDI0562), an anti-CSF1R antibody (particularly a pathway-blocking anti-CSF1R antibody; e.g., IMC-CS4 or RG7155), an anti-IDO antibody (particularly a pathway-blocking anti-IDO antibody), or an anti-CD40 antibody (particularly an agonistic anti-CD40 antibody; e.g., CP-870,893 or Chi Lob 7/4). Further immunooncology therapeutics are known in the art and are described, e.g., in: Kyi C et al., FEBS Lett, 2014, 588(2):368-76; Intlekofer AM et al., J Leukoc Biol, 2013, 94(1):25-39; Callahan MK et al., J Leukoc Biol, 2013, 94(1):41-53; Ngiow SF et al., Cancer Res, 2011, 71(21):6567-71; and Blattman JN et al., Science, 2004, 305(5681):200-5.

A BRD4 inhibitor (preferably a direct BRD4 inhibitor), such as CeMMEC2, may also be used as a further therapeutic agent in combination with the compound of formula (I).

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the compound of the present invention (particularly the compound of formula (I) or a pharmaceutically acceptable salt or solvate thereof) or the further therapeutic agent(s) may be administered first. When administration is simultaneous, the combination may be administered either in the same pharmaceutical composition or in different pharmaceutical compositions. When combined in the same formulation, it will be appreciated that the two or more compounds must be stable and compatible with each other and the other components of the formulation. When formulated separately, they may be provided in any convenient formulation.

The compounds provided herein, particularly the compounds of formula (I), can also be administered in combination with physical therapy, such as radiotherapy. Radiotherapy may commence before, after, or simultaneously with administration of the compounds of the invention. For example, radiotherapy may commence 1-10 minutes, 1-10 hours or 24-72 hours after administration of the compounds. Yet, these time frames are not to be construed as limiting. The subject is exposed to radiation, preferably gamma radiation, whereby the radiation may be provided in a single dose or in multiple doses that are administered over several hours, days and/or weeks. Gamma radiation may be delivered according to standard radiotherapeutic protocols using standard dosages and regimens.

The present invention thus relates to a compound of formula (I) or a pharmaceutically acceptable salt or solvate thereof, or a pharmaceutical composition comprising any of the aforementioned entities in combination with a pharmaceutically acceptable excipient, for use in the treatment or prevention of cancer, wherein the compound or the pharmaceutical composition is to be administered in combination with one or more anticancer drugs and/or in combination with radiotherapy.

Yet, the compounds of formula (I) can also be used in monotherapy, particularly in the monotherapeutic treatment or prevention of cancer (i.e., without administering any other anticancer agents until the treatment with the compound(s) of formula (I) is terminated). Accordingly, the invention also relates to a compound of formula (I) or a pharmaceutically acceptable salt or solvate thereof, or a pharmaceutical composition comprising any of the aforementioned entities in combination with a pharmaceutically acceptable excipient, for use in the monotherapeutic treatment or prevention of cancer.

The compound of the present invention is preferably for use in the treatment of a subject or patient selected from an animal (e.g., a non-human animal), a vertebrate animal, a mammal, a rodent (e.g., a guinea pig, a hamster, a rat, or a mouse), a canine (e.g., a dog), a feline (e.g., a cat), a porcine (e.g., a pig), an equine (e.g., a horse), a primate or a simian (e.g., a monkey or an ape, such as a marmoset, a baboon, a gorilla, a chimpanzee, an orangutan, or a gibbon), or a human. The compound of the present invention is more preferably for use in the treatment of animals which are economically, agronomically or scientifically important. Scientifically important organisms include, but are not limited to, mice, rats, and rabbits. Lower organisms such as, e.g., fruit flies like *Drosophila melagonaster* and nematodes like *Caenorhabditis elegans* may also be used in scientific approaches. Non-limiting examples of agronomically important animals are sheep, cattle and pigs, while, for example, cats and dogs may be considered as economically important animals. Preferably, the subject/patient is a mammal. More preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient is a human.

It will be understood that the invention disclosed and defined herein extends to all alternative combinations of two or more of the individual features mentioned or evident from the text or drawings. All of these different combinations constitute various alternative aspects of the invention.

In this specification, a number of documents including patent applications, scientific literature and manufacturers' manuals are cited.

As used herein, unless explicitly indicated otherwise or contradicted by context, the terms "a", "an" and "the" are used interchangeably with "one or more" and "at least one". Thus, for example, a composition comprising "a" compound of formula compounds of formulae (I) can be interpreted as referring to a composition comprising "one or more" compounds of formula compounds of formulae (I).

As used herein, the term "comprising" (or "comprise", " "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

Moreover, unless indicated otherwise, any reference to an industry standard, a pharmacopeia, or a manufacturer's manual refers to the corresponding latest version that was available at the priority date (i.e., at the earliest filing date) of the present specification.

The following Figures and Examples further illustrate the present invention but should not be construed to limit the scope thereof.

### FIGURES

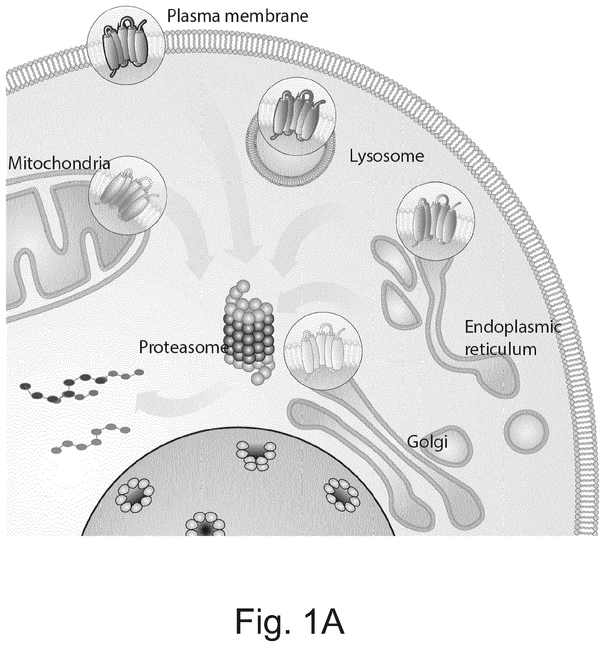

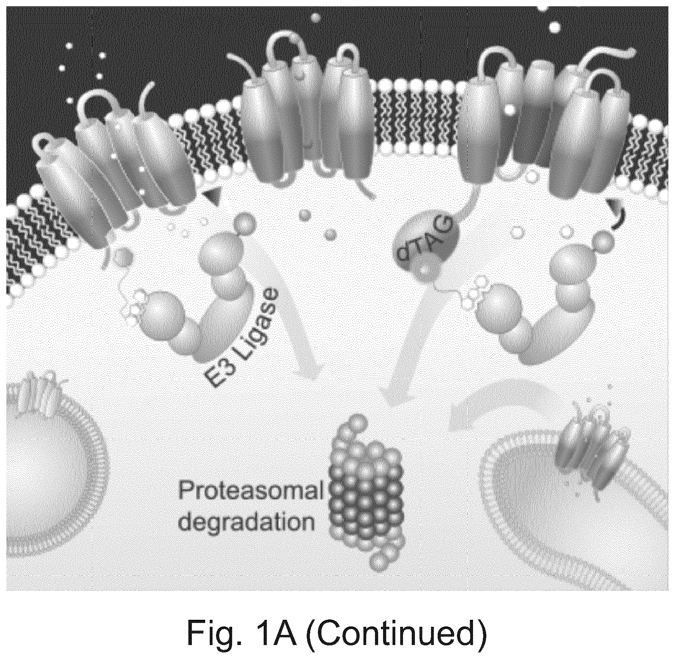

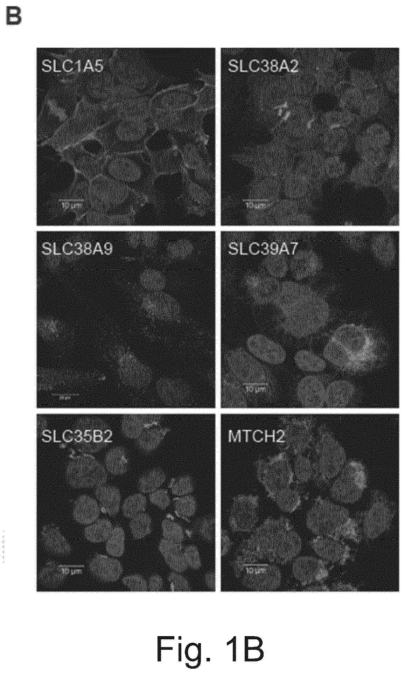

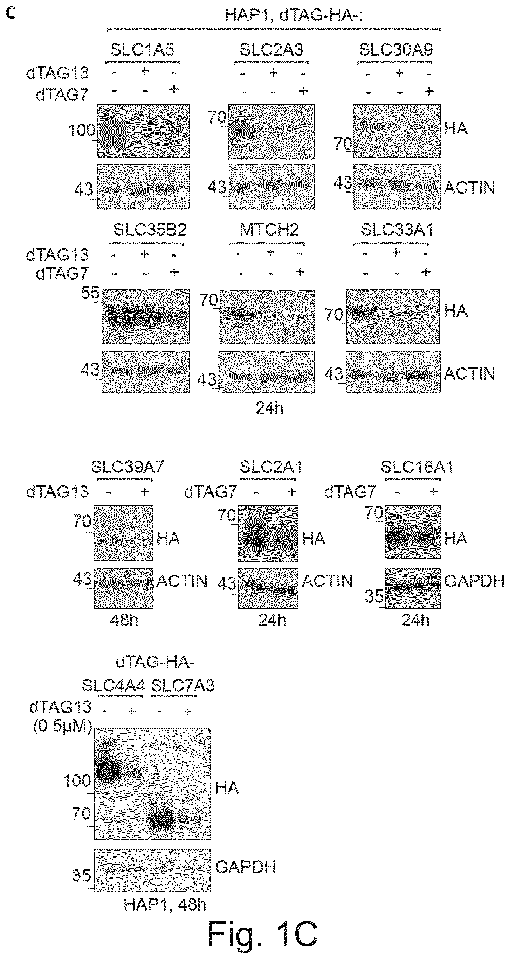

### Figure 1: Amenability of SLCs to targeted degradation

Exogenous expression of solute carriers (SLCs) tagged by FKBP12F36V ("dTAG") demonstrates amenability to targeted degradation in different cellular compartments:
(A) Upper graph - Illustration representing SLC proteins from different subcellular locations (plasma membrane, Lysosome, mitochondria, Golgi, endoplasmic reticulum) were selected for testing their amenability to targeted degradation
   Lower graph - Endogenous expression of solute carriers (SLCs) (e.g., physiologically expressed as shown on the left) and tagged by FKBP12F36V ("dTAG") (tagged SLC as described in the Examples and shown on the right). Degradation of untagged SLCs in different cellular compartments (exemplarily shown for degradation of SLCs in the plasma membrane) by compounds of the present invention targeting physiologically expressed SLCs.
(B) Immunofluorescence (αHA) imaging of the indicated dTAG-HA SLCs was carried out to confirm subcellular localization
(C)HAP1 cells expressing dTAG-HA SLC1A5, 2A3, 30A9, 35B2, MTCH2, and 33A1 were treated for 24 hours with 0.5 µM of dTAG7 or dTAG13, as indicated. HAP1 cells expressing dTAG-HA SLC2A1 and 16A1 were treated for 24 hrs with 0.5 µM of dTAG7. HAP1 cell lines expressing dTAG-HA SLC39A7, dTAG-HA SLC4A4, and SLC7A3-dTAG-HA were treated for 48 hrs with 0.5 µM of dTAG13.

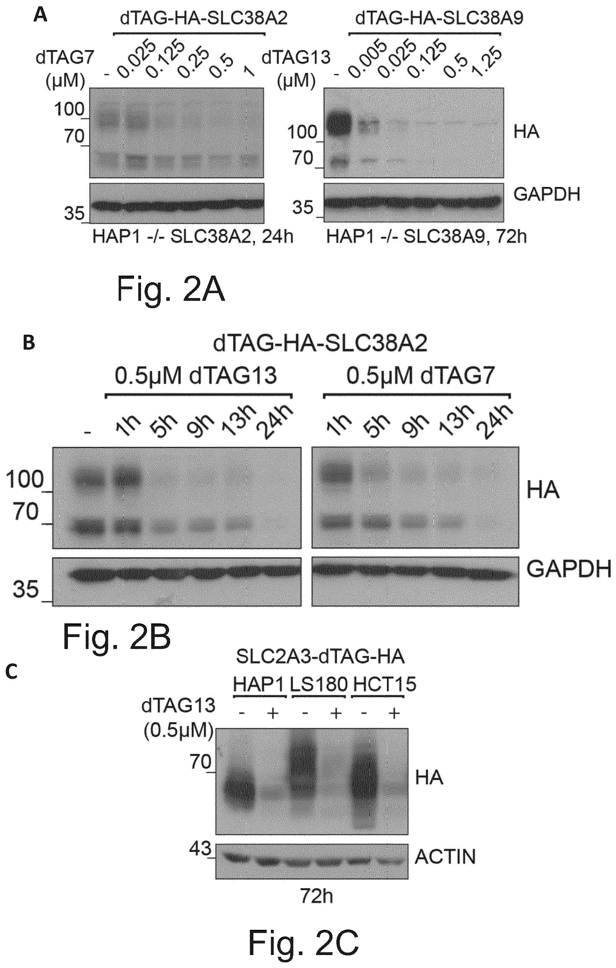

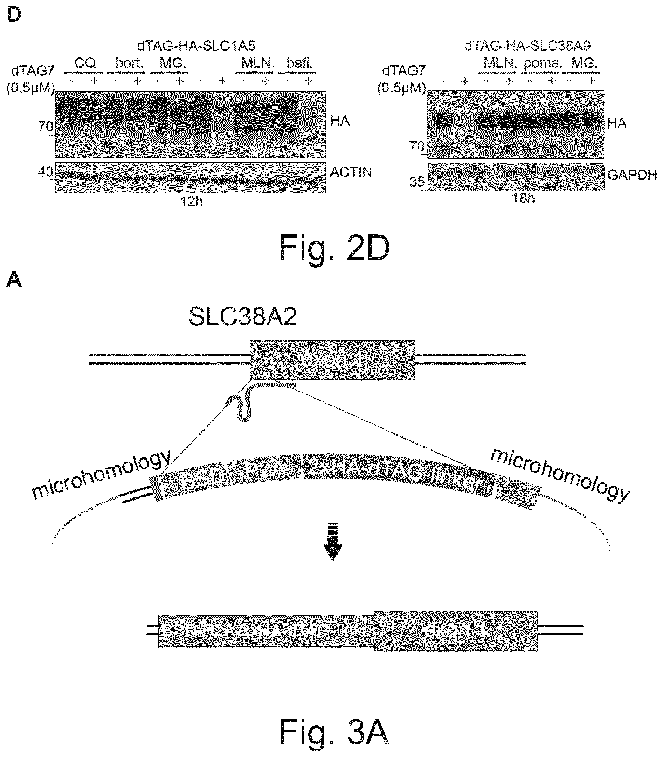

### Figure 2: Characteristics of targeted degradation of SLCs by dTAG system

(A) A range of the degrader molecule concentrations was tested in a cell line reconstituted to express only dTAG-HA SLC38A2 or 38A9. Close to complete degradation can be achieved at low nanomolar range for these example SLCs.
(B) A time course of dTAG driven SLC degradation. A HAP1 cell line expressing dTAGHA SLC38A2 was treated with 0.5 µM of dTAG7 or dTAG13, and samples were harvested at several time points. The glycosylated form (upper band) appeared to be degraded faster, within five hours, than the unglycosylated form.
(C)dTAG-HA SLC2A3 was stably expressed in HAP1, LS180 and HCT15 cells. Following 72 hours of treatment with 0.5 µM dTAG13 SLC2A3 was completely degraded.
(D)Chemical "rescue" of dTAG-driven degradation of SLCs. HAP1 cell lines expressing dTAG-HA SLC1A5 or dTAG-HA SLC38A9 were treated with 0.5 µM dTAG7 for 12 or 18 hours, respectively. These cells were also treated with chloroquine (CQ, 50µM), bortezomib (bort., 1µM), MG-132 (MG, 1µM), MLN4924 (MLN, 1µM), pomalidomide (poma., 10µM), or bafilomycin A1 (bafi., 2.5µM). SLC degradation was rescued by inhibiting CRL activity or the proteasome, but not by inhibiting the lysosome machinery.

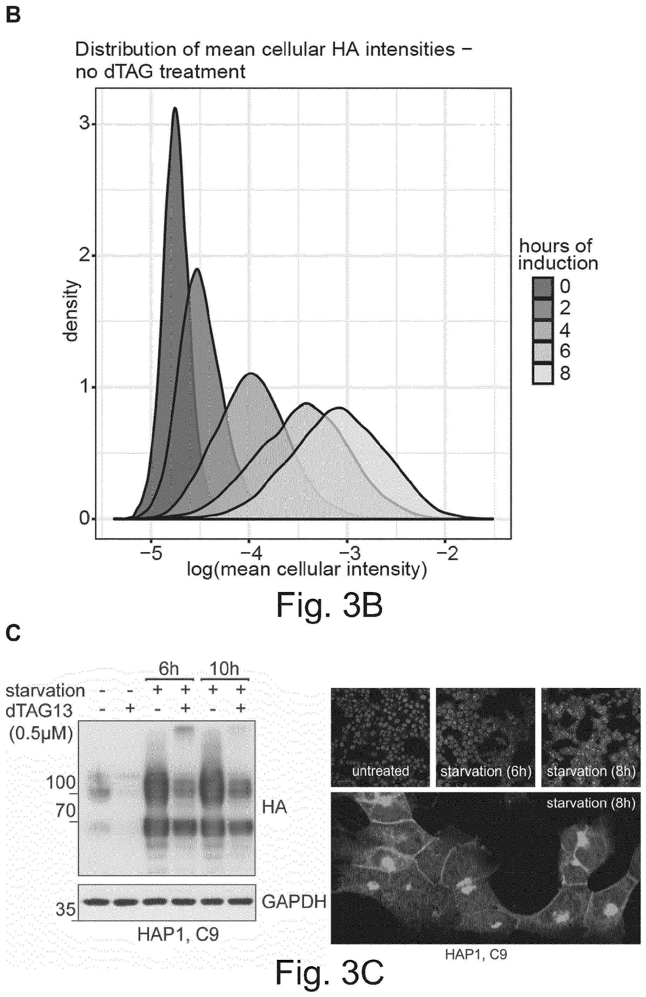

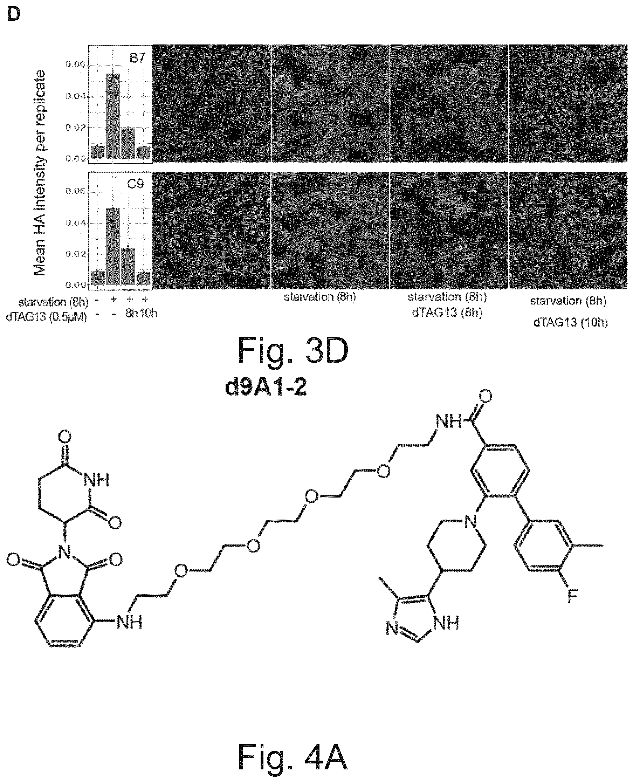

### Figure 3: dTAG knock-in are equally amenable to targeted degradation

(A) dTAG knock-in: SLC38A2 was tagged at N-terminus with dTAG-HA. Blasticidn was used as the selection marker. Single cell clones were derived and characterized as previously described. Two representative clones, B7 and C9 are presented here.
(B) The expression of dTAG-HA SLC38A2 was induced by replacing the normal culture media with media deprived of amino acids and FBS. Expression of the endogenous SLC is induced rapidly and was monitored by immunofluorescence imaging and quantified by automated image analysis. Representative images are shown.
(C)The effect of amino acid starvation on dTAG-HA SLC38A2 expression in HAP1 cells. Cells were treated with 0.5 µM dTAG13 and/or media lacking amino acids/FBS for either 6 or 10 hours, after which expression and degradation were monitored via western blot. Near complete degradation can be achieved by cotreatment.
(D)Immunofluorescence imaging of two different HAP1 clones expressing dTAG-HA SLC38A2. Cells were incubated in media lacking amino acids/FBS for 8 hours and treated with 0.5 µM dTAG13 for either 8 or 10 hours. Expression of the endogenous SLC is induced rapidly and was monitored by immunofluorescence and quantified by automated image analysis. Representative images are presented. The two-hour pre-treatment with dTAG13 is sufficient to completely abrogate any SLC expression.

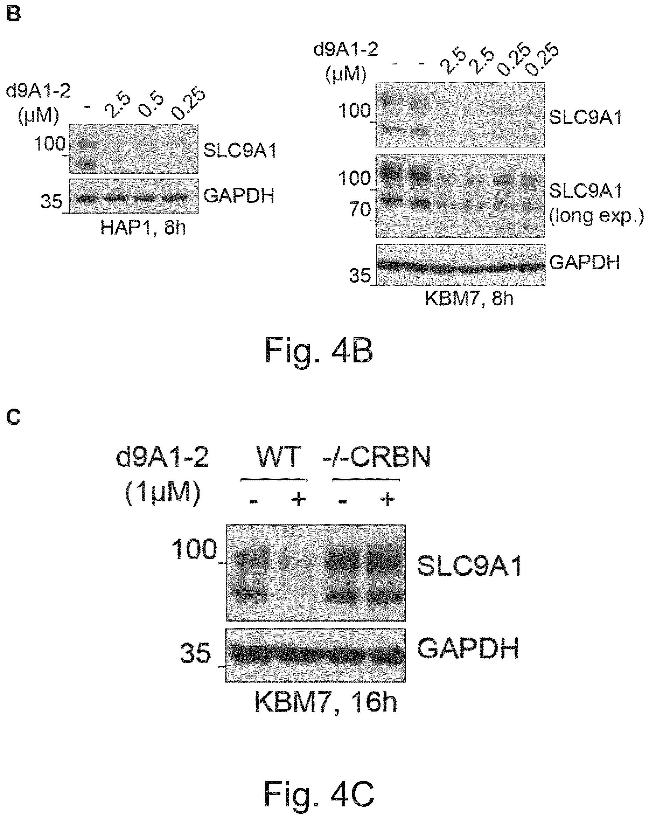

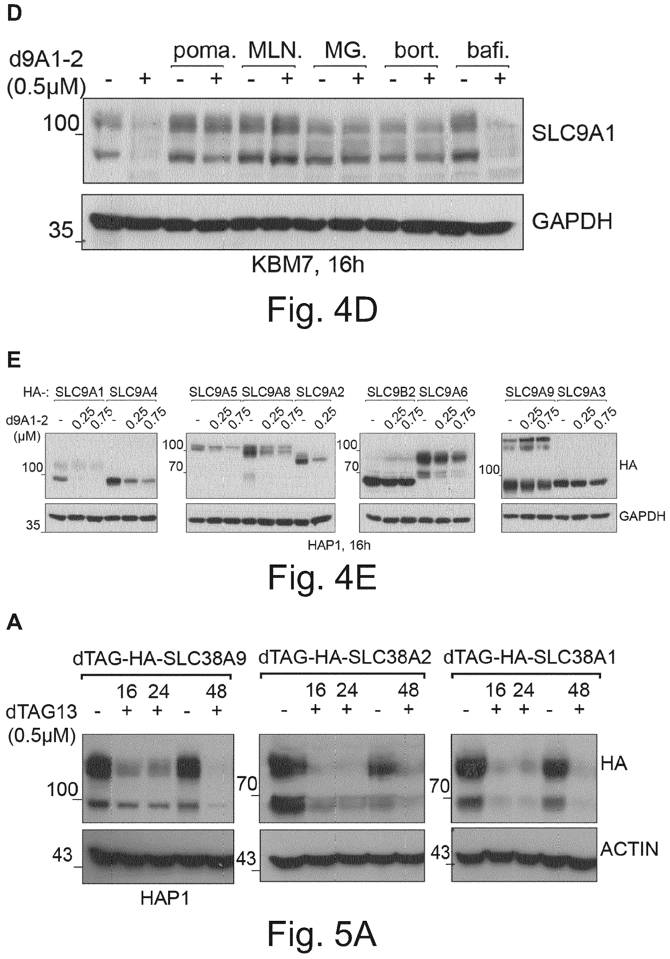

### Figure 4: New SLC9A degrader series

(A) The chemical structure of the SLC degrader "d9A1-2"
(B) HAP1 and KBM7 cells were treated with different concentrations of d9A1-2. Within 8 hours degradation of SLC9A1 was observed in both cell lines.
(C)Both WT and CRBN knockout KBM7 cell lines were treated with 1µM d9A1-2 for 16 hours. SLC9A1 degradation is observed in WT but not CRBN knockout.
(D) Chemical "rescue" of d9A1-2 driven degradation. WT KBM7 cell lines were treated with 0.5 µM d9A1-2 for 16 hours. These cells were also treated with bortezomib (bort. 0.25 µM), MG-132 (MG, 1 µM), MLN4924 (MLN,1 µM), pomalidomide (poma., 1 µM), or bafilomycin A1 (bafi., 10 µM). All molecules, apart from bafilomycin, could rescue SLC9A1 from degradation.
(E) Selectivity of d9A1-2 was tested across HAP1 cell lines expressing Strep/HASLC9A1, 9A4, 9A5, 9A8, 9A2, 9B2, 9A6, 9A9, and 9A3. Cells were treated with varying concentrations of d9A1-2 for 16 hours, after which degradation of the exogenous SLCs was monitored.

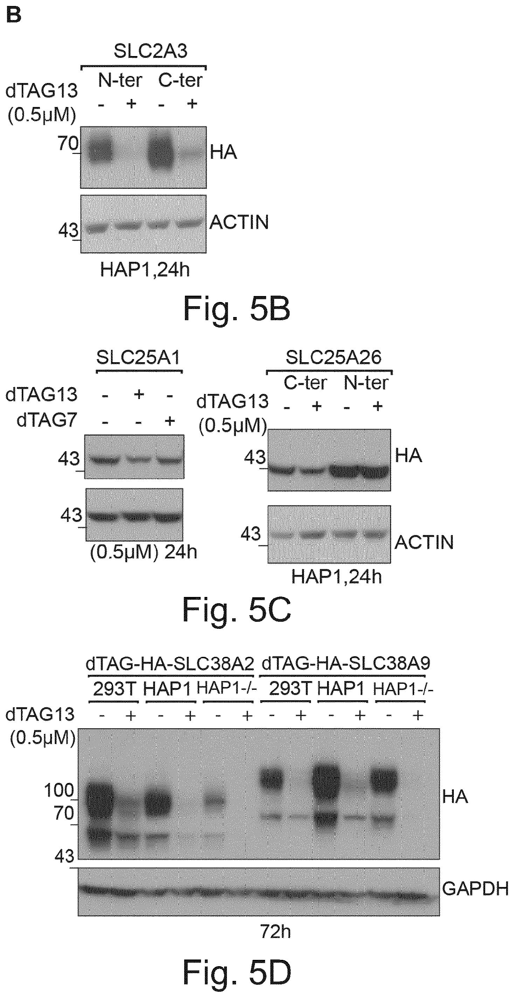

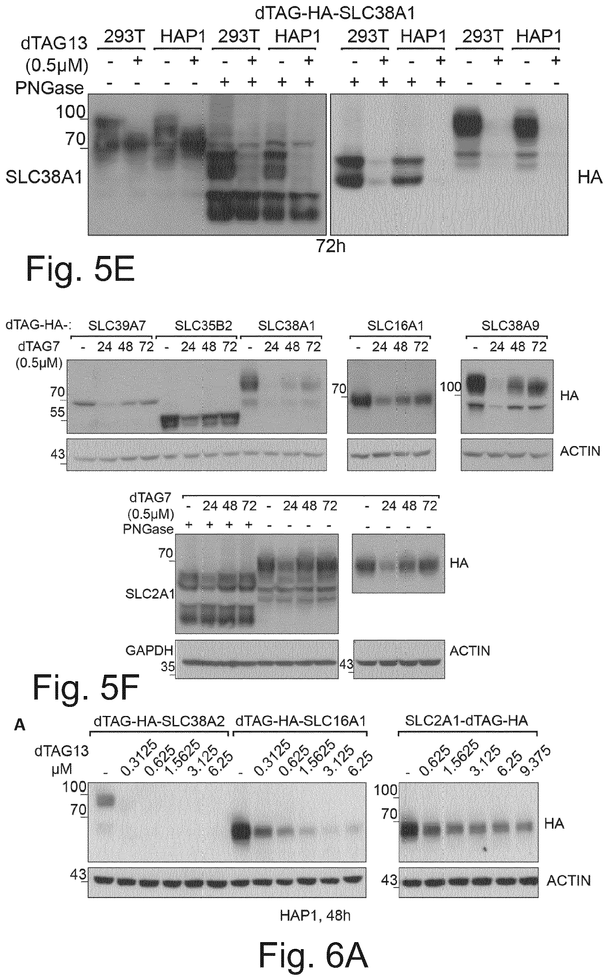

### Figure 5: Degradation of tagged SLCs

(A) HAP1 cells expressing dTAG-HA SLC38A9, 38A2, or 38A1 were treated with 0.5 µM dTAG13 for 16, 24 or 48 hours. Near-complete degradation of the target SLC was observed and maintained.
(B) SLC2A3 was tagged on the N- or C-terminus with dTAG-HA. The two HAP1 cell lines were treated for 24 hours with dTAG13 and SLC2A3 was degraded in both.
(C) HAP1 cell lines expressing dTAG-HA SLC25A1 and 25A26 did not demonstrate significant degradation after 24 hours of treatment with 0.5 µM dTAG.
(D) Expression of dTAG-HA SLC38A2 or 38A9 in either 293T or HAP1 WT or a HAP1 clone in which the respective SLC was genetically ablated. The variance in expression levels indicated that degradation is achieved irrespective of the initial stable expression level, nor dependent on the presence of the endogenous SLC.
(E) dTAG-HA SLC38A1, expressed in HAP1 or 293T, was degraded by treatment with dTAG13. Indicated protein extracts were de-glycosylated by treatment with PNGase. Western blotting with antibodies against the HA-tag and the total protein confirmed that the tagged protein as a whole is completely degraded.
(F) HAP1 cells expressing dTAG-HA SLC39A7, 35B2, 16A1, 38A9 or 2A1 were treated with 0.5 µM of dTAG7 for 24, 48 or 72 hours. dTAG7 leads to reversible degradation of the target SLC, allowing accurate temporal control over protein levels. Western blotting with antibodies against the HA-tag and the total protein mirrored the observed pattern.

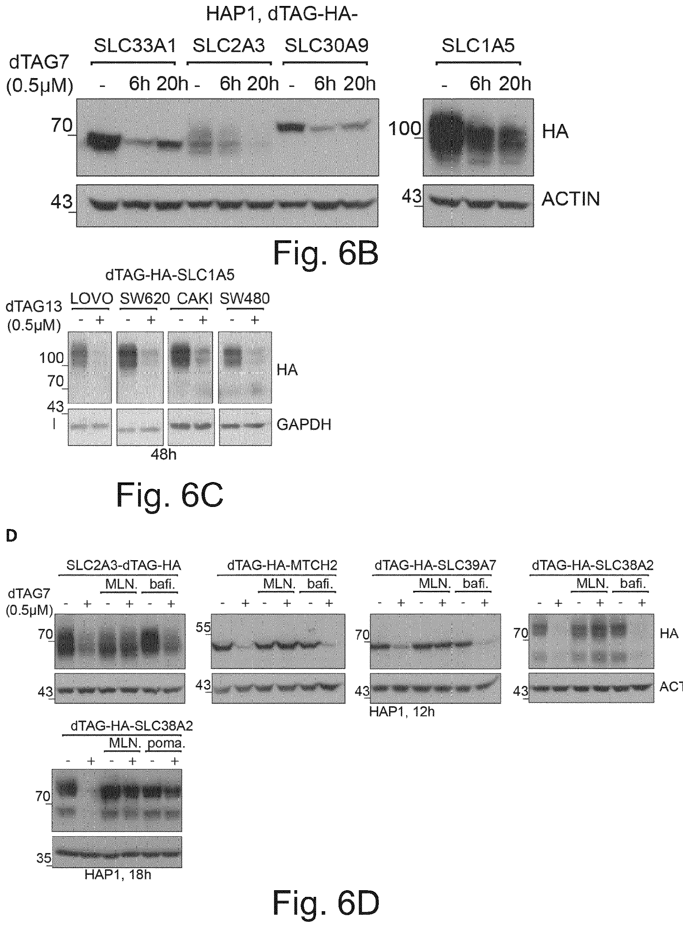

### Figure 6: Degradation of tagged SLCs

(A) A range of the degrader molecule dTAG13 concentrations was tested in HAP1 cell lines expressing dTAG-HA SLC38A2, 16A1 and 2A1. Cells were treated for 48 hours. The concentration required to achieve complete or near to complete degradation depends on the SLC.
(B) A time course of dTAG driven SLC degradation demonstrates initiation of degradation within 6 hours. HAP1 cell lines expressing dTAG-HA SLC33A1, 2A3, 30A9 or 1A5 were treated with 0.5 µM of dTAG7.
(C) dTAG-HA SLC1A5 was expressed in LOVO, SW620, CAKI and SW480 cell lines. Cells were treated with 0.5 µM dTAG13 and degradation was observed after 48 hours.
(D) Chemical "rescue" of dTAG driven degradation. HAP1 cell lines expressing dTAGHA SLC2A3, 39A7, 38A2, and MTCH2 were treated with 0.5 µM dTAG7 for 12 hours. A cell line expressing dTAG-HA SLC38A2 was treated with 0.5 µM dTAG7 for 18 hours. These cells were treated with MLN4924 (MLN, 1µM), pomalidomide (poma., 10µM), or bafilomycin A1 (bafi., 2.5µM) to monitor the inhibition of degradation.

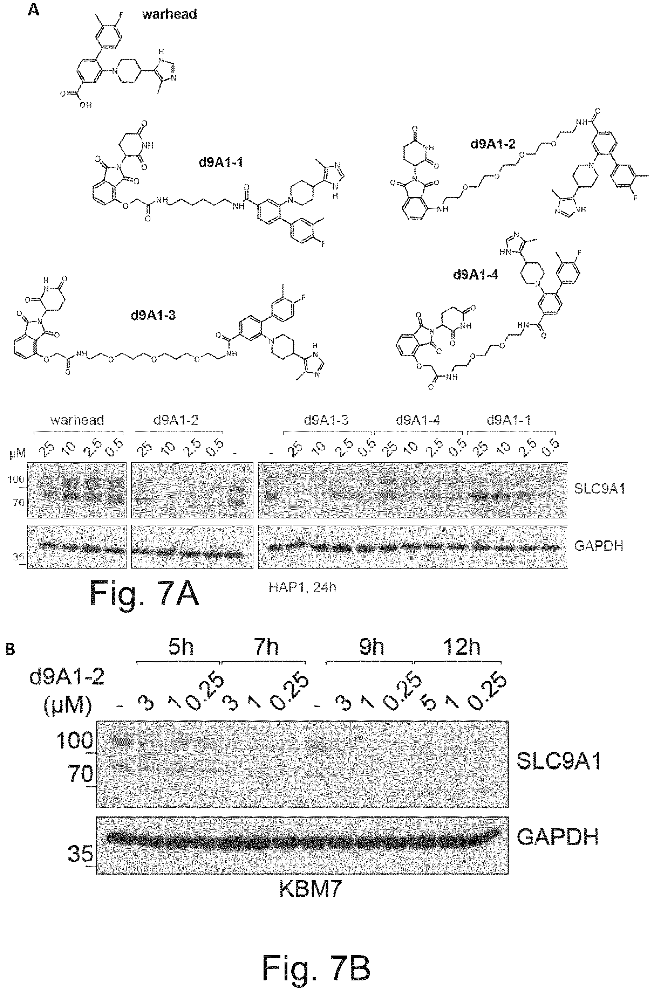

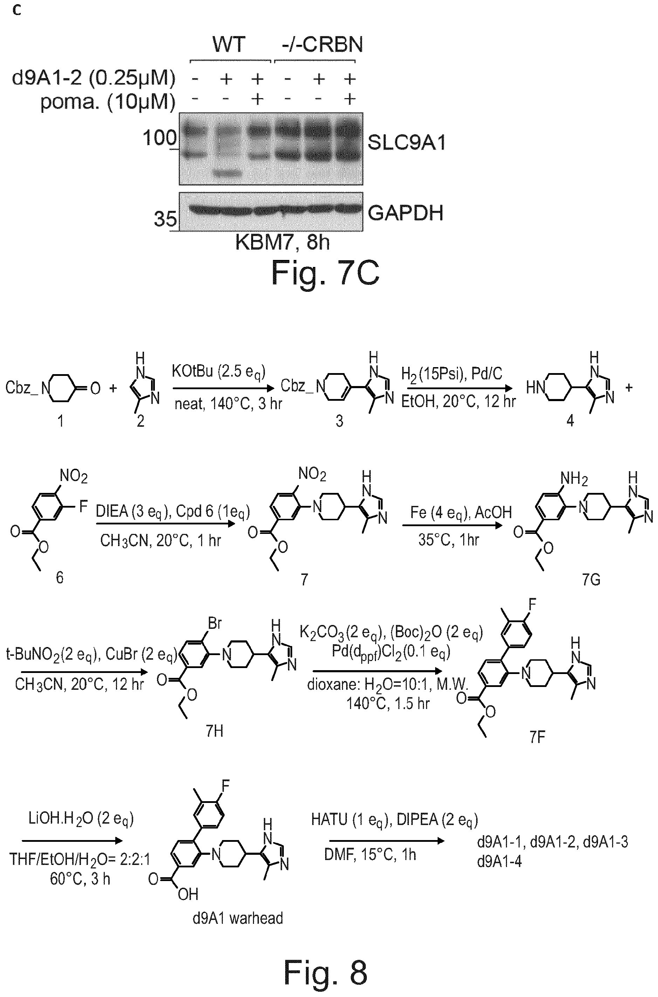

### Figure 7: Chemical structures

(A) Chemical structures of warhead and derived compounds of the d9A1 series.
(B) HAP1 cells were treated with a dose curve of the warhead, d9A1-1, d9A1-2, d9A1-3, d9A1-4, for 24 hours. SLC9A1 degradation was most effective with d9A1-2 but could also be achieved with d9A1-3.
(C) KBM7 cells were treated with varying concentrations of d9A1-2. Reduction of SLC9A1 expression was observed at 5, 7, 9 or 12 hours.

### Figure 8: Workflow for synthesis

Workflow for the synthesis of PROTAC d9A1 warhead and ensuing degraders, including the HNMR data.

### Figure 9: Viability assay

Relative viability of Peripheral blood mononuclear cells (PBMCs) after treatment with the d9A1-2 molecule PBMCs were isolated from healthy donors and patients with acute myeloid leukemia, multiple myeloma, B cell lymphoma and Hodgkin's lymphoma, chronic lymphocytic leukeumia and treated with a dose curve of d9A1-2. Viability was assayed using the CellTiter-Glo assay, 72 hours post treatment.

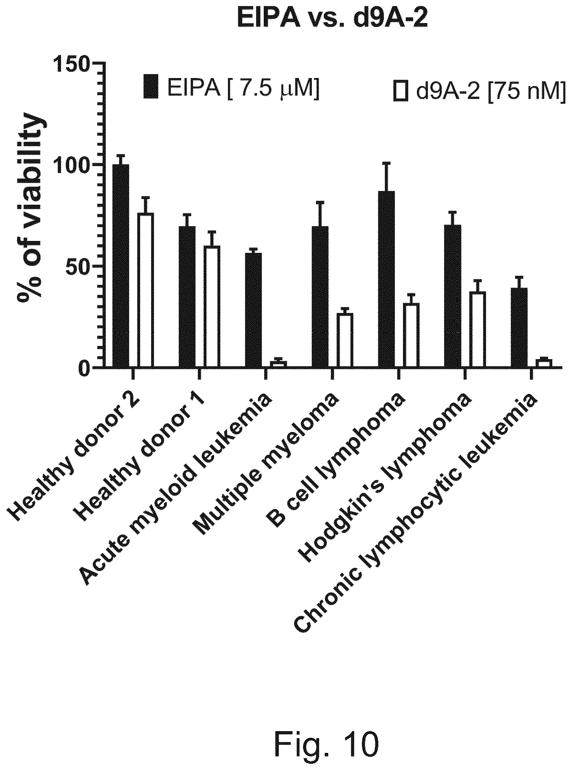

**Figure 10****: Viability assay** Relative viability of Peripheral blood mononuclear cells (PBMCs) after single-dose treatment with the d9A1-2 molecule or ethyl-isopropyl amiloride (EIPA). PBMCs were isolated from healthy donors and patients with acute myeloid leukemia, multiple myeloma, B cell lymphoma and Hodgkin's lymphoma, chronic lymphocytic leukeumia. Viability was assayed using the CellTiter-Glo assay, 72 hours post treatment.

### EXAMPLES

### EXAMPLE 1

It was assessed whether SLCs, despite their complex membrane-binding topology and heterogenous subcellular localization, were amenable to chemical degradation. First SLC-dTAG fusion proteins were ectopically expressed. Then SLC proteins expressed from their natural chromosomal locus, and therefore exposed to more physiological regulation, were tested whether they could be made degradable through dTAG knock-in. Finally, the development of a new chemical entity able to degrade an untagged endogenous SLC protein was assessed elucidating SLC functions.

### Materials and Methods

### Synthesis of d9A1-1 - d9A1-4

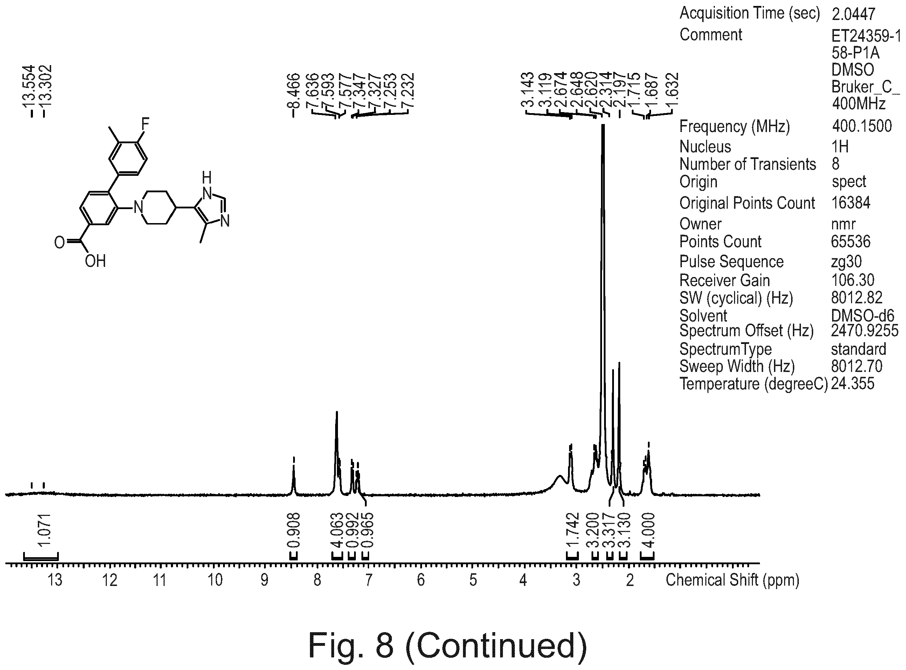

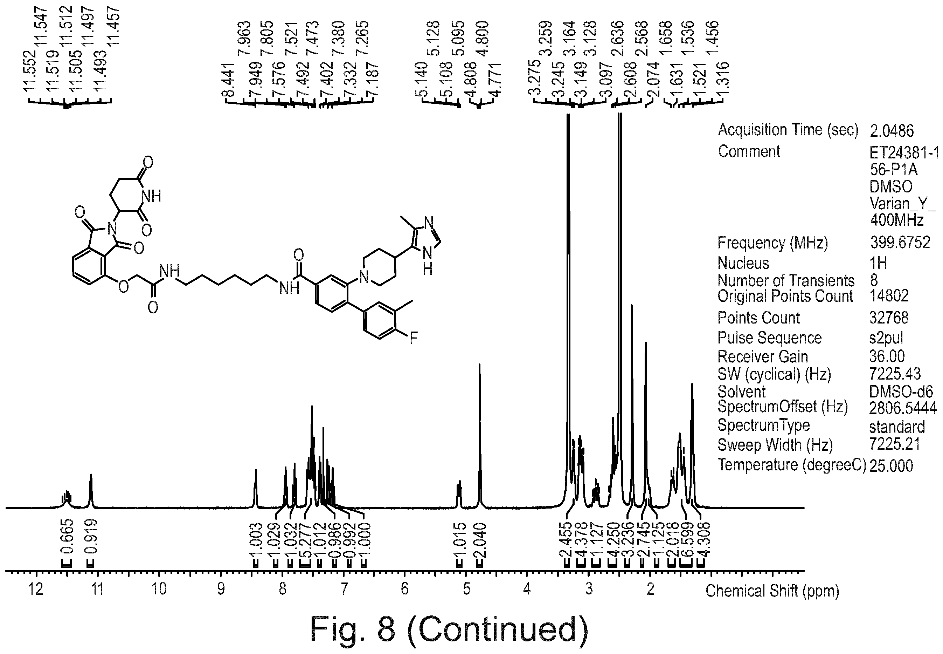

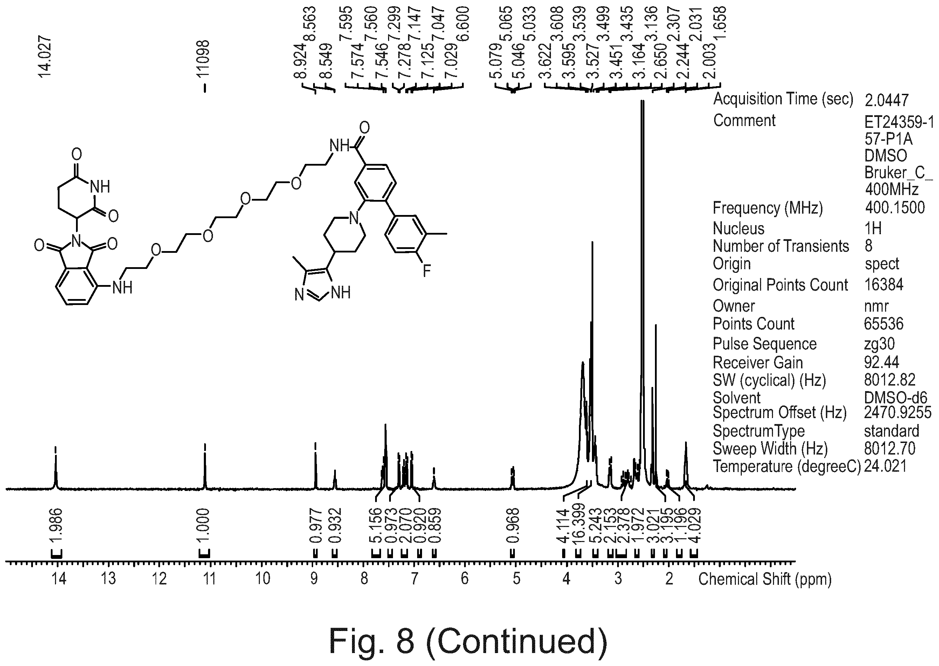

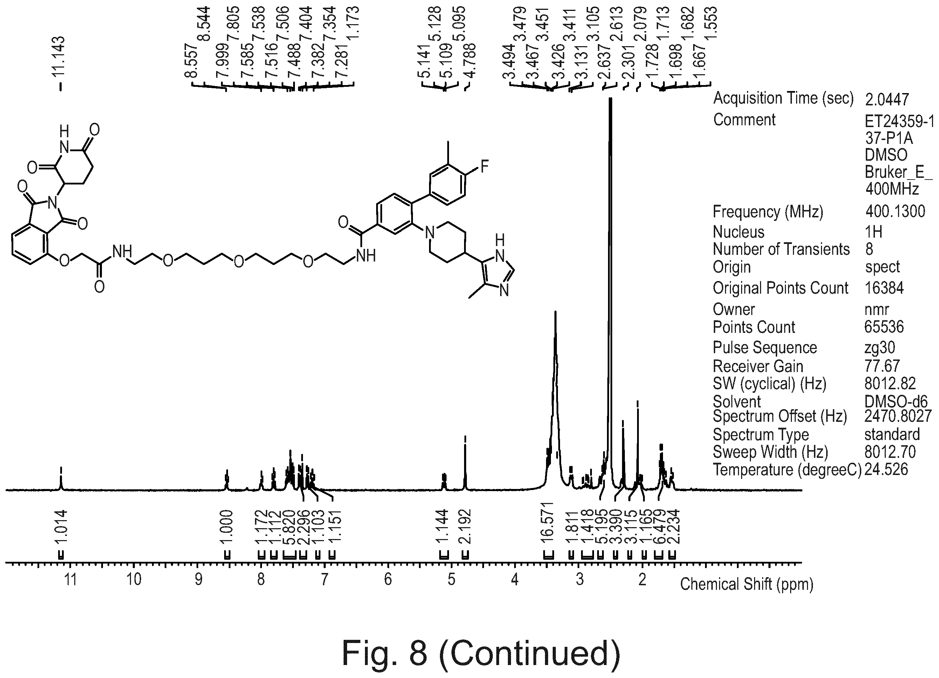

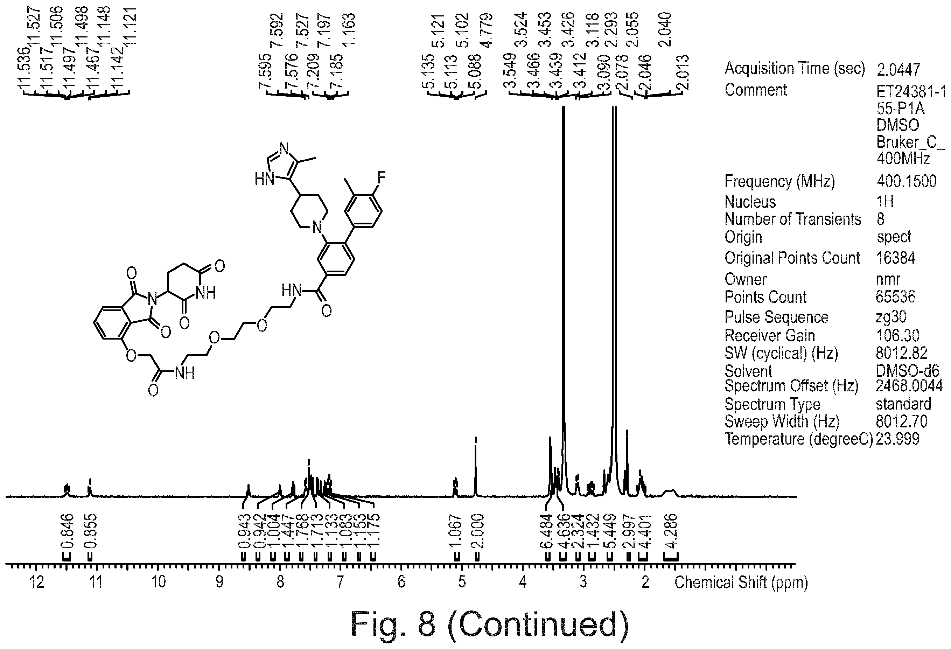

Intermediate 3 was synthesised by the reaction of 1-benzyloxycarbonyl-4-piperidone with 4-methyl-1H-imidazole using 2.5 eq KOtBu as base at 140°C in 4.3% yield (Figure 8). Intermediate 3 was subjected to hydrogenation in EtOH with Pd/C at 15 psi for 12 h, thereby both reducing the tetrahydropyridine ring and deprotecting the CBz group to piperidine 4 in quantitative yield (Figure 8). Compound 4 easily underwent nucleophilic aromatic substitution with ethyl 3-fluoro-4-nitrobenzoate in MeCN at 20°C using iPr₂NEt as base (Figure 8). The obtained nitroarene 7 was reduced to the aromatic amine 7G with iron and acetic acid in 63% yield (Figure 8). This amine was diazotized with 2 eq t-BuONO in MeCN and the diazo intermediate was reacted with CuBr₂ at 20°C for 12 h to obtain 28% of bromoarene 7H (Figure 8). Bromide 7H underwent Suzuki coupling with 4-fluoro-3-methylphenylboronic acid using 10 mol% Pd(dppf)Cl₂ as the catalyst, 2 eq Boc₂O for the *in situ* protection of the substrate, and 2 eq (Figure 8). K₂CO₃ as the base in dioxane with 10% H₂O under MW irradiation at 140°C for 1.5 h (Figure 8). The product of the Suzuki coupling, intermediate 7F, was obtained in 37% yield, the Boc group having undergone deprotection *in situ* (Figure 8). It was then hydrolysed with LiOH in THF / EtOH / H₂O to the carboxylic acid, d9A1 warhead. The compounds d9A1-1 to d9A1-4 were synthesized from the d9A1 warhead and the aminoalkyl substituted Cereblon ligands (synthesized as described in Zhang et al., Eur J Med Chem, 151, 304-314 (2018)) by amide couplings using HATU and iPr₂NEt in DMF (Figure 8).

### Detailed methods of synthesis

| Compound Code | Structure |
|---|---|
| CP-19076070-1 (d9A1 warhead with carboxylic acid attachment point) | 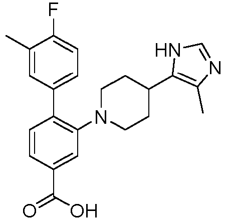 |
| CP-19076070-2 (d9A1 warhead without attachment point) | 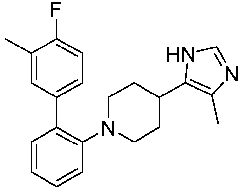 |
| CP-19076070-3 (d9A1 warhead with amine attachment point) | 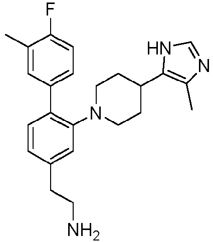 |
| CP-19076070-4 (d9A1 warhead with alkyne attachment point) | 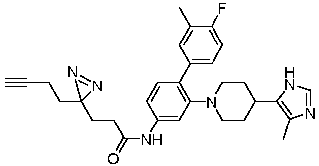 |
| CP-19076070-5 (d9A1-2) | 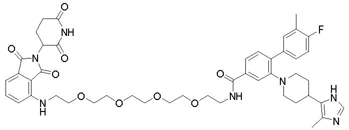 |

### Synthesis of CP-19076070-1

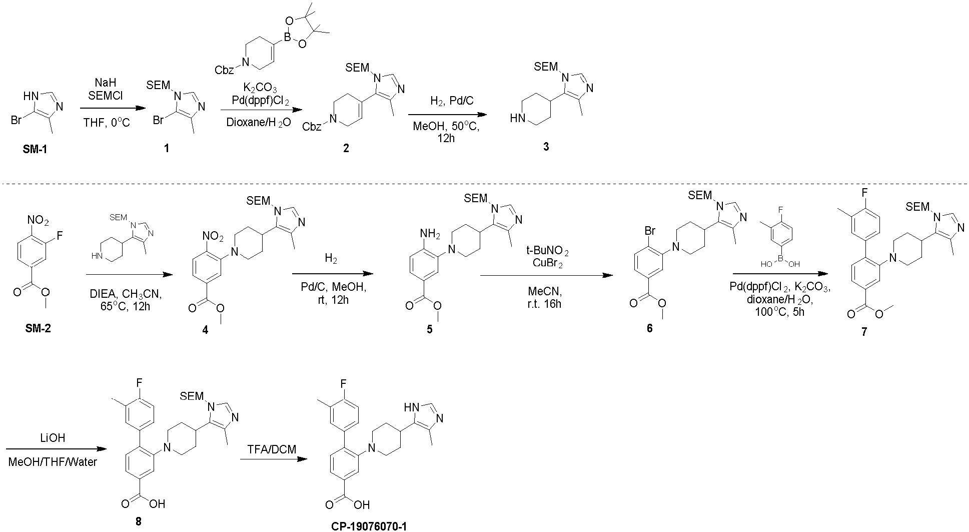

### Step A: Synthesis of 5-bromo-4-methyl-1-((2-(trimethylsilyl)ethoxy)methyl)-1H-imidazole

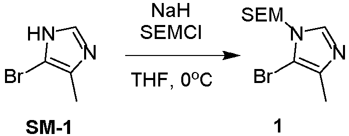

To a solution of 5-bromo-4-methyl-1H-imidazole (5 g, 24.0 mmol) in tetrahydrofuran (50 mL) was added NaH (1.25 g, 31.2 mmol, 60% in oil) under cooling with an ice-bath. The reaction mixture was stirred in the ice-bath for 1 h. Then (2-(trimethylsilyl)ethoxy)methyl-chloride (5.2 g, 31.2 mmol) was added dropwise to the solution and the reaction mixture was stirred at room temperature for 2 h. LCMS monitored no starting material. The mixture was quenched with saturated NH₄Cl solution and the mixture was extracted with ethyl acetate (100 mL × 3). The combined organic layers were concentrated. The residue was purified by flash chromatography (petroleum ether/ethyl acetate=5:1) to give the product 5-bromo-4-methyl-1-((2-(trimethylsilyl)ethoxy)methyl)-1H-imidazole (6.7 g, 93% yield) as a brown oil.

LC purity: 97.00 % (214 nm); Mass: 339.0 [M+H]⁺ at 1.76 min.

### Step B: Synthesis of benzyl 4-(4-methyl-1-((2-(trimethylsilyl)ethoxy)methyl)-1H-imidazol-5-yl)-3,6-dihydropyridine-1(2H)-carboxylate

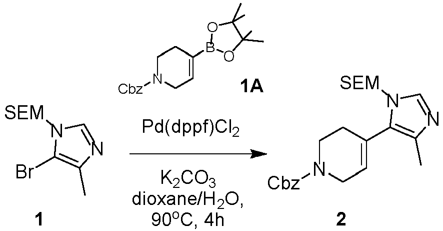

To a solution of 5-bromo-4-methyl-1-((2-(trimethylsilyl)ethoxy)methyl)-1H-imidazole (5.3 g, 15.9 mmol) in dioxane (50 mL) and water (10 mL) was added benzyl 4-(4,4,5,5-tetramethyl-1,3,2-dioxaborolan-2-yl)-3,6-dihydropyridine-1(2H)-carboxylate (6 g, 17.5 mmol), K₂CO₃ (4.3 g, 31.4 mmol) and Pd(dppf)Cl₂ (1.2 g, 1.6 mmol). The reaction mixture was stirred at 90°C under N₂ for 4 h. LCMS monitored no starting material. The mixture was diluted with water and the aqueous layer was extracted with ethyl acetate (120 mL × 3). The combined organic layers were concentrated. The residue was purified by flash chromatography (petroleum ether/ethyl acetate=1:2) to give the product benzyl 4-(4-methyl-1-((2-(trimethylsilyl)ethoxy)methyl)-1H-imidazol-5-yl)-3,6-dihydropyridine-1(2H)-carboxylate (4.5 g, 57.8% yield) as a brown oil.

LC purity: 92.88 % (214 nm); Mass: 428.1 [M+H]⁺ at 1.57 min.

### Step C: Synthesis of 4-(4-methyl-1-((2-(trimethylsilyl)ethoxy)methyl)-1H-imidazol-5-yl)piperidine

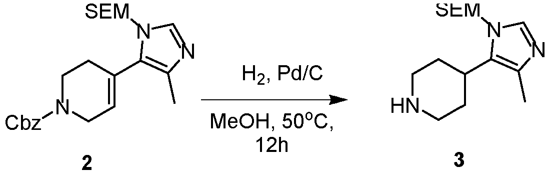

To a solution of benzyl 4-(4-methyl-1-((2-(trimethylsilyl)ethoxy)methyl)-1H-imidazol-5-yl)-3,6-dihydropyridine-1(2H)-carboxylate (3.8 g, 8.9 mmol) in MeOH (300 mL) was added Pd/C (1 g). The reaction mixture was stirred at 60°C overnight under H₂. LCMS showed no remaining starting material. The reaction mixture was filtered and the mother liquid was concentrated in vacuo to give 4-(4-methyl-1-((2-(trimethylsilyl)ethoxy)methyl)-1H-imidazol-5-yl)piperidine (1.09 g, 22.2% yield) as a yellow solid.

LC purity: 100 % (214 nm); Mass: 296.1 [M+H]⁺ at 1.23 min.

¹H NMR (500 MHz, DMSO-*d*₆) δ 7.58 (s, 1H), 5.25 (s, 2H), 3.48 (t, *J* = 8.0 Hz, 2H), 3.02 (s, 2H), 2.18 (s, 3H), 1.62 (dt, *J* = 54.0, 12.0 Hz, 4H), 0.86 (t, *J* = 8.0 Hz, 2H), 0.00 (s, 9H).

### Step D: Synthesis of methyl 3-(4-(4-methyl-1-((2-(trimethylsilyl)ethoxy)methyl)-1H-imidazol-5-yl)piperidin-1-yl)-4-nitrobenzoate

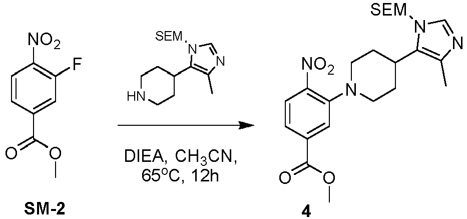

To a solution of methyl 3-fluoro-4-nitrobenzoate (1.5 g, 7.5 mmol) in acetonitrile (20 mL) was added 4-(4-methyl-1-((2-(trimethylsilyl)ethoxy)methyl)-1H-imidazol-5-yl)piperidine (1.8 g, 6.3 mmol) and diisopropyl ethyl amine (2.4 g, 18.8 mmol) at room temperature The reaction was stirred at 65°C for 12 hr. The reaction mixture was diluted with ethyl acetate (100 mL), washed with brine (100 mL), dried over Na₂SO₄, filtered and concentrated in vacuo. The residue was purified by column chromatography on silica gel (0-100% acetone in petroleum ether, v/v) to give methyl 3-(4-(4-methyl-1-((2-(trimethylsilyl)ethoxy)methyl)-1H-imidazol-5-yl)piperidin-1-yl)-4-nitrobenzoate. (1.5 g, 43% yield) as a yellow oil.

LC purity: 100% (214 nm); Mass: 475.1 [M+H]⁺ at 1.58 min.

### Step E: Synthesis of methyl 4-amino-3-(4-(4-methyl-1-((2-(trimethylsilyl)ethoxy)methyl)-1H-imidazol-5-yl)piperidin-1-yl)benzoate

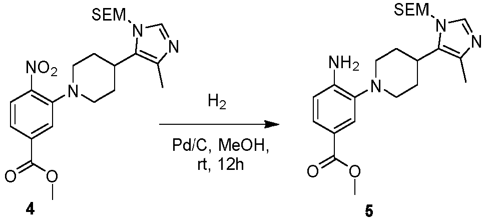

To a solution of methyl 3-(4-(4-methyl-1-((2-(trimethylsilyl)ethoxy)methyl)-1H-imidazol-5-yl)piperidin-1-yl)-4-nitrobenzoate (1.5 g, 3.2 mmol) in MeOH (20 mL) was added 10% Pd/C (300 mg) at room temperature The reaction was stirred at room temperature under H₂ for 12 hr. The reaction mixture was filtered and concentrated in *vacuo.* The residue was purified by column chromatography on silica gel (0-100% acetone in petroleum ether, v/v) to give methyl 4-amino-3-(4-(4-methyl-1-((2-(trimethylsilyl)ethoxy)methyl)-1H-imidazol-5-yl)piperidin-1-yl)benzoate (1.23 g, 87% yield) as a yellow oil.

LC purity: 100% (214 nm); Mass: 445.1 [M+H]⁺ at 1.53 min.

### Step F: Synthesis of methyl 4-bromo-3-(4-(4-methyl-1-((2-(trimethylsilyl)ethoxy)methyl)-1H-imidazol-5-yl)piperidin-1-yl)benzoate

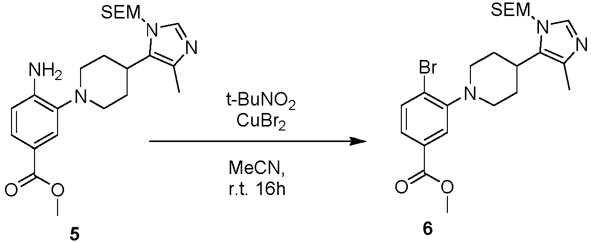

To a solution of methyl 4-amino-3-(4-(4-methyl-1-((2-(trimethylsilyl)ethoxy)methyl)-1H-imidazol-5-yl)piperidin-1-yl)benzoate (1.23 g, 2.77 mmol) in acetonitrile (20 mL) was added t-BuNO₂ (0.57 g, 5.53 mmol), CuBr₂ (0.62 g, 2.77 mmol) at 0 °C and the reaction was stirred at room temperature for 16 hr. The reaction mixture was diluted with ethyl acetate (100 mL), washed with brine (100 mL), dried over Na₂SO₄, filtered and concentrated in vacuo. The residue was purified by column chromatography on silica gel (0-10% MeOH in ethyl acetate, v/v) to give methyl 4-bromo-3-(4-(4-methyl-1-((2-(trimethylsilyl)ethoxy)methyl)-1H-imidazol-5-yl)piperidin-1-yl)benzoate (750 mg, 53% yield) as a colorless oil.

LC purity: 74.29% (214 nm); Mass: 508.2 [M+H]⁺ at 1.23 min.

### Step G: Synthesis of methyl 4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-carboxylate

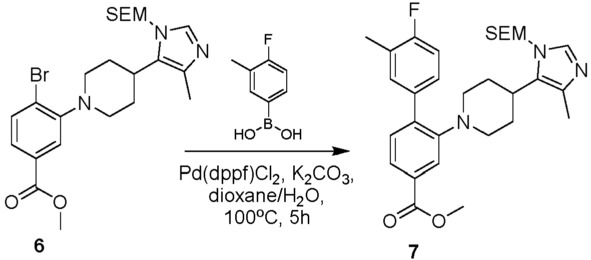

To a solution of methyl 4-bromo-3-(4-(4-methyl-1-((2-(trimethylsilyl)ethoxy)methyl)-1H-imidazol-5-yl)piperidin-1-yl)benzoate (750 mg, 1.47 mmol) in dioxane (5 mL) and water (1 mL) was added K₂CO₃ (610 mg, 4.41 mmol) and Pd(dppf)Cl₂ (108 mg, 0.147 mmol) at room temperature The reaction was stirred at 100°C for 5 hr. The mixture was purified by preparative HPLC to give methyl 4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-carboxylate (370 mg, 46% yield) as a brown oil.

LC purity: 100% (214 nm); Mass: 538.3 [M+H]⁺ at 1.37 min.

### Step H: Synthesis of 4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-carboxylic acid

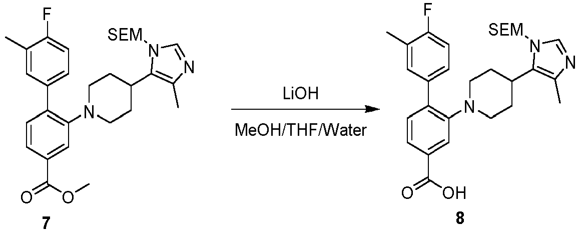

To a solution of methyl 4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-carboxylate (470 mg, 0.87 mmol) in MeOH/tetrahydrofuran/Water (21 mL) was added LiOH (74 mg, 1.75 mmol). The reaction mixture was stirred at room temperature for 2 h. LCMS showed no remaining starting material. The mixture was diluted with water and concentrated to remove the solvent, the aqueous layer was adjusted pH to 4~5 with 1N HCl solution in water. The mixture was extracted with ethyl acetate (40 mL × 3). The combined organic layers were concentrated in vacuo to give 4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-carboxylic acid (400 mg, 87.4% yield) as a brown solid.

LC purity: 100 % (214 nm); Mass: 524.1 [M+H]⁺ at 1.63 min.

### Step I: Synthesis of 4'-fluoro-3'-methyl-2-(4-(4-methyl-1H-imidazol-5-yl)piperidin-1-yl)-[1,1'-biphenyl]-4-carboxylic acid

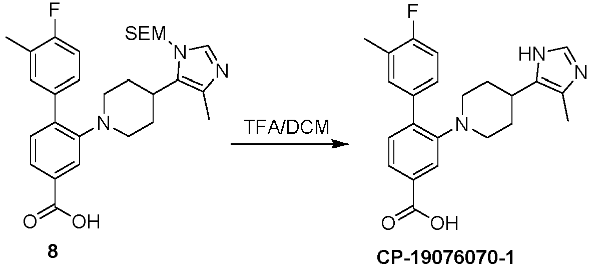

To a solution of 4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-carboxylic acid (400 mg, 0.76 mmol) in CH₂Cl₂ (9 mL) was added trifluoro acetic acid (872 mg, 7.6 mmol) while cooling with an ice-bath. The reaction was stirred at room temperature for 2h. LCMS showed no remaining starting material. The mixture was purified by preparative HPLC to give the product (200 mg, 66.5% yield, trifluoro acetic acid salt) as a white solid.

LC purity: 100 % (214 nm); Mass: 394.1 [M+H]⁺ at 1.46 min.

1H NMR (400 MHz, DMSO-*d*₆) δ 8.16 (s, 1H), 7.66 - 7.56 (m, 4H), 7.45 (s, 1H), 7.32 (d, *J* = 8.0 Hz, 1H), 7.21 (t, *J =* 9.2 Hz, 1H), 3.10 (d, *J =* 12.0 Hz, 2H), 2.62 (t, *J =* 12.0 Hz, 2H), 2.30 (s, 3H), 2.09 (s, 3H), 1.65 (dd, *J =* 24.0, 12.0 Hz, 2H), 1.55 (d, *J =* 10.4 Hz, 2H).

### Synthesis of CP-19076070-2

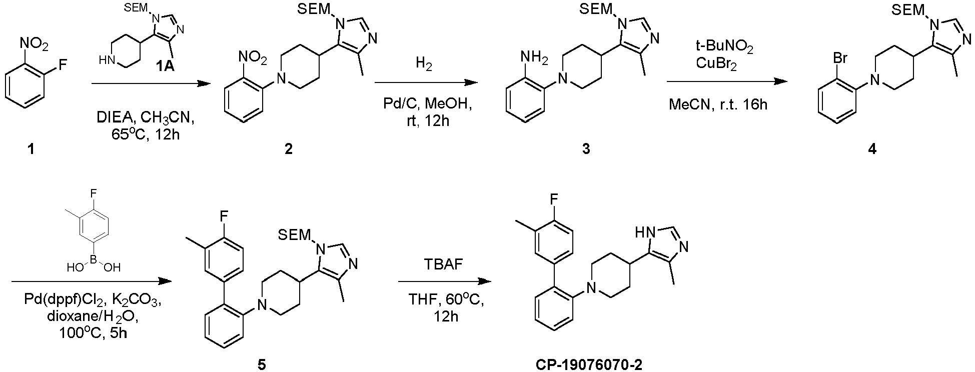

### Step A: Synthesis of 4-(4-methyl-1-((2-(trimethylsilyl)ethoxy)methyl)-1H-imidazol-5-yl)-1-(2-nitrophenyl)piperidine

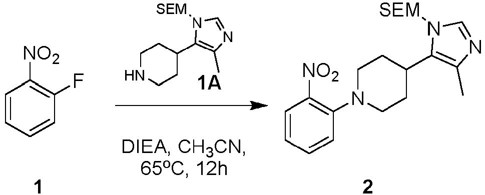

To a mixture of 1-fluoro-2-nitrobenzene (0.6 g, 4.25 mmol) and 4-(4-methyl-1-((2-(trimethylsilyl)ethoxy)methyl)-1H-imidazol-5-yl)piperidine (1.01 g, 3.40 mmol) in CH₃CN (15 mL) was added diisopropyl ethyl amine (1.1 g, 8.5 mmol). The mixture was stirred at 60°C for 12h. The mixture was concentrated in vacuo. The residue was purified by silica gel chromatography on silica (petroleum ether/ethyl acetate = 1:1, v/v) to afford 4-(4-methyl-1-((2-(trimethylsilyl)ethoxy)methyl)-1H-imidazol-5-yl)-1-(2-nitrophenyl)piperidine (1.3 g, 91.3%) as a yellow oil.

LC-Mass Method: Mobile Phase: A:Water (0.01% trifluoro acetic acid) B: acetonitrile (0.01% trifluoro acetic acid) Gradient: 5% B for 0.2 min, increase to 95% B within 1.3 min, 95% B for 1.7 min, back within 0.01 min. Flow Rate: 1.8 ml/min; Column: Xbridge C18, 3.5µm,4.6*50mm ; Column Temperature: 50°C. LC purity: 100% (254 nm), Mass: 417.1 [M+H]⁺ at 1.59 min.

### Step B: Synthesis of 2-(4-(4-methyl-1-((2-(trimethylsilyl)ethoxy)methyl)-1H-imidazol-5-yl)piperidin-1-yl)aniline

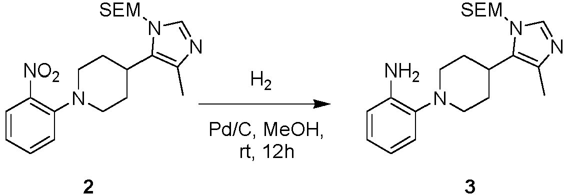

To a solution of 4-(4-methyl-1-((2-(trimethylsilyl)ethoxy)methyl)-1H-imidazol-5-yl)-1-(2-nitrophenyl)piperidine (1.3 g, 3.12 mmol) in MeOH (20 mL) was added Pd/C (0.5 g). The mixture was stirred at 20~25°C for 12h under H₂ (15 psi). The mixture was filtered and the mother liquid was concentrated in vacuo to give desired product 2-(4-(4-methyl-1-((2-(trimethylsilyl)ethoxy)methyl)-1H-imidazol-5-yl)piperidin-1-yl)aniline (1.1 g, 85.4% yield) as a yellow oil.

LC-Mass Method: Mobile Phase: A: water (0.01% trifluoro acetic acid) B: acetonitrile (0.01% trifluoro acetic acid) Gradient: 5% B for 0.2 min, increase to 95% B within 1.3 min, 95% B for 1.5 min, back within 0.01 min. Flow Rate: 2 ml/min; Column: Sunfire, 50*4.6mm, 3.5µm; Column Temperature: 50°C. LC purity: 94% (254 nm), Mass: 387.1 [M+1]⁺ at 1.505 min.

### Step C: Synthesis of 1-(2-bromophenyl)-4-(4-methyl-1-((2-(trimethylsilyl)ethoxy)methyl)-1H-imidazol-5-yl)piperidine

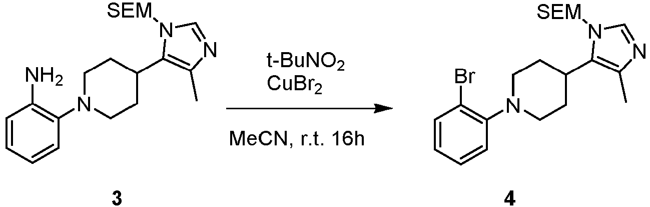

To a solution of 2-(4-(4-methyl-1-((2-(trimethylsilyl)ethoxy)methyl)-1H-imidazol-5-yl)piperidin-1-yl)aniline (1.1 g, 2.85 mmol) in CH₃CN (20 mL) was added t-BuNO₂ (0.59 g, 5.70 mmol) and CuBr₂ (0.64 g, 2.85 mmol) under ice-bath. The mixture was stirred at 20~25°C for 6h. LCMS showed no remaining starting material. The mixture was extracted with ethyl acetate (50 mL x 3). The combined organic layers were concentrated in vacuo. The residue was purified by chromatography (petroleum ether: ethyl acetate = 1:1, v/v) to afford 1-(2-bromophenyl)-4-(4-methyl-1-((2-(trimethylsilyl)ethoxy)methyl)-1H-imidazol-5-yl)piperidine (0.6 g, 46.8%) as a yellow solid.

LC-Mass Method: Mobile Phase: A: water (0.01% trifluoro acetic acid) B: acetonitrile (0.01% trifluoro acetic acid) gradient: 5% B for 0.2 min, increase to 95% B within 1.3 min, 95% B for 1.5 min, back within 0.01 min. Flow Rate: 2 ml/min; Column: Sunfire, 50*4.6mm, 3.5µm; Column Temperature: 50°C. LC purity: 95% (214 nm), Mass: 450.0 [M+1]⁺ at 1.679 min.

### Step D: Synthesis of 1-(4'-fluoro-3'-methyl-[1,1'-biphenyl]-2-yl)-4-(4-methyl-1-((2-(trimethylsilyl)ethoxy)methyl)-1H-imidazol-5-yl)piperidine

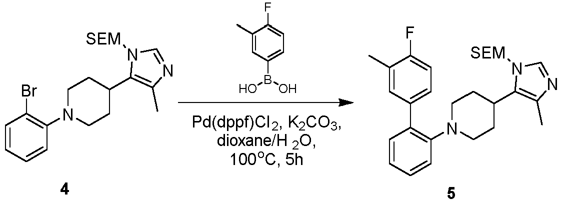

To a solution of 1-(2-bromophenyl)-4-(4-methyl-1-((2-(trimethylsilyl)ethoxy)methyl)-1H-imidazol-5-yl)piperidine (0.6 g, 1.33 mmol) and (4-fluoro-3-methylphenyl)boronic acid (0.25 g, 1.60 mmol) in dioxane/H₂O (6 mL/ 2 mL) was added K₂CO₃ (0.37 g, 2.66 mmol) and Pd(dppf)Cl₂ (95 mg, 0.13 mmol). The mixture was stirred at 100°C under N₂ for 5h. LCMS showed no remaining starting material. The mixture was concentrated in vacuo. The residue was purified by chromatography (petroleum ether: ethyl acetate = 1:1, v/v) to afford 1-(4'-fluoro-3'-methyl-[1,1'-biphenyl]-2-yl)-4-(4-methyl-1-((2-(trimethylsilyl)ethoxy)methyl)-1H-imidazol-5-yl)piperidine (0.35 g, 54.8%) as a yellow solid.

LC-Mass Method: Mobile Phase: A: water (0.01% trifluoro acetic acid) B: acetonitrile (0.01% trifluoro acetic acid) Gradient: 5% B for 0.2 min, increase to 95% B within 1.3 min, 95% B for 1.5 min, back within 0.01 min. Flow Rate: 2 ml/min; Column: Sunfire, 50*4.6mm, 3.5µm; Column Temperature: 50°C. LC purity: 80% (214 nm), Mass: 480.1 [M+1]⁺ at 1.76 min.

### Step E: Synthesis of 1-(4'-fluoro-3'-methyl-[1,1'-biphenyl]-2-yl)-4-(4-methyl-1H-imidazol-5-yl)piperidine

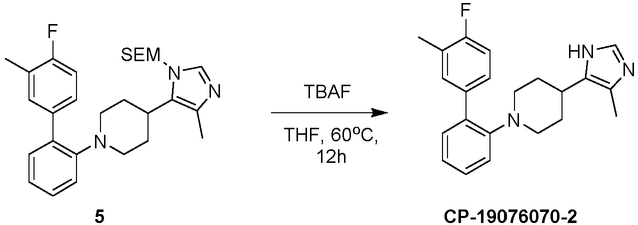

To a solution of 1-(4'-fluoro-3'-methyl-[1,1'-biphenyl]-2-yl)-4-(4-methyl-1-((2-(trimethylsilyl)ethoxy)methyl)-1H-imidazol-5-yl)piperidine (0.6 g, 0.42 mmol) in tetrahydrofuran (6 mL) was added tetra-n-butylammonium fluoride (2.1 mL, 2.1 mmol, 1N in tetrahydrofuran). The mixture was stirred at 60°C for 12 h. LCMS showed no remaining starting material. The mixture was extracted with ethyl acetate (20 mL x 3). The combined organic layers were concentrated in vacuo. The residue was purified by preparative HPLC to afford 1-(4'-fluoro-3'-methyl-[1,1'-biphenyl]-2-yl)-4-(4-methyl-1H-imidazol-5-yl)piperidine (50 mg, 34.3%) as a white solid.

LC-Mass Method: Mobile Phase: A: water (0.01% trifluoro acetic acid) B: acetonitrile (0.01% trifluoro acetic acid) Gradient: 5% B for 0.2 min, increase to 95% B within 1.3 min, 95% B for 1.5 min, back within 0.01 min. Flow Rate: 2 ml/min; Column: Sunfire, 50*4.6mm, 3.5µm; Column Temperature: 50°C. LC purity: 96% (214 nm), Mass: 350.2 [M+1]⁺ at 1.537 min.

¹H NMR (500 MHz, MeOD) δ 7.60 - 7.44 (m, 2H), 7.32 - 7.24 (m, 1H), 7.20 (dd, *J* = 7.5, 1.5 Hz, 1H), 7.16 - 6.96 (m, 3H), 3.20 (d, *J* = 12.0 Hz, 2H), 2.64 (t, *J* = 12.0 Hz, 3H), 2.34 (d, *J* = 1.5 Hz, 3H), 2.17 (s, 3H), 1.82 - 1.68 (m, 2H), 1.63 (d, *J* = 10.5 Hz, 2H).

### Synthesis of CP-19076070-3

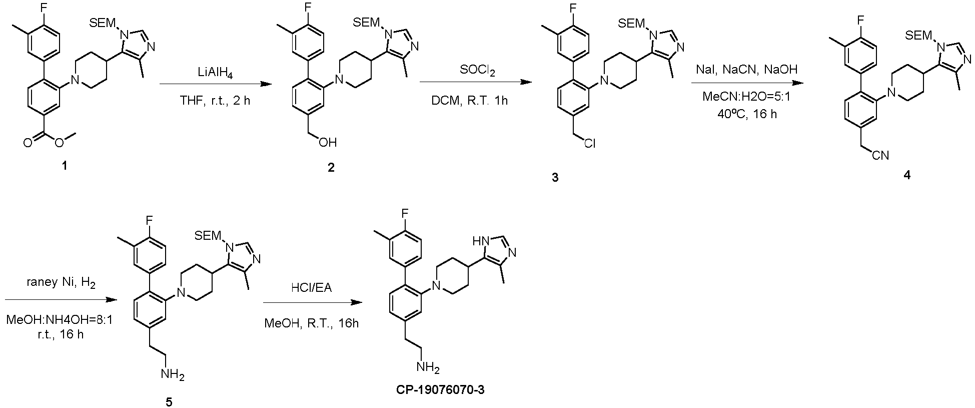

### Step A: Synthesis of methyl (4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)methanol

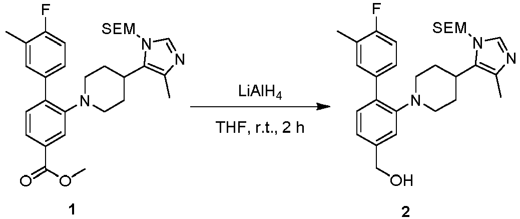

A mixture of methyl 4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-carboxylate (370 mg, 0.69 mmol) in dry tetrahydrofuran (10 mL) was added dropwise to LiAlH₄ (33 mg, 0.86 mmol) in dry tetrahydrofuran (10 mL) under ice-bath. The mixture was stirred at room temperature for 2 hours. Then H₂O (0.033 mL), NaOH solution (0.033 mL,15% in water) and H₂O (0.099 mL) was added, diluted with ethyl acetate:dichloro methane=1:1 (100 mL) and filtered. The solid was added ethyl acetate:dichloro methane=1:1 (100 mL) and tetrahydrofuran (5 mL), stirred 15 min and the filtered. The combined filtrates were concentrated and the residue was purified by chromatography on silica gel eluting with ethyl acetate - petroleum ether (0-100%) to give (4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)methanol (350 mg, 0.68 mmol, yield: 98%) as a yellow oil.

LC-Mass Method: A: H₂O (0.01% trifluoro acetic acid) B: acetonitrile (0.01% trifluoro acetic acid) Gradient: 5%-95% B in 1.3 min; Flow Rate: 1.8 ml/min; Column: Zorbox SB-C18, 4.6*30mm, 1.8um; Over Temperature: 40°C; LC purity: 99.30% (214 nm); Mass: 518.3 [M+H]⁺ at 1.24 min.

### Step B: Synthesis of methyl 1-(4-(chloromethyl)-4'-fluoro-3'-methyl-[1,1'-biphenyl]-2-yl)-4-(4-methyl-1-((2-(trimethylsilyl)ethoxy)methyl)-1H-imidazol-5-yl)piperidine

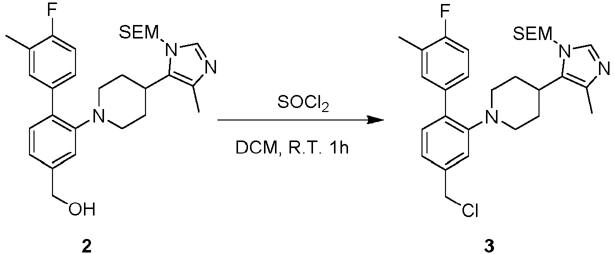

To a solution of (4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)methanol (350 mg, 0.09 mmol) in dichloro methane (10 mL) was added SOCl₂ (250 mg, 2.08 mmol) at 0 °C and the reaction was stirred at room temperature for 1 hr. The reaction mixture was concentrated in vacuo to give 1-(4-(chloromethyl)-4'-fluoro-3'-methyl-[1,1'-biphenyl]-2-yl)-4-(4-methyl-1-((2-(trimethylsilyl)ethoxy)methyl)-1H-imidazol-5-yl)piperidine (360 mg, 0.68 mmol, yield: 98%, HCl salt) as a yellow oil.

LC-Mass Method: A: H₂O (0.01% trifluoro acetic acid) B: acetonitrile (0.01% trifluoro acetic acid) Gradient: 5%-95% B in 1.3 min; Flow Rate: 1.8 ml/min; Column: Zorbox SB-C18, 4.6*30mm, 1.8um; Over Temperature: 40°C; LC purity: 93.49% (214 nm); Mass: 528.3 [M+H]⁺ at 1.41 min.

### Step C: Synthesis of methyl 2-(4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)acetonitrile

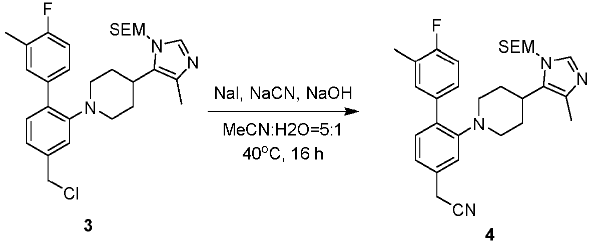

To a solution of 1-(4-(chloromethyl)-4'-fluoro-3'-methyl-[1,1'-biphenyl]-2-yl)-4-(4-methyl-1-((2-(trimethylsilyl)ethoxy)methyl)-1H-imidazol-5-yl)piperidine (424 mg, 0.80 mmol) in acetonitrile:H₂O=5:1 (5 mL) were added NaOH (aq. 0.1 mL), NaI (12 mg, 0.08 mmol) and NaCN (196 mg, 4.0 mmol) at room temperature and the reaction was stirred at 40°C for 16 hr. The reaction mixture was diluted with ethyl acetate (100 mL), washed with brine (100 mL), dried over Na₂SO₄, filtered and concentrated in vacuo. The residue was purified by column chromatography on silica gel (0-100% acetone in petroleum ether, v/v) to give 2-(4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)acetonitrile (150 mg, 0.29 mmol, yield: 36%) as a colorless oil. LC-Mass Method: A: H₂O (0.01% trifluoro acetic acid) B: acetonitrile (0.01% trifluoro acetic acid) Gradient: 5%-95% B in 1.3 min; Flow Rate: 2.0 ml/min; Column: Sunfire C18, 4.6×50mm, 3.5µm; Over Temperature: 50 °C; LC purity: 91.83% (214 nm); Mass: 519.2 [M+H]⁺ at 1.73 min.

### Step D: Synthesis of 2-(4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)ethan-1-amine

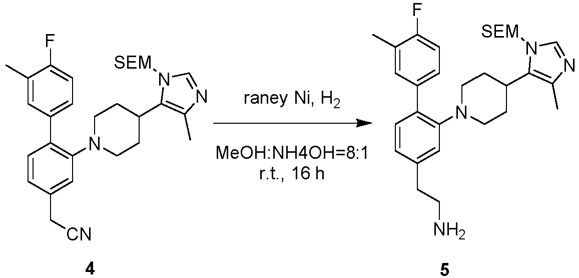

To a solution of 2-(4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)acetonitrile (150 mg, 0.29 mmol) in MeOH (8 mL) and NH₄OH (1 mL) was added Raney Ni (15 mg) at room temperature and the reaction was stirred at room temperature for 16 h. The reaction mixture was filtered and the mother liquid was concentrated in vacuo to give 2-(4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)ethan-1-amine (150 mg, 0.28 mmol, yield: 96%) as a colorless oil.

LC-Mass Method: A: H₂O (0.01% trifluoro acetic acid) B: acetonitrile (0.01% trifluoro acetic acid) Gradient: 5%-95% B in 1.3 min; Flow Rate: 2.0 ml/min; Column: Sunfire C18, 4.6×50mm, 3.5µm; Over Temperature: 50 °C; LC purity: 96.30% (214 nm); Mass: 523.2 [M+H]⁺ at 1.60 min.

### Step E: Synthesis of 2-(4'-fluoro-3'-methyl-2-(4-(4-methyl-1H-imidazol-5-yl)piperidin-1-yl)-[1,1'-biphenyl]-4-yl)ethan-1-amine

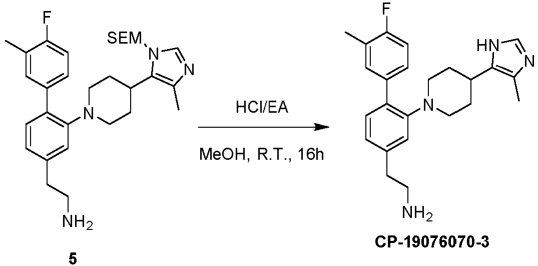

To a solution of 2-(4'-fluoro-3'-methyl-2-(4-(4-methyl-1-((2-(trimethylsilyl)ethoxy)methyl)-1H-imidazol-5-yl)piperidin-1-yl)-[1,1'-biphenyl]-4-yl)ethan-1-amine (150 mg, 0.28 mmol) in MeOH (5 mL) was added HCl/ethyl acetate (5 mL) at 0 °C and the reaction was stirred at R.T. for 16 hr. The reaction was purified by preparative HPLC to give 2-(4'-fluoro-3'-methyl-2-(4-(4-methyl-1H-imidazol-5-yl)piperidin-1-yl)-[1,1'-biphenyl]-4-yl)ethan-1-amine (20 mg, 0.05 mmol, yield: 18%) as a white solid.

LC-Mass Method: A: H₂O (0.01% trifluoro acetic acid) B: acetonitrile (0.01% trifluoro acetic acid) Gradient: 5%-95% B in 1.3 min; Flow Rate: 2.0 ml/min; Column: Sunfire C18, 4.6×50mm, 3.5µm; Over Temperature: 50°C; LC purity: 100% (214 nm); Mass: 393.1 [M+H]⁺ at 1.37 min.

¹H NMR (500 MHz, MeOD) δ 7.57 - 7.52 (m, 1H), 7.49 (d, *J* = 7.5 Hz, 1H), 7.44 (s, 1H), 7.18 (d, *J* = 7.5Hz, 1H), 7.09 (t, *J* = 9.0 Hz, 1H), 7.01 (s, 1H), 6.96 (d, *J* = 7.5 Hz, 1H), 3.23 (d, *J* = 12.0 Hz, 2H), 3.03 (q, *J* = 7.0 Hz, 2H), 2.85 (dd, *J* = 11.5, 7.5 Hz, 2H), 2.66 (t, *J* = 11.5 Hz, 2H), 2.64 - 2.59 (m, 1H), 2.35 (s, 3H), 2.19 (s, 3H), 1.77 (dd, *J* = 23.0, 12.0 Hz, 2H), 1.64 (d, *J* = 11.5 Hz, 2H).

¹⁹F NMR (376 MHz, MeOD) δ -122.84 (d, *J* = 6.0 Hz).

¹³C NMR (101 MHz, MeOD) δ 161.40 (s), 158.99 (s), 151.25 (s), 138.97 (s), 137.11 (d, *J* = 3.7 Hz), 132.41 (s), 131.46 (d, *J* = 4.9 Hz), 131.05 (s), 127.61 (d, *J* = 7.8 Hz), 124.03 (d, *J* = 17.1 Hz), 122.45 (s), 118.78 (s), 114.20 (d, *J* = 22.3 Hz), 52.05 (s), 48.24 (s), 48.03 (s), 47.81 (s), 47.60 (s), 47.39 (s), 47.17 (s), 46.96 (s), 42.26 (s), 37.39 (s), 33.61 (s), 31.86 (s), 13.21 (d,*J* = 3.6 Hz), 9.27 (s).

### Cell lines and viruses

HEK293T, LS180 were obtained from ATCC (Manassas, VA, USA), HAP1 WT, HAP1 -/-SLC38A2 (HZGHC001975c003), HAP1 -/-SLC38A9 (HZGHC000777c011) were obtained from Horizon Genomics. Other cell lines utilized here: CAKI (a gift of Georg Winter's lab), SW620, SW480 (both a gift from Walter Berger's lab, Medical University Vienna), LOVO, HCT15 (both a gift from Chris Gasche), KBM7 (a gift from Thijn Brummelkamp's lab), KBM7 -/- CRBN was described in Mayor-Ruiz et al. Cells were cultured, according to the ATCC recommendation in DMEM, RPMI or IMDM medium (Gibco) supplemented with 10% (v/v) FBS and antibiotics (100 U/ml penicillin and 100 mg/ml streptomycin). Knock-in single cell clones of dTAG-HA SLC38A2 HAP1 cell line were generated as previously described. For starvation, culture media was replaced with media without amino acids or FBS.

### Plasmids and stable cell line generation

SLC1A5, SLC2A1, SLC2A3, SLC16A1, SLC38A1, SLC38A2 were obtained as gateway-compatible pENTR/pDONR vectors from the Harvard PlasmID Repository. SLC39A7 was obtained from Dharmacon, GE Healthcare. The following vectors were synthesized by Genescript: SLC4A4, SLC7A3, SLC38A9, SLC25A1, SLC9A1, SLC9A2, SLC9A3, SLC9A4, SLC9A5, SLC9A6, SLC9A7, SLC9A8, and SLC9B2. SLC30A9, SLC33A1, SLC35B2, SLC25A26, and MTCH2 were a gift from the RESOLUTE consortium. cDNAs were transferred into gateway-compatible lentiviral expression vectors: pLX305 dTAG vectors (Addgene #91797/8) or pRRL Strep-HA (described previously43, EF1a promotor driven expression) using LR recombination (ThermoFisher Scientific). For the generation of lentiviral stable overexpression cells, HEK293T cells were transfected with psPAX2 (Addgene #12260) and pMD2.G (Addgene #12259) and expression vectors using polyethylenimine (PEI). 12 hours post transfection medium was replaced with fresh medium. The medium, containing the virus, was harvested 48 hours later, filtered (0.45 µm), supplemented with 5µg/ml Polybrene (Hexadimethrine bromide, Sigma) and added to target cells. 48 hours after transduction the medium was supplemented with the respective selection antibiotics (puromycin, blasticidin), to derive stably expressing cell populations. dTAG SLC38A2 knock-in cell line was generated using the vector pCRIS-PITCHv2-dTAG-BSD (Addgene #91792) and pX330A, as previously described14,42, using the following primers:
SLC38A2_5-PITCH (SEQ ID NO. 10): 
CGCGTTACATAGCATCGTACGCGTACGTGTTTGGTAGCTTGAAGAAGGCCGAAAC
CATGGCCAAGCCTTTGTCTCAAGAAGAATCC
SLC38A2_3-PITCH (SEQ ID NO. 11): 
CATCAGCATTCTAGAGCATCGTACGCGTACGTGTTTGGGGGGAAATACTGAATCG
TCCCATCTCTGCTTTCTTCATAGATCCGCCGCCACCCGAC
SLC38A2_gRNA_S (SEQ ID NO. 12):
   CACCGAATACTGAATCGTCCCATTT
SLC38A2_gRNA_AS (SEQ ID NO. 13):
   AAACAAATGGGACGATTCAGTATTC

### Antibodies and Immunoblotting

For immunoblotting, whole cell extracts were prepared using RIPA lysis buffer (25mM Tris/HCl pH 7.6, 150mM NaCl, 1% NP-40, 1% sodium deoxycholate, 0.1% SDS, EDTA-free protease inhibitor (Roche) and phosphatase inhibitors 2+3 (Sigma- Aldrich)). Cell extracts were incubated for 15 min on ice in 50 µL RIPA buffer. Lysates were cleared by centrifugation (20,000 g, 10 min, 4°C). Protein extracts were quantified and normalized using the BCA assay (Thermo Scientific). Where indicated, cleared lysates where treated with the enzyme PNGase (NEB) to deglycosylate the proteins. Lämmli Sample Buffer 4x was added to protein extract samples without boiling. Cell lysates were run on SDS-polyacrylamide gel in tris-glycine running buffer and transferred to nitrocellulose membranes Amersham Protran 0.45 µm (GE Healthcare), according to standard methods. The membranes were incubated with the antibodies indicated below and visualized with horseradish peroxidase-conjugated secondary antibodies (described below) using the ECL Western blotting system (Thermo Scientific). The following antibodies were used: GAPDH (Santa Cruz, sc-365062), Actin (Cyoskeleton Inc., #AAN01), HA (Covance, cat #MMS-101R), HA (Cell Signaling, #3724), HA7-HRP (Sigma, H6533), HA (Roche, #11867423001), SLC9A1 (Santa Cruz, sc-136239), SLC2A1 (Cell Signaling, #12939), SLC38A1 (Cell Signaling, #36057). Secondary antibody for imaging goat anti-Rat IgG Alexa Fluor 488 (ThermoFisher, A11006). The following secondary antibodies were used for immunoblotting: goat anti-mouse HRP (115-035-003, Jackson ImmunoResearch) and goat anti-rabbit HRP (111-035-003, Jackson ImmunoResearch).

### Immunofluorescence staining and imaging

For immunofluorescence detection of the expressed SLCs in HAP1 cells, cells were seeded onto poly-L-lysine hydrobromide (P6282, Sigma-Aldrich)-coated 96-well CellCarrier Ultra plates (PerkinElmer) or onto 13mm No. 1.5H cover glasses (PaulMarienfeld). Cells were incubated and thereafter fixed with 4% formaldehyde in PBS 1x. After fixation, cells were incubated in blocking buffer (0.3% Saponin (47036, Sigma- Aldrich), 10% FCS in PBS 1x) for 1 hour at room temperature (RT), rocking. Primary antibody staining was performed for 2 hours at RT in blocking solution and fluorophore conjugated secondary antibody staining was applied for 1 hour after 3 washes in blocking solution. Final washing was performed three times and counterstaining was done for 15 min at RT with DAPI (1:1000 in PBS 1x) for nuclei and HCS CellMask Deep Red Stain (1:20000 in PBS 1x). Cover glasses were mounted using ProLong Gold antifade reagent (Thermo Scientific) and imaged using a Zeiss LSM-780 confocal microscope. Imaging of CellCarrier Ultra plates was performed on an Opera Phenix High Content Screening System (Perkin Elmer).

### Automated image analysis

For the quantification of cellular HA-tag intensities, CellProfiler v3.1.8 was used. In brief, nuclei were identified from DAPI staining and cells as secondary objects from CellMask images. HA-tag intensities were quantified on a single cell level and consecutive plotting of the data was performed in R 3.4.4.

### Chemicals

MG132, Bortezomib, Pomalidomide were obtained from Selleckchem. MLN4924 was obtained from MedchemExpress. Bafilomycin was obtained from Enzo Life Sciences. dTAG13 was a generous gift from the Nathanael Gray laboratory, Dana Farber Cancer Institute. The d9A1 degrader series (warhead, d9A1-1 to d9A1-4) was synthesized by Wuxi AppTec. The synthesis route and NMR data are provided in Figure 8. All chemicals were dissolved in DMSO (Sigma-Aldrich) and utilized at the concertation described in respective figures.

### Results

SLCs were ectopically expressed as fusions to a mutated FKBP domain, also known as the dTAG system. In brief, a dTAGed protein is amenable to degradation by specific chimeric degrader molecules (e.g. dTAG7/dTAG13) that simultaneously bind to the dTAG and the CRL4CRBN E3 ligase, inducing molecular proximity and ensuing degradation by the proteasome. Given that SLCs were expressed in all cellular compartments, the amenability of differentially located SLCs to targeted proteolysis was tested (Figure 1A, upper graph). Each of the tested SLCs was stably expressed in HAP1 cells, and subcellular localization was assessed by immunofluorescence microscopy (Figure 1B). In detail, SLCs located at the cell surface (SLC1A5, SLC38A1, SLC2A1, SLC2A3, SLC16A1, SLC4A4, SLC7A3), cell surface and vesicles (SLC38A2), lysosome (SLC38A9), Golgi (SLC35B2, SLC33A1), mitochondria (SLC25A26, SLC25A1, and MTCH2) and endoplasmic reticulum (SLC39A7, SLC30A9) were assayed. Cellular treatment with a specific degrader molecule (dTAG7 or dTAG13) led to identification of SLCs expressed at cell surface, endoplasmic reticulum and lysosome that were amenable to targeted degradation (Figure 1C, 5A). The dTAG could be placed on either N- or C-terminus of the SLC, if both face the cytoplasm (Figure 5B), as exemplified by SLC2A3. The outer mitochondrial protein MTCH2 (SLC25A50) was amenable to degradation (Figure 1B), while the inner mitochondrial SLCs tested could not be degraded within the assayed timeframe (Figure 5C). While some SLCs, such as SLC38A2 or SLC2A3 could be completely degraded following treatment, other SLCs such as SLC35B2 or SLC39A7 were not degraded to completion (see Table 1).

Table 1: summarizing the list of SLC proteins tested as the targets of degradation in Figure 1 and 5, as well as the number of trans-membrane (TM) segments predicted by PROTTER (Omasits U1, Ahrens CH, Müller S, Wollscheid B. Protter: interactive protein feature visualization and integration with experimental proteomic data. Bioinformatics. 2014 Mar 15;30(6):884-6, their subcellular localization and how amendable to degradation these proteins were. PM: plasma membrane; ER: endoplasmic reticulum; in.: inner; out.: outer.

**Table 1**

| SLC | Predicted, TMs | Cellular localization | Amenable to targeted degradation |
|---|---|---|---|
| SLC1A5 | 8 | PM | +++ |
| SLC2A1 | 13 | PM | ++ |
| SLC2A3 | 12 | PM | +++ |
| SLC16A1 | 12 | PM | ++ |
| SLC38A1 | 11 | PM | +++ |
| SLC38A2 | 11 | PM/vesicles | +++ |
| SLC38A9 | 11 | Lyosome | +++ |
| SLC30A9 | 5 | ER | ++ |
| SLC39A7 | 6 | ER | ++ |
| SLC33A1 | 11 | ER/Golgi | ++ |
| SLC35B2 | 9 | Golgi | + |
| SLC25A1 | 6 | Mitochondria (in.) | - |
| SLC25A26 | 6 | Mitochondria (in.) | - |
| MTCH2 | 3 | Mitochondria (out.) | + |

Complete degradation did not appear to correlate with the stable expression level of the dTAG protein nor with the expression of the endogenous protein (Figure 5D). Targeted degradation was also validated via protein-specific antibodies, which generally showed good consistency with detection via the HA epitope of the dTAG (Figure 5E and F). Of note, temporal control of targeted SLC degradation could be influenced by the choice of the respective heterobifunctional molecule: degradation with the PROTAC dTAG7 led to reversible degradation, while dTAG13 maintained the target degradation for at least 72h (Figure 5E and F). Collectively, these data confirm that an intracellular target engagement is sufficient to recruit the CRL^{CRBN} E3 ligase complex and prompt SLC degradation. Near-complete degradation could be achieved in the low nanomolar range for some SLCs, such as SLC38A2, although the dose of degrader required for maximal target degradation varied based on the studied SLC (Figure 2A, 6A). Targeted degradation of SLCs was typically initiated within 3-6 hours at least depending on the SLC (Figure 2B, 6B). Degradation of ectopically expressed dTAG-SLCs was not unique to the HAP1 cell line, but could also be achieved in other cancer cell lines such as HCT15, LS180 and others (Figure 2C, 6C). To validate that the observed SLC destabilization was dependent on an active CRL4^{CRBN} complex, chemical competition experiments were set up. dTAG7 mediated degradation was blocked by inhibiting cullin neddylation, and thus CRL activity, by co-treatment with the NAE1 inhibitor MLN4924 (Figure 2D, 6D). Similarly, saturating the CRBN binding site by treatment with excess concentration of pomalidomide prevented measurable SLC degradation. Finally, treatment with proteasome inhibitors (bortezomib or MG132) rescued SLCs degradation. Noteworthy, degradation appeared unaffected upon cotreatment with the lysosomal modulator bafilomycin A1 (Figure 2D). Thus, targeted degradation of SLCs appears to utilize the proteasome but not the lysosome machinery. Next, knock-in the dTAG domain at a genomic locus to demonstrate feasibility of chemical control of an endogenous SLC protein was performed. SLC38A2 was selected for endogenous tagging at the N-terminus (Figure 3A). SLC38A2 protein levels have been shown to be kept low under normal culture conditions and strongly induced following amino acid starvation (Nardi, F. et al. Proteasomal modulation of cellular SNAT2 (SLC38A2) abundance and function by unsaturated fatty acid availability. J. Biol. Chem. 290, 8173-8184 (2015)). Indeed, in the cell line model used, the endogenously tagged SLC38A2 protein was rapidly expressed within a few hours and could localize to cell membranes upon starvation (Figure 3B). Despite the strong induction of expression, the transporter remained amenable to targeted degradation (Figure 3C). It was found that even under this strong stimulus, SLC38A2 expression was completely ablated by 2 hours of pre-treatment with dTAG13, while co-treatment reduced SLC38A2 expression by more than 50% (Figure 3D). Altogether, the data for exogenous and endogenous expression of dTAGed SLCs indicated that multi-pass transmembrane proteins, such as SLCs, are within reach of the recruited proteolytic machinery and amenable to proteasome mediated targeted degradation. However, the use of genetically encoded degradation systems, such as the dTAG approach, limits its use to established and genetically tractable cell culture systems. To investigate whether SLCs could be degraded by chemically reprogramming the CRL4^{CRBN} E3 ligase complex, a directly-acting first in class, SLC degrader was designed. SLC9A1, also known as NHE1, is an electroneutral and reversible ion transporter that exchanges one Na+ ion for one H+ ion. It contributes to both cytoplasmic alkalization (pHi), and acidification of the microenvironment (Parks, S. K., Chiche, J. & Pouysségur, J. Disrupting proton dynamics and energy metabolism for cancer therapy. Nature Reviews Cancer 13, 611-623 (2013)). It has been previously shown to be involved in cancer and therefore is an attractive model target for our purpose (Stock, C. & Pedersen, S. F. Roles of pH and the Na+/H+ exchanger NHE1 in cancer: From cell biology and animal models to an emerging translational perspective, Seminars in Cancer Biology 43, 5-16 (2017)). A focused library of putative SLC9A1 degraders was synthesized by systematically varying linker length, composition and attachment chemistry to the pomalidomide-based CRBN binder (Figure 4A, 7A). Degradation of SLC9A1 was assessed in two cell lines, HAP1 and KBM7. The most potent degrader, denoted here as d9A1-2, led to degradation of endogenous SLC9A1 at sub-micromolar concentration within eight hours post treatment (Figure 4B, 7B). One additional degrader of the same series, denoted as d9A1-3, may also lead to degradation of the target, albeit at lower potency (Figure 7A), stressing the importance of linker design in generating an efficient degrader. As expected, genetic depletion of the E3 ligase adaptor CRBN ablated the degradation of SLC9A1 (Figure 4C, 7C). Again in line with a mechanism of controlled proteolysis, SLC9A1 destabilization was abrogated by blocking CRL activity via pharmacologic inhibition of NAE1 via MLN4924. Similarly, competition with excess amounts of pomalidomide blocked d9A1-2-induced degradation of SLC9A1 (Figure 4D, 7C). Treatment with proteasome inhibitors (bortezomib or MG132), but not bafilomycin A1, rescued SLCs degradation (Figure 4D). Next, the selectivity for the degradation of SLC9A1 over other SLC9 family members was assessed. In order to render this survey independent of endogenous transporter expression levels in HAP1 cells, tagged proteins were ectopically overexpressed, corresponding to nine members of the SLC9 family (A1,A2,A3,A4,A5,A6,A8,A9,B2). At 16 hours post treatment with d9A1-2 at 250 or 750 nM, SLC9A1 demonstrated the most efficient degradation. In addition, d9A1-2 also prompted degradation of the closely-related SLC9A2 and SLC9A4 (Figure 4E). Comparatively, abundance of the other assayed SLCs was not significantly affected at these experimental conditions. Thus, while not exclusively selective for SLC9A1, d9A1-2 features some level of intra-family selectivity. These experiments further attest to the degradability of SLCs by heterobifunctional degraders. Collectively, the data demonstrates the amenability of multi-pass transmembrane proteins to chemically induced degradation and introduce the first SLC-directed PROTAC.

### EXAMPLE 2

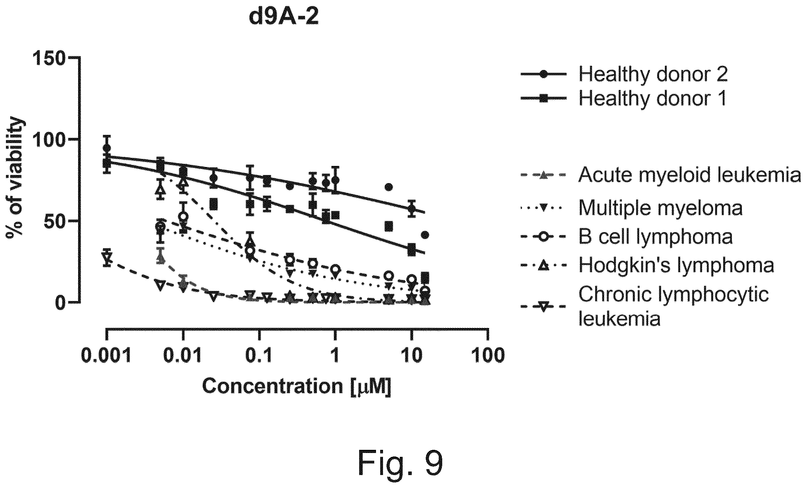

### Cellular Viability Assay as shown in Figure 9

Peripheral blood mononuclear cells (PBMCs) were isolated using Lymphoprep (Stem Cell Technologies) from healthy donors and patients with acute myeloid leukemia, multiple myeloma, B cell lymphoma, Hodgkin's lymphoma, chronic lymphocytic leukeumia. For comparison of different samples, compounds were transferred on black 384-well plates using an acoustic liquid handler (Echo, Labcyte). Cells were seeded in these plates at densities of 20,000 cells per well in RPMI media (Gibco), supplemented with 10% fetal calf serum (Gibco). Viability was measured after 72 hours using the CellTiter-Glo assay (Promega) on a plate reader (SpectraMax i3x, Molecular Probes). All measurements were done in technical quadruplicates. Toxicity achieved by treatment with d9A1-2 was normalized to the reference controls. Dose response fitting curves were obtained and plotted using GraphPad Prism V8. Data is presented as mean +/- SD.

The data demonstrates that d9A1-2 is more toxic in patient samples, than in healthy donor samples.

### Cell Viability Assay as shown in Figure 10

Peripheral blood mononuclear cells (PBMCs) were isolated using Lymphoprep (Stem Cell Technologies) from healthy donors and patients with acute myeloid leukemia, multiple myeloma, B cell lymphoma, Hodgkin's lymphoma, chronic lymphocytic leukeumia. For comparison of different samples, compounds were transferred on black 384-well plates using an acoustic liquid handler (Echo, Labcyte).

Cells were seeded in these plates at densities of 20,000 cells per well in RPMI media (Gibco), supplemented with 10% fetal calf serum (Gibco). Viability was measured after 72 hours using the CellTiter-Glo assay (Promega) on a plate reader (SpectraMax i3x, Molecular Probes). All measurements were done in technical quadruplicates. Toxicity achieved by treatment with d9A1-2 or ethyl-isopropyl amiloride (EIPA) (Sigma) was normalized to the reference controls. Dose response fitting curves were obtained and plotted using GraphPad Prism V8. Data is presented as mean +/- SD.

Treatment with 75nM d9A1-2 is considerably more toxic than 7.5µM EIPA in the patient samples, demonstrating the improved effect of d9A-12 in terms of toxicity.

## Claims

1. A compound having the following formula (I): 
Transmembrane Protein
Binding Moiety
(TMPBM)
Linker (L)
E3 Ligase Binding Moiety
(EBM)
 wherein
TMPBM is a moiety binding to a transmembrane protein,
L is a linker moiety; and
EBM is a moiety modifying the function of the E3 ligase and/or binding to at least one member of the E3 ligase complex,
wherein TMPBM is a moiety having the following partial formula (II) or a stereoisomer, tautomer, pharmaceutically acceptable salt or solvate: 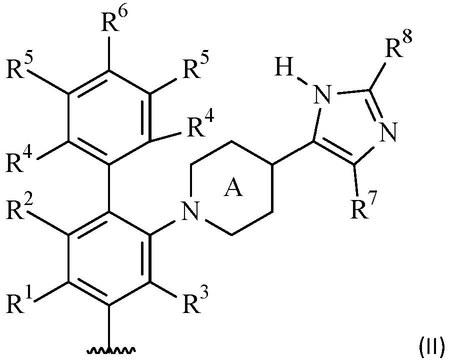 wherein
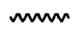 indicates the position of attachment of the partial formula (II) to the linker (L),
R¹, R², R³, R⁴, R⁵, R⁶, R⁷ and R⁸ are each independently selected from -H, -halogen, - NO₂, -CN, -C₁₋₃ alkyl, -OH, -O-C₁₋₂ alkyl, NH₂, -NH(C₁₋₂ alkyl), -N(C₁₋₂ alkyl)₂, -CHO, - CO(C₁₋₂ alkyl), COOH, COO(C₁₋₂ alkyl), CONH₂, CONH(C₁₋₂ alkyl), and CON(-C₁₋₂ alkyl)₂, wherein each C₁₋₂ alkyl is optionally substituted with one or more F,
and Ring A is optionally substituted with one or more F.

2. The compound according to claim 1, wherein in the moiety of partial formula (II) the following definitions apply:
R¹, R², R³ and each R⁴ are each hydrogen,
R⁷ is methyl,
R⁸ is H or NH₂,
and Ring A is not substituted with any F.

3. The compound according to claim 1 or 2, wherein in the moiety of partial formula (II) the following definitions apply:
one R⁵ is selected from H, F, Cl, Me, CF₃, CF₂H and CH₂F,
the other R⁵ is H, and
R⁶ is F or Cl.

4. The compound according to any one of claims 1 to 3, wherein the moiety of partial formula (II) has the following formula (IIa):

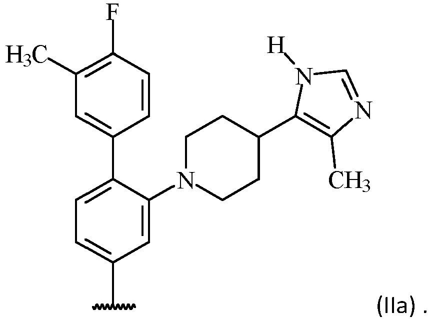

5. The compound according to any one of claims 1 to 4, wherein L is selected from C₁₄₋₂₄ alkylene, C₁₄₋₂₄ alkenylene, and C₁₄₋₂₄ alkynylene, wherein said alkylene, said alkenylene and said alkynylene are each optionally substituted with one or more groups independently selected from halogen, C₁₋₅ haloalkyl, -O(C₁₋₅ haloalkyl), -CN, -OR²¹, -NR²¹R²¹, -NR²¹OR²¹, -COR²¹, -COOR²¹, -OCOR²¹, -CONR²¹R²¹, -NR²¹COR²¹, -NR²¹COOR²¹, -OCONR²¹R²¹, -SR²¹, -SOR²¹, -SO2R²¹, -SO₂NR²¹R²¹, -NR²¹SO₂R²¹, -SO₃R²¹, and -NO₂, and further wherein one or more -CH₂- units comprised in said alkylene, said alkenylene or said alkynylene are each optionally replaced by a group independently selected from - O-, -NR²¹-, -CO-, -S-, -SO-, and -SO₂-;
each R²¹ is independently selected from hydrogen, C₁₋₅ alkyl, C₂₋₅ alkenyl, C₂₋₅ alkynyl, carbocyclyl, and heterocyclyl, wherein said alkyl, said alkenyl and said alkynyl are each optionally substituted with one or more groups R^{Alk}, and further wherein said carbocyclyl and said heterocyclyl are each optionally substituted with one or more groups R^{Cyc};
any two R²¹ are optionally linked to form a ring;
each R^{Alk} is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl);
each R^{Cyc} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), -NH-OH, -N(C₁₋₅ alkyl)-OH, -NH-O(C₁₋₅ alkyl), -N(C₁₋₅ alkyl)-O(C₁₋₅ alkyl), halogen, C₁₋₅ haloalkyl, -O(C₁₋₅ haloalkyl), -CN, -NO₂, -CHO, -CO(C₁₋₅ alkyl), -COOH, -COO(C₁₋₅ alkyl), -O-CO(C₁₋₅ alkyl), -CO-NH₂, -CO-NH(C₁₋₅ alkyl), -CO-N(C₁₋₅ alkyl)(C₁₋₅ alkyl), -NH-CO(C₁₋₅ alkyl), -N(C₁₋₅ alkyl)-CO(C₁₋₅ alkyl), -NH-COO(C₁₋₅ alkyl), -N(C₁₋₅ alkyl)-COO(C₁₋₅ alkyl), -O-CO-NH(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO₂-(C₁₋₅ alkyl), -SO-(C₁₋₅ alkyl), aryl, heteroaryl, cycloalkyl, and heterocycloalkyl, wherein said aryl, said heteroaryl, said cycloalkyl, and said heterocycloalkyl are each optionally substituted with one or more groups independently selected from C₁₋₅ alkyl, C₂₋₅ alkenyl, C₂₋₅ alkynyl, halogen, C₁₋₅ haloalkyl, -CN, -OH, -O(C₁₋₅ alkyl), -SH, -S(C₁₋₅ alkyl), -NH₂, -NH(C₁₋₅ alkyl), and -N(C₁₋₅ alkyl)(C₁₋₅ alkyl).

6. The compound according to claim 5, wherein
(i) the C₁₄₋₂₄ alkylene, C₁₄₋₂₄ alkenylene, and C₁₄₋₂₄ alkynylene are C₁₅₋₂₂ alkylene, C₁₅₋₂₂ alkenylene, and C₁₅₋₂₂ alkynylene, respectively, preferably C₁₆₋₂₀ alkylene, C₁₆₋₂₀ alkenylene, and C₁₆₋₂₀ alkynylene respectively wherein each of the alkylenes, alkenylenes and alkynylenes may be modified as set out in claim 5;
(ii) L is selected from C₁₄₋₂₄ alkylene and C₁₄₋₂₄ alkenylene, more preferably C₁₅₋₂₂ alkylene, C₁₅₋₂₂ alkenylene, even more preferably C₁₆₋₂₀ alkylene, still more preferably C₁₇₋₁₉ alkylene, most preferably C₁₇ alkylene, wherein each of the alkylenes and alkenylenes may be modified as set out in claim 5;
(iii) L has one of the following structures:
-O-L¹-NH-C(=O)- and -NH-L¹-NH-C(=O)-
wherein L¹ is selected from C₁₃₋₁₇ alkylene and C₁₃₋₁₇ alkenylene, wherein said alkylene and said alkenylene are each optionally substituted with one or more groups independently selected from halogen, C₁₋₃ alkyl and C₁₋₃ haloalkyl, and further wherein one or more -CH₂- units comprised in said alkylene or said alkenylene is/are replaced by a group independently selected from -O-, -NR²¹-, -CO-, -S-, -SO-, and -SO₂-; wherein it is preferred that the -O- or -NH- on the left hand side binds to EBM as defined in any of the preceding items and the NH-C(=O)- on the right hand side is bound to the TMPBM as defined in any of the preceding items; or
(iv) L has one of the following structures:
-O-L¹-N H-C(=O)- and -NH-L¹-NH-C(=O)-
wherein L¹ is selected from C₁₃₋₁₇ alkylene, wherein said alkylene is optionally substituted with one or more groups independently selected from halogen, C₁₋₃ haloalkyl and C₁₋₃ haloalkyl, and further wherein one or more -CH₂- units comprised in said alkylene or said alkenylene is/are replaced by a group independently selected from -O-, -NR²¹ and -CO-; wherein it is preferred that the -O- or -NH- on the left hand side binds to EBM as defined in any of the preceding items and the NH-C(=O)- on the right hand side is bound to the TMPBM as defined in any of the preceding items.

7. The compound of formula (I) according to any one of the preceding claims, wherein EBM is a moiety of partial formula (III): 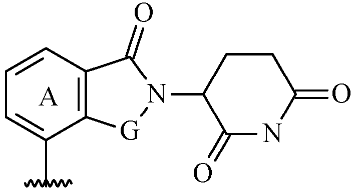 wherein
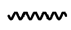 indicates the position of attachment of the partial formula (III) to the linker (L),
G is selected from -C(=O)- and CH₂,
and Ring A is optionally substituted with one or more F,
or any stereoisomer thereof.

8. The compound according to claim 7, wherein in partial formula (III), G is -C(=O)- and Ring a is not substituted with any F.

9. A composition comprising a compound of any of claims 1 to 8.

10. The compound of any of claims 1 to 8 or the composition of claim 9 further comprising a pharmaceutically acceptable diluent, excipient or carrier.

11. The compound of any of claims 1 to 8 or 10 or the composition of claim 9 or 10 for use as a medicament.

12. The compound of any of claims 1 to 8, 10 or 11, or the composition of any of claims 9 to 11 for use in treating or preventing cancer, particularly leukemia such as chronic myeloid leukemia.

13. The compound of any of claims 1 to 8, 10 or 11, or the composition of any of claims 9 to 11 for use in treating or preventing a disease selected from: 2p21 microdeletion syndrome, abnormal coordination, abnormality of the cornea, absence seizures, ache, type ib (disorder) achondrogenesis, acidosis, acrodermatitis, acrodermatitis enteropathica, acute cerebrovascular accidents, acute confusional senile dementia, acute kidney injury, acute kidney insufficiency, acute lung injury, adenocarcinoma of lung (disorder), adenoid cystic carcinoma, adenoma, basal cell adenoma, microcystic adenoma, monomorphic adenoma, trabecular adenoma, adult fanconi syndrome, adult learning disorders, adult neuronal ceroid lipofuscinosis, adult-onset citrullinemia type 2, adverse reaction to drug, psychotic affective disorders, age related macular degeneration, age-related memory disorders, akinetic-rigid variant of Huntington disease, ocular albinism, alcohol abuse, alcohol sensitivity, alcohol use disorder, alcohol withdrawal seizures, alcohol withdrawal-induced major motor seizure, alcoholic intoxication, chronic alcoholic intoxication, Allan-Herndon-Dudley syndrome (ahds), allodynia, alloxan diabetes, alternating hemiplegia of childhood, early onset Alzheimer disease, late onset Alzheimer disease, Alzheimer's disease, focal onset Alzheimer's disease, amelogenesis imperfecta, amelogenesis imperfecta hypomaturation type, hypomaturation type iia5 amelogenesis imperfecta, type iii amelogenesis imperfecta, inborn errors amino acid metabolism, inherited disorders amino acid metabolism, amphetamine abuse, amphetamine addiction, amphetamine-related disorders, amyotrophic lateral sclerosis, amyotrophic lateral sclerosis with dementia, guam form amyotrophic lateral sclerosis, anaplastic carcinoma, anasarca, anemia, hemolytic anemia, acquired hemolytic anemia, idiopathic acquired hemolytic anemia, hereditary spherocytic hemolytic anemia, hypochromic microcytic anemia with iron overload, hypochromic microcytic anemia with iron overload 1, microangiopathic anemia, pyridoxine-refractory sideroblastic anemia 2, pyridoxine-refractory autosomal recessive sideroblastic anemia, anhedonia, animal mammary neoplasms, anxiety disorders, anxiety neurosis (finding), neurotic anxiety states, thoracic aortic aneurysm, aortic valve calcification, apnea, aprosodia, arterial tortuosity, arterial tortuosity syndrome, arthrogryposis, arthrogryposis, mental retardation and seizures, as if personality, asperger syndrome, ataxia, appendicular ataxia, motor ataxia, sensory ataxia, truncal ataxia, hereditary ataxias, atelosteogenesis type 2, atherogenesis, atherosclerosis, atonic absence seizures, atrophic, attention deficit disorder, attention deficit hyperactivity disorder, attention deficit hyperactivity disorder (ADHD), atypical ductal breast hyperplasia, aura, autism spectrum disorders, autistic disorder, autoimmune hemolytic anemia, autosomal dominant juvenile parkinson disease, autosomal dominant parkinsonism, autosomal recessive parkinsonism, autosomal recessive polycystic kidney disease, autosomal recessive primary microcephaly, autosomal recessive sideroblastic anemia, avoidant personality disorder, awakening epilepsy, nonobstructive azoospermia, Barrett epithelium, Barrett esophagus, Bartter disease, antenatal type 1 Bartter syndrome, basal cell carcinoma, idiopathic basal ganglia calcification 6, biotin-responsive basal ganglia disease, basal ganglia diseases, benign neoplasm, bilateral deafness, bile acid coa ligase deficiency and defective amidation, primary bile acid malabsorption, biliary cirrhosis, secondary biliary cirrhosis, bipolar disorder, bipolar i disorder, Birk-Landau-Perez syndrome, bladder neoplasm, blastocyst disintegration, body mass index quantitative trait locus 11, body mass index quantitative trait locus 4 (disorder), bone diseases, developmental bone diseases, brain diseases, brain dopamine-serotonin vesicular transport disease, brain hemorrhage, brain injuries, focal brain injuries, brain ischemia, brain lacerations, breast cancer, familial breast cancer, breast carcinoma, brittle diabetes, bronchioloalveolar adenocarcinoma, brown oculocutaneous albinism, Brown-Vialetto-van Laere syndrome, Brown-Vialetto-van Laere syndrome 2, brucellosis, pulmonary brucellosis, bulbar palsy, burn induced multiple organ injury, buruli ulcer, calcinosis, calcium pyrophosphate arthropathy, calcium pyrophosphate deposition disease, embryonal cancer, embryonal and mixed cancer, cannabis use, carbohydrate deficient glycoprotein syndrome type 2k, carcinoma, carcinoma of lung, spindle-cell carcinoma, carcinomatosis, cardiac arrhythmia, cardiac hypertrophy, cardiac ischaemic reperfusion, cardiomegaly, dilated cardiomyopathy, familial idiopathic cardiomyopathy, cardiovascular diseases, carditis, carnitine-acylcarnitine translocase deficiency, cataract, cataract 47, cataract and cardiomyopathy, juvenile cataract with microcornea and glucosuria, celiac disease, central hypothyroidism, cerebral injury, cerebral ischemia, cerebrovascular accident, cerebrovascular disorders, neuronal ceroid lipofuscinosis 7, parry type neuronal ceroid lipofuscinosis, ceruloplasmin deficiency, cervical cancer, Charcot-Marie-Tooth disease, type ia (disorder) Charcot-Marie-Tooth disease, type ib Charcot-Marie-Tooth disease, chemical and drug induced liver injury, chemically-induced liver toxicity, child development deviations, specific child development disorders, childhood acute lymphoblastic leukemia, childhood progressive bulbar palsy, childhood tic disorders, cholangiocarcinoma, choledochal cyst, type i choledochal cyst, type ii choledochal cyst, type iii choledochal cyst, type iv choledochal cyst, type v choledochal cyst, cholestasis, cholestasis in newborn, episodic choreoathetosis/spasticity, chromophobe renal cell carcinoma, chronic depression, chronic motor or vocal tic disorder, cirrhosis, type ii neonatal-onset citrullinemia, cleft palate, isolated cleft palate, cleft upper lip, cluttering, cocaine abuse, cocaine dependence, cocaine-induced mood disorder, cocaine-related disorders, cognition disorders, cognitive impairment, cold hemagglutinin disease, collecting duct carcinoma of the kidney, collodion fetus, colon cancer, colonic neoplasms, colorectal cancer, colorectal carcinoma, colorectal neoplasms, combined d-2- and l-2-hydroxyglutaric aciduria, combined oxidative phosphorylation deficiency 18, combined oxidative phosphorylation deficiency 28, complete hearing loss, complex partial seizures, complex partial status epilepticus, preneoplastic condition, conduction disorder of the heart, congenital nonprogressive cone-rod synaptic disorder, congenital abnormality, congenital anemia, congenital aneurysm of ascending aorta, congenital cataracts, hearing loss and neurodegeneration, congenital chloride diarrhea, type 2c congenital disorder of glycosylation, type iif congenital disorder of glycosylation, type iim congenital disorder of glycosylation, type iin congenital disorder of glycosylation, congenital disorders of glycosylation, congenital glucose-galactose malabsorption, congenital heart defects, congenital hypothyroidism, congenital ichthyosis, postsynaptic congenital myasthenic syndromes, presynaptic congenital myasthenic syndromes, congenital myopathy (disorder), congenital nonbullous ichthyosiform erythroderma, sodium type (disorder) congenital secretory diarrhea, congestive heart failure, connective tissue diseases, constipation, constipation-predominant irritable bowel syndrome, contact dermatitis, contact hypersensitivity, conventional (clear cell) renal cell carcinoma, convulsions, convulsive seizures, corneal dystrophy and perceptive deafness, fuchs endothelial corneal dystrophy 4, corneal endothelial dystrophy 2, corneal opacity, corpus callosum agenesis neuronopathy, cortical dysplasia, coxsackievirus infections, craniofacial abnormalities, autosomal dominant craniometaphyseal dysplasia, cranioosteoarthropathy, craniosynostosis, x-linked creatine deficiency, crohn disease, Crohn's disease of large bowel, Crohn's disease of the ileum, stomatin-deficient cryohydrocytosis with mental retardation, seizures, cataracts, and massive hepatosplenomegaly, cystic fibrosis, cystic fibrosis modifier 1, cystinuria, type a cystinuria, type a-b cystinuria, type b cystinuria, cytopenia, de la chapelle dysplasia, de Toni-Debre-Fanconi syndrome, deaf mutism, deafness, acquired deafness, autosomal dominant 25 (disorder) deafness, autosomal dominant 72 deafness, autosomal recessive 4 deafness with enlarged vestibular aqueduct, autosomal recessive 61 deafness, deficiency of glutamate decarboxylase, degenerative polyarthritis, delirium, dementia, depressed mood, depression, bipolar depression, neurotic depression, postpartum depression, depressive disorder, treatment-resistant depressive disorder, depressive symptoms, depressive syndrome, allergic contact dermatitis, atopic dermatitis, developmental academic disorder, developmental disabilities, diabetes mellitus, experimental diabetes mellitus, insulin-dependent diabetes mellitus, ketosis-prone diabetes mellitus, non-insulin-dependent diabetes mellitus, diabetes mellitus type 2, autoimmune diabetes, diabetic cardiomyopathies, diabetic cataract, diarrhea, diastrophic dysplasia, dicarboxylicaminoaciduria, disease exacerbation, distal renal tubular acidosis, down syndrome, partial trisomy 21 down syndrome, drug abuse, drug dependence, drug habituation, drug toxicity, drug use disorders, drug withdrawal symptoms, drug-induced acute liver injury, drug-induced depressive state, drug-induced liver disease, ductal carcinoma, dysarthria, flaccid dysarthria, guttural dysarthria, mixed dysarthria, scanning dysarthria, spastic dysarthria, dysglossia, dyslalia, dysosteosclerosis, dysphoric mood, dysthymic disorder, dystonia, dystonia 18 (disorder), diurnal dystonia, limb dystonia, paroxysmal dystonia, ear diseases, early infantile epileptic encephalopathy with suppression bursts, early myoclonic encephalopathy, infantile eczema, edema, Ehlers-Danlos syndrome, type iv Ehlers-Danlos syndrome, elliptocytosis 4, hereditary elliptocytosis, embryo disintegration, embryo loss, embryonal neoplasm, embryonal rhabdomyosarcoma, encephaloclastic proliferative vasculopathy, encephalopathies, endogenous depression, endometrioma, endometriosis, enterovirus infections, epilepsy, anterior fronto-polar epilepsy, benign psychomotor childhood epilepsy, childhood absence epilepsy, cingulate epilepsy, cryptogenic epilepsy, frontal lobe epilepsy, susceptibility to idiopathic generalized epilepsy 12, susceptibility to idiopathic generalized epilepsy 14, lateral temporal epilepsy, opercular epilepsy, orbito-frontal epilepsy, pyridoxine-dependent epilepsy, supplementary motor epilepsy, temporal lobe epilepsy, epileptic drop attack, epileptic encephalopathy, early infantile epileptic encephalopathy 25, early infantile epileptic encephalopathy 34, early infantile epileptic encephalopathy 41, epileptic seizures, multiple epiphyseal dysplasia 4, type 6 (disorder) episodic ataxia, erythrocyte lactate transporter defect, erythrocytosis, esophageal neoplasms, etat marbre, riboflavin-responsive exercise intolerance, experimental hepatoma, external ophthalmoplegia, extrahepatic cholangiocarcinoma, extrapyramidal disorders, extravascular hemolysis, fahr's syndrome (disorder), familial Alzheimer disease (fad), familial apoceruloplasmin deficiency, familial dementia, familial juvenile parkinsonism, familial ménière disease, familial renal glucosuria, fanconi syndrome, fanconi-bickel syndrome, fatty liver, fazio-londe syndrome, fibrosis, liver fibrosis, flaccid muscle tone, floppy muscles, hereditary folate malabsorption, follicular adenoma, food allergy, foveal hypoplasia 2, foveal hypoplasia and anterior segment dysgenesis, fowler syndrome, fracture, spiral fracture, benign childhood frontal epilepsy, fuchs endothelial dystrophy, gastric cancer, gastrointestinal dysfunction, gastrointestinal neoplasms, gastrointestinal stromal sarcoma, gastrointestinal stromal tumors, generalized absence seizures, generalized pustular psoriasis, generalized seizures, germ cell cancer, germ cell tumor, gianotti-crosti syndrome, gitelman syndrome, glaucoma, glioma, glomerular filtration rate, glomerulosclerosis, glucose-6-phosphate transport defect, glut1 deficiency syndrome, autosomal recessive glut1 deficiency syndrome 1, glycine encephalopathy with normal serum glycine, glycogen storage disease ic, glycogen storage disease type id, renal glycosuria, goiter, gorlin chaudhry moss syndrome, gout, gout susceptibility 2, grand mal status epilepticus, gustatory seizure, harlequin fetus, hartnup disease, head and neck squamous cell carcinoma, hearing impairment, extreme hearing loss, heart decompensation, heart diseases, heart failure, right-sided heart failure, hematological disease, hemochromatosis, type 4 hemochromatosis, hemolysis (disorder), nonalcoholic fatty liver disease hepatic steatosis, hepatitis b, drug-induced hepatitis, toxic hepatitis, morris hepatoma, novikoff hepatoma, hepatomegaly, hereditary clear cell renal cell carcinoma, hereditary clubbing, hereditary diffuse gastric cancer, hereditary hemochromatosis, hereditary hyperexplexia, hereditary motor and sensory neuropathy type i, hereditary motor and sensory neuropathy with optic atrophy (disorder), hereditary motor and sensory-neuropathy type ii, hereditary spherocytosis, herpes encephalitis, herpetic acute necrotizing encephalitis, herpetic meningoencephalitis, hhh syndrome, histiocytosis, histiocytosis with joint contractures and sensorineural deafness, hiv coinfection, hiv infections, Huntington disease, late onset Huntington disease, hydrocephalus, hydrops fetalis, hyperactive behavior, hyperalgesia, primary hyperalgesia, secondary hyperalgesia, thermal hyperalgesia, hyperammonaemia, hyperammonemia, hypercalcemia, idiopathic hypercalcemia of infancy, infantile hypercalcemia, infantile hypercalcemia 1, infantile hypercalcemia 2, hypercalciuria, familial hypercholesterolemia, hyperekplexia 3, hyperglycinuria (disorder), hyperhomocysteinemia, familial hyperinsulinemic hypoglycemia 7, hyperinsulinism due to uncoupling protein 2 deficiency, generalized hyperkinesia, hypermanganesemia with dystonia 2, hypermanganesemia with dystonia polycythemia and cirrhosis, hyperoxaluria, hyperphosphatemia, hyperpigmentation, hypertensive disease, hypertrichosis, hypertrophic cardiomyopathy, primary autosomal dominant hypertrophic osteoarthropathy, primary autosomal recessive hypertrophic osteoarthropathy 2, hyperuricemia, hypogonadism, isolated hypogonadotropic hypogonadism, hypogonadotropic hypogonadism, global cerebral hypomyelination, hypophosphatemia, hereditary hypophosphatemic rickets with hypercalciuria, hypotension, hypothyroidism, hypotonia-cystinuria syndrome, renal hypouricemia 2, ichthyosis congenita ii, ichthyosis prematurity syndrome, congenital autosomal recessive ichthyosis 6, idiopathic autoimmune hemolytic anemia, idiopathic generalized epilepsy, idiopathic hypogonadotropic hypogonadism, iga glomerulonephritis, iieocolitis, iminoglycinuria, immersion related epilepsy, impaired glucose tolerance, impulse-ridden personality, inadequate personality, inborn errors of metabolism, infantile free sialic acid storage disease, infantile neuronal ceroid lipofuscinosis, infantile nystagmus, infantile sialic acid storage disease, inflammation, inflammatory bowel disease, inflammatory bowel diseases, inflammatory responses in cerebral ischemia and traumatic brain injury, influenza, acute brain injuries, insulin resistance, susceptibility to insulin resistance, insulin sensitivity, intellectual developmental disorder with neuropsychiatric features, intellectual disability, internal ophthalmoplegia, intestinal obstruction, intracranial hemorrhages, intracranial hypertension, intrahepatic cholangiocarcinoma, intravascular hemolysis, involutional depression, involutional paraphrenia, iron deficiency anemia, iron overload, irritable heart, jacksonian seizure, juvenile arthritis, juvenile Huntington disease, juvenile neuronal ceroid lipofuscinosis, juvenile psoriatic arthritis, juvenile-onset still disease, ketotic hypoglycemia, kidney calculi, kidney diseases, acute kidney failure, kidney injury, kidney neoplasm, kohlschutter tonz syndrome, kyphosis deformity of spine, lactic acidosis induced pulmonary vein arrhythmogenesis, lactic acidosis induced sepsis, late-infantile neuronal ceroid lipfuscinosis, learning disabilities, learning disorders, learning disturbance, left-sided heart failure, visceral leishmaniasis, lens opacities, lenticulostriate disorders, leprosy, leukemia, acute lymphocytic leukemia l2, leukodystrophy, libman-sacks disease, lichtenstein-knorr syndrome, lipoidosis, liver cancer, liver carcinoma, liver cirrhosis, experimental liver cirrhosis, liver diseases, liver dysfunction, liver neoplasms, experimental liver neoplasms, long sleeper syndrome, low tension glaucoma, lung cancer, lung neoplasms, systemic lupus erythematosus, lysinuric protein intolerance, macular dystrophy with central cone involvement, major depressive disorder, malabsorption syndrome, malaria, malformations of cortical development, malignant glioma, malignant mesothelioma, malignant neoplasm of breast, malignant neoplasm of esophagus, malignant neoplasm of kidney, malignant neoplasm of liver, malignant neoplasm of lung, malignant neoplasm of ovary, malignant neoplasm of pancreas, malignant neoplasm of prostate, malignant neoplasm of salivary gland, malignant neoplasm of stomach, malignant neoplasm of urinary bladder, malignant neoplasms, malignant tumor of colon, animal mammary carcinoma, mammary neoplasms, experimental mammary neoplasms, human mammary neoplasms, manganese poisoning, manic, manic disorder, marfan syndrome, marijuana abuse, mechanical allodynia, susceptibility to meconium ileus in cystic fibrosis, megacolon, melancholia, melanoma, semantic memory disorder, spatial memory disorder, memory disorders, memory impairment, memory loss, meningococcal meningitis of serogroup a, meningococcal meningitis of serogroup b, meningococcal meningitis of serogroup c, meningococcal meningitis of serogroup w-135, meningococcal meningitis of serogroup y, meningococcal meningitis, mental deficiency, mental depression, south african type mental retardation x-linked, autosomal recessive 48 mental retardation, autosomal recessive 7 mental retardation, psychosocial mental retardation, x-linked mental retardation, x-linked syndromic christianson type mental retardation, mesothelioma, metabolic acidosis, metabolic bone disorder, microangiopathic hemolytic anemia, microcalcification, microcephaly, primary autosomal recessive microcephaly 15, amish type (disorder) microcephaly, microlissencephaly, milk-alkali syndrome, minimal brain dysfunction, mitochondrial diseases, autosomal dominant mitochondrial dna depletion syndrome 12a (cardiomyopathic type) autosomal recessive mitochondrial dna depletion syndrome 12b (cardiomyopathic type), mitochondrial phosphate carrier deficiency, mitochondrial pyruvate carrier deficiency, mitral valve prolapse syndrome, mixed gliomas, monocarboxylate transporter 1 deficiency, mood disorders, motor tic disorders, movement disorders, multiple epiphyseal dysplasia, multiple sclerosis, acute fulminating multiple sclerosis, muscle hypotonia, muscle tone atonic, presynaptic congenital myasthenic syndrome 20, presynaptic congenital myasthenic syndrome 21, congenital myasthenic syndromes, congenital slow channel myasthenic syndromes, protection against mycobacterium tuberculosis, susceptibility to (finding) mycobacterium tuberculosis, myocardial failure, myocardial infraction, myocardial ischemia, myocardial reperfusion injury, myocarditis, myoclonic astatic epilepsy, myoclonic seizures, myoclonic-atonic epilepsy, myopathy, autosomal dominant myopia 24, na, n-acetylneuraminic acid storage disease, narcissistic personality disorder, necrosis, neonatal hypotonia, neoplasm invasiveness, neoplasm metastasis, neoplasms, embryonal and mixed neoplasms, germ cell and embryonal neoplasms, neoplastic cell transformation, nephrocalcinosis, calcium oxalate nephrolithiasis, hypophosphatemic nephrolithiasis-osteoporosis 1, nerve degeneration, nervous system disorder, neurocirculatory asthenia, neurodevelopmental disorders, neuronal ceroid-lipofuscinoses, neuropathic pain, distal hereditary motor type viia neuropathy, hereditary motor and sensory type vibneuropathy, nova scotian type niemann-pick disease, type c niemann-pick disease, type c1 niemann-pick disease, type d niemann-pick disease, congenital stationary night blindness, congenital stationary type 1 night blindness, congenital stationary type 1a night blindness, congenital stationary type 1b night blindness, congenital stationary type 2a night blindness, congenital stationary type 2b (disorder) night blindness, non-convulsive status epilepticus, nonepileptic convulsion, nonepileptic seizures, noninfiltrating intraductal carcinoma, nonorganic psychosis, non-small cell lung carcinoma, obesity, obsessive-compulsive disorder, oculocutaneous albinism type 2, oculocutaneous albinism type 6, type iv oculocutaneous albinism, odontome, olfactory seizure, ophthalmoparesis, ophthalmoplegia, optic atrophy, substance-induced organic mental disorders, orthostatic intolerance, primary hypertrophic osteoarthropathy, osteoarthrosis deformans, osteogenesis imperfecta, osteopenia, malaysian-melanesian-filipino type ovalocytosis, ovarian cancer, ovarian neoplasm, oxalosis, pain, burning pain, crushing pain, migratory pain, splitting pain, pancreatic cancer, pancreatic neoplasm, pancytopenia, papillary adenoma, papillary renal cell carcinoma, paranoia, parkinson disease, autosomal recessive juvenile parkinson disease 2, parkinsonian disorders, experimental parkinsonism, juvenile parkinsonism, infantile parkinsonism-dystonia, pediatric autoimmune disease, pendred's syndrome, peripheral diabetic neuropathy, peripheral neuropathy, peritoneal neoplasms, peritoneal surface malignancy, personality disorders, petit mal status, petty laxova wiedemann syndrome, physiological wound healing, pigmented basal cell carcinoma, pitt-rogers-danks syndrome, poisoning, polycystic kidney disease, polycythemia, disseminated superficial actinic type porokeratosis 8, disseminated superficial actinic porokeratosis, posterior column ataxia with retinitis pigmentosa, posterior fossa hemorrhage, post-traumatic tic disorder, precancerous conditions, precursor cell lymphoblastic leukemia lymphoma, prelingual deafness, prenatal injuries, prescription drug abuse, presenile dementia, primary biliary cirrhosis, primary hypogonadism, primary hypothyroidism, primary microcephaly, profound mental retardation, progressive bulbar palsy, autosomal dominant progressive external ophthalmoplegia with mitochondrial dna deletions 1, autosomal dominant progressive external ophthalmoplegia with mitochondrial dna deletions 2, prostate cancer, prostatic neoplasms, pseudoaphakia, pseudohyperkalemia cardiff, drug psychoses, substance-induced psychoses, involutional psychosis, psychotic disorders, pulmonary alveolar microlithiasis, pulmonary hypertension, radiating pain, ramsay hunt paralysis syndrome, recurrent depression, regional enteritis, renal carnitine transport defect, renal cell carcinoma, renal hypouricemia, renal tubular acidosis, distal renal tubular acidosis with hemolytic anemia (disorder), distal renal tubular acidosis with normal red cell morphology, proximal renal tubular acidosis with ocular abnormalities and mental retardation, type ii renal tubular acidosis, reperfusion injury, respiratory depression, adult respiratory distress syndrome, respiratory failure, respiratory hypersensitivity, respiratory insufficiency, retinal degeneration, retinitis pigmentosa, retinitis pigmentosa 68, rh deficiency syndrome, rhabdomyolysis, rhabdomyosarcoma, rhabdomyosarcoma 1, rheumatoid arthritis, rhinolalia, regulator type rh-null, riboflavin deficiency, rotor syndrome, roussy-levy syndrome (disorder), salivary gland neoplasms, sarcoidosis, sarcomatoid renal cell carcinoma, schizophrenia, catatonic schizophrenia, schneckenbecken dysplasia, schwartz-lelek syndrome, seasonal affective disorder, secondary hypothyroidism, seizures, auditory seizures, clonic seizures, focal seizures, sensory seizures, somatosensory seizures, senile paranoid dementia, sensorineural hearing loss (disorder), sensory hearing loss, severe congenital microcephaly, severe depression, severe major depression with psychotic features, hemorrhagic shock, short sleeper syndrome, finnish type (disorder) sialic acid storage disease, sialuria, sideroblastic anemia, simple partial status epilepticus, single seizure, sinus histiocytosis, variation in skin-hair-eye pigmentation 4, sleep disorders, sleep quality, sleep wake disorders, sleep-related neurogenic tachypnea, southeast asian ovalocytosis, autosomal dominant spastic paraplegia 42, autosomal dominant (disorder) spastic paraplegia 6, hereditary spastic paraplegia, thin corpus callosum spastic tetraplegia and progressive microcephaly, speech disorders, type 4 spherocytosis, autosomal recessive 3 spinocerebellar ataxia, Ehlers-Danlos syndrome-like spondylocheirodysplasia, squamous cell carcinoma, squamous cell carcinoma of esophagus, status epilepticus, alcohol withdrawal-induced status epilepticus, subclinical status epilepticus, steatohepatitis, stomach carcinoma, stomach neoplasms, stomatocytosis i, streptozotocin diabetes, stroke, substance abuse problem, substance dependence, substance use disorders, substance withdrawal syndrome, substance-related disorders, subwakefullness syndrome, sudden infant death syndrome, physical suffering, tactile allodynia, testicular microlithiasis, thiamine metabolism dysfunction syndrome 4 (bilateral striatal degeneration and progressive polyneuropathy type), thiamine responsive megaloblastic anemia syndrome, thinness, thyroid agenesis, thyroid carcinoma, thyroid dyshormonogenesis 1, thyroid gland follicular adenoma, thyroid hormone resistance syndrome, thyroid hypoplasia, thyroid neoplasm, tic disorder, vocal tic disorders, tissue fibrosis, susceptibility to (finding) tobacco addiction, tonic - clonic seizures, tonic seizures, tooth abnormalities, transient tic disorder, rubral tremor, meiotic nondisjunction trisomy 21, mitotic nondisjunction trisomy 21, tuberculosis, tumoral calcinosis, ulcerative colitis, uncinate epilepsy, undifferentiated carcinoma, unilateral hypotonia, unipolar depression, serum quantitative trait locus uric acid concentration 2, serum quantitative trait locus uric acid concentration 4, urolithiasis, vascular diseases, vascular hypertrophy, verbal fluency disorders, vertiginous seizure, visual seizure, withdrawal symptoms, wolf-hirschhorn syndrome, xerocytosis, x-linked csnb, epstein-barr virus infection and neoplasia x-linked immunodeficiency with magnesium defect and neonatal zinc deficiency due to low breast milk zinc.

## Patentansprüche

1. Verbindung, die die folgende Formel (I) aufweist: 
Transmembran-Protein-Bindungs-
Gruppe
(Transmembrane Protein Binding
Moiety; TMPBM)
Linker (L)
E3-Ligase-Bindungs-Gruppe
(E3 Ligase Binding Moiety;
EBM)
,
 wobei
TMPBM eine Gruppe ist, die an ein Transmembranprotein bindet,
L eine Linkergruppe ist; und
EBM eine Gruppe ist, die die Funktion der E3-Ligase modifiziert und/oder an mindestens ein Mitglied des E3-Ligasekomplexes bindet,
wobei TMPBM eine Gruppe mit der folgenden Teilformel (II) ist oder ein Stereoisomer, Tautomer, ein pharmazeutisch verträgliches Salz oder Solvat: 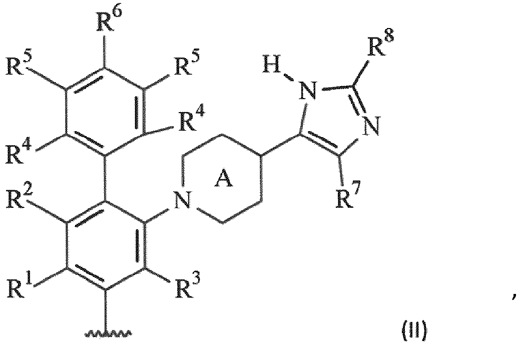 wobei
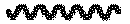 die Position der Anheftung der Teilformel (II) an den Linker (L) angibt,
R¹, R², R³, R⁴, R⁵, R⁶, R⁷ and R⁸ jeweils unabhängig voneinander ausgewählt sind aus -H, -Halogen, -NO₂, -CN, -C₁₋₃-Alkyl, -OH, -O-C₁₋₂-Alkyl, NH₂, -NH(C₁₋₂-Alkyl), -N(C₁₋₂-Alkyl)₂, -CHO, -CO(C₁₋₂-Alkyl), COOH, COO(C₁₋₂-Alkyl), CONH₂, CONH(C₁₋₂-Alkyl), und CON(-C₁₋₂-Alkyl)₂, wobei jedes C₁₋₂-Alkyl gegebenenfalls mit einem oder mehreren F substituiert ist,
und Ring A gegebenenfalls mit einem oder mehreren F substituiert ist.

2. Verbindung nach Anspruch 1, wobei in der Gruppe der Teilformel (II) die folgenden Definitionen gelten:
R¹, R², R³ und jedes R⁴ sind jeweils Wasserstoff,
R⁷ ist Methyl,
R⁸ ist H oder NH₂,
und Ring A ist nicht mit einem F substituiert.

3. Verbindung nach Anspruch 1 oder 2, wobei in der Gruppe der Teilformel (II) die folgenden Definitionen gelten:
ein R⁵ ist ausgewählt aus H, F, Cl, Me, CF₃, CF₂H und CH₂F,
das andere R⁵ ist H, und
R⁶ ist F oder Cl.

4. Verbindung nach einem der Ansprüche 1 bis 3, wobei die Gruppe der Teilformel (II) die folgende Formel (IIa) aufweist:

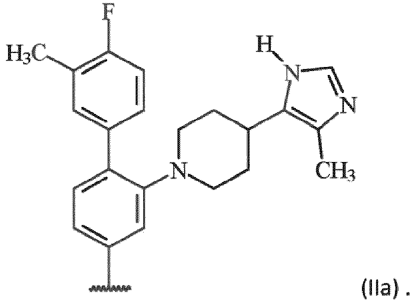

5. Verbindung nach einem der Ansprüche 1 bis 4, wobei L ausgewählt ist aus C₁₄₋₂₄-Alkylen, C₁₄₋₂₄-Alkenylen und C₁₄₋₂₄-Alkinylen, wobei das Alkylen, das Alkenylen und Alkinylen jeweils gegebenenfalls mit einer oder mehreren Gruppen substituiert ist, die unabhängig ausgewählt ist aus Halogen, C₁₋₅-Haloalkyl, -O(C₁₋₅-Haloalkyl), -CN, -OR²¹, -NR²¹R²¹, -NR²¹OR²¹, -COR²¹, -COOR²¹, -OCOR²¹, -CONR²¹R²¹, -NR²¹COR²¹, -NR²¹COOR²¹, -OCONR²¹R²¹, -SR²¹, -SOR²¹, -SO₂R²¹, -SO₂NR²¹R²¹, -NR²¹SO₂R²¹, -SO₃R²¹, und -NO₂, und des Weiteren wobei eine oder mehrere -CH₂-Einheiten, die in dem Alkylen, dem Alkenylen oder dem Alkinylen umfasst sind, jeweils gegebenenfalls ersetzt sind durch eine Gruppe, die unabhängig ausgewählt ist aus -O-, -NR²¹-, -CO-, -S-, -SO- und -SO₂-;
jedes R²¹ unabhängig ausgewählt ist aus Wasserstoff, C₁₋₅-Alkyl, C₂₋₅-Alkenyl, C₂₋₅-Alkinyl, Carbocyclyl und Heterocyclyl, wobei das Alkyl, das Alkenyl und das Alkinyl jeweils gegebenenfalls mit einen oder mehreren Gruppen R^{Alk} substituiert sind, und des Weiteren wobei das Carbocyclyl und Heterocyclyl jeweils gegebenenfalls mit einer oder mehreren Gruppen R^{Cyc} substituiert sind;
beliebige zwei R²¹ gegebenenfalls verbunden sind, um einen Ring zu bilden;
jedes R^{Alk} unabhängig ausgewählt ist aus -OH, -O(C₁₋₅-Alkyl), -O(C₁₋₅-Alkylen)-OH, -O(C₁₋₅-Alkylen)-O(C₁₋₅-Alkyl), -SH, -S(C₁₋₅-Alkyl), -S(C₁₋₅-Alkylen)-SH, -S(C₁₋₅-Alkylen)-S(C₁₋₅-Alkyl), -NH₂ , -NH(C₁₋₅-Alkyl), -N(C₁₋₅-Alkyl)(C₁₋₅-Alkyl), -NH-OH, -N(C₁₋₅-Alkyl)-OH, -NH-O(C₁₋₅-Alkyl), -N(C₁₋₅-Alkyl)-O(C₁₋₅-Alkyl), Halogen, C₁₋5-Haloalkyl, -O(C₁₋₅-Haloalkyl), -CN, -NO₂ , -CHO, -CO(C₁₋₅-Alkyl), -COOH, -COO(C₁₋₅-Alkyl), -O-CO(C₁₋₅-Alkyl), -CO-NH₂, -CO-NH(C₁₋₅-Alkyl), -CO-N(C₁₋₅-Alkyl)(C₁₋₅-Alkyl), -NH-CO(C₁₋₅-Alkyl), -N(C₁₋₅-Alkyl)-CO(C₁₋₅-Alkyl), -NH-COO(C₁₋₅-Alkyl), -N(C₁₋₅-Alkyl)-COO(C₁₋₅-Alkyl), -O-CO-NH(C₁₋₅-Alkyl), -O-CO-N(C₁₋₅-Alkyl)(C₁₋₅-Alkyl), -SO₂-NH₂ , -SO₂-NH(C₁₋₅-Alkyl), -SO₂-N(C₁₋₅-Alkyl)(C₁₋₅-Alkyl), -NH-SO₂-(C₁₋₅-Alkyl), -N(C₁₋₅-Alkyl)-SO₂-(C₁₋₅-Alkyl), -SO₂-(C₁₋₅-Alkyl), -SO-(C₁₋₅-Alkyl), Aryl, Heteroaryl, Cycloalkyl, und Heterocycloalkyl, wobei das Aryl, das Heteroaryl, das Cycloalkyl und das Heterocycloalkyl jeweils gegebenenfalls substituiert sind mit einer oder mehreren Gruppen, die unabhängig ausgewählt sind aus C₁₋₅-Alkyl, C₂₋₅-Alkenyl, C₂₋₅-Alkinyl, Halogen, C₁₋₅-Haloalkyl, -CN, -OH, -O(C₁₋₅-Alkyl), -SH, -S(C₁₋₅-Alkyl), -NH₂, -NH(C₁₋₅-Alkyl) und -N(C₁₋₅-Alkyl)(C₁₋₅-Alkyl);
jedes R^{Cyc} unabhängig ausgewählt ist aus C₁₋₅-Alkyl, C₂₋₅-Alkenyl, C₂₋₅-Alkinyl, -OH, -O(C₁₋₅-Alkyl), -O(C₁₋₅-Alkylen)-OH, -O(C₁₋₅-Alkylen)-O(C₁₋₅-Alkyl), -SH, -S(C₁₋₅-Alkyl), -S(C₁₋₅-Alkylen)-SH, -S(C₁₋₅-Alkylen)-S(C₁₋₅-Alkyl), -NH₂, -NH(C₁₋₅-Alkyl), -N(C₁₋₅-Alkyl)(C₁₋₅-Alkyl), -NH-OH, -N(C₁₋₅-Alkyl)-OH, -NH-O(C₁₋₅-Alkyl), -N(C₁₋₅-Alkyl)-O(C₁₋₅-Alkyl), Halogen, C₁₋₅Haloalkyl, -O(C₁₋₅-Haloalkyl), -CN, -NO₂ , -CHO, -CO(C₁₋₅-Alkyl), -COOH, -COO(C₁₋₅-Alkyl), -O-CO(C₁₋₅-Alkyl), -CO-NH₂, -CO-NH(C₁₋₅-Alkyl), -CO-N(C₁₋₅-Alkyl)(C₁₋₅-Alkyl), -NH-CO(C₁₋₅-Alkyl), -N(C₁₋₅-Alkyl)-CO(C₁₋₅-Alkyl), -NH-COO(C₁₋₅-Alkyl), -N(C₁₋₅-Alkyl)-COO(C₁₋₅-Alkyl), -O-CO-NH(C₁₋₅-Alkyl), -O-CO-N(C₁₋₅-Alkyl)(C₁₋₅-Alkyl), -SO₂-NH₂, -SO₂-NH(C₁₋₅-Alkyl), -SO₂-N(C₁₋₅-Alkyl)(C₁₋₅-Alkyl), -NH-SO₂-(C₁₋₅-Alkyl), -N(C₁₋₅-Alkyl)-SO₂-(C₁₋₅-Alkyl), -SO₂-(C₁₋₅-Alkyl), -SO-(C₁₋₅-Alkyl), Aryl, Heteroaryl, Cycloalkyl, und Heterocycloalkyl, wobei das Aryl, das Heteroaryl, das Cycloalkyl und das Heterocycloalkyl jeweils gegebenenfalls substituiert sind mit einer oder mehreren Gruppen, die ausgewählt sind aus C₁₋₅-Alkyl, C₂₋₅-Alkenyl, C₂₋₅-Alkinyl, Halogen, C₁₋₅-Haloalkyl, -CN, -OH, -O(C₁₋₅-Alkyl), -SH, -S(C₁₋₅-Alkyl), -NH₂, -NH(C₁₋₅-Alkyl) und -N(C₁₋₅-Alkyl)(C₁₋₅-Alkyl).

6. Verbindung nach Anspruch 5, wobei
(i) das C₁₄₋₂₄-Alkylen, C₁₄₋₂₄-Alkenylen und C₁₄₋₂₄-Alkinylen C₁₅₋₂₂-Alkylen, C₁₅₋₂₂-Alkenylen bzw. C₁₅₋₂₂-Alkinylen sind, bevorzugt C₁₆₋₂₀-Alkylen, C₁₆₋₂₀-Alkenylen bzw. C₁₆₋₂₀-Alkinylen, wobei jedes der Alkylene, Alkenylene und Alkinylene wie in Anspruch 5 angegeben modifiziert werden kann;
(ii) L ausgewählt ist aus C₁₄₋₂₄-Alkylen und C₁₄₋₂₄-Alkenylen, stärker bevorzugt C₁₅₋₂₂-Alkylen, C₁₅₋₂₂-Alkenylen, noch stärker bevorzugt C₁₆₋₂₀-Alkylen, noch stärker bevorzugt C₁₇₋₁₉-Alkylen, am stärksten bevorzugt C₁₇-Alkylen, wobei jedes der Alkylene und Alkenylene wie in Anspruch 5 angegeben modifiziert werden kann;
(iii) L eine der folgenden Strukturen aufweist:
-O-L¹-NH-C(=O)- and -NH-L¹-NH-C(=O)-,
wobei L¹ ausgewählt ist aus C₁₃₋₁₇-Alkylen und C₁₃₋₁₇-Alkenylen, wobei das Alkylen und das Alkenylen jeweils gegebenenfalls substituiert sind mit einer oder mehreren Gruppen, die unabhängig ausgewählt sind aus Halogen, C₁₋₃-Alkyl und C₁₋₃-Haloalkyl, und des Weiteren wobei eine oder mehrere -CH₂-Einheiten, die in dem Alkylen oder dem Alkenylen umfasst ist/sind, durch eine Gruppe ersetzt ist/sind, die unabhängig ausgewählt ist aus -O-, -NR²¹-, -CO-, -S-, -SO- und -SO₂-; wobei es bevorzugt ist, dass das -O- oder -NH- auf der linken Seite an EBM bindet, wie in einem der vorangehenden Punkte definiert ist, und das NH-C(=O)- auf der rechten Seite an die TMPBM gebunden ist, wie in einem der vorangehenden Punkte definiert ist; oder
(iv) L eine der folgenden Strukturen aufweist:
-O-L¹-NH-C(=O)- and -NH-L¹-NH-C(=O)-,
wobei L¹ ausgewählt ist aus C₁₃₋₁₇-Alkylen, wobei das Alkylen gegebenenfalls substituiert ist mit einer oder mehreren Gruppen, die unabhängig ausgewählt sind aus Halogen, C₁₋₃-Haloalkyl und C₁₋₃-Haloalkyl, und des Weiteren wobei eine oder mehrere -CH₂-Einheiten, die in dem Alkylen oder dem Alkenylen umfasst ist/sind, durch eine Gruppe ersetzt ist/sind, die unabhängig ausgewählt ist aus -O-, -NR²¹- und -CO-; wobei es bevorzugt ist, dass das -O- oder -NH- auf der linken Seite an EBM bindet, wie in einem der vorangehenden Punkte definiert ist, und das NH-C(=O)- auf der rechten Seite an die TMPBM gebunden ist, wie in einem der vorangehenden Punkte definiert ist.

7. Verbindung nach Formel (I) nach einem der vorangehenden Ansprüche, wobei EBM eine Gruppe der Teilformel (III) ist: 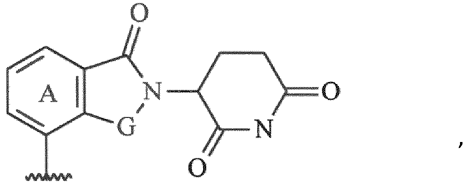 wobei
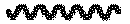 die Position der Anheftung der Teilformel (III) an den Linker (L) angibt,
G ausgewählt ist aus -C(=O)- und CH₂,
und Ring A gegebenenfalls mit einem oder mehreren F substituiert ist,
oder ein Stereoisomer davon.

8. Verbindung nach Anspruch 7, wobei in der Teilformel (III) G -C(=O)- ist und Ring A nicht mit einem F substituiert ist.

9. Zusammensetzung, die eine Verbindung nach einem der Ansprüche 1 bis 8 umfasst.

10. Verbindung nach einem der Ansprüche 1 bis 8 oder Zusammensetzung nach Anspruch 9, des Weiteren umfassend ein(en) pharmazeutisch verträgliches/verträglichen Verdünnungsmittel, Exzipienten oder Träger.

11. Verbindung nach einem der Ansprüche 1 bis 8 oder 10 oder Zusammensetzung nach Anspruch 9 oder 10 zur Verwendung als Medikament.

12. Verbindung nach einem der Ansprüche 1 bis 8, 10 oder 11, oder Zusammensetzung nach einem der Ansprüche 9 bis 11 zur Verwendung bei der Behandlung oder Vorbeugung von Krebs, insbesondere Leukämie wie chronischer myeloider Leukämie.

13. Verbindung nach einem der Ansprüche 1 bis 8, 10 oder 11, oder Zusammensetzung nach einem der Ansprüche 9 bis 11 zur Verwendung bei der Behandlung oder Vorbeugung einer Erkrankung, die ausgewählt ist aus: 2p21-Mikrodeletion-Syndrom, abnormer Koordination, Anomalie der Hornhaut, Absenceepilepsie, Schmerzen, Achondrogenesie Typ IB (Störung), Azidose, Acrodermatitis, Acrodermatitis enteropathica, akuten zerebrovaskulären Unfällen, akuter verwirrtheitsbedingter seniler Demenz, akutem Nierenversagen, akuter Niereninsuffizienz, akutem Lungenversagen, Adenokarzinom der Lunge (Störung), adenoid-zystischem Karzinom, Adenom, Basalzelladenom, mikrocystischem Adenom, monomorphem Adenom, trabekulärem Adenom, Fanconi-Syndrom bei Erwachsenen, Lernbehinderungen bei Erwachsenen, neuronaler Ceroid-Lipofuszinose bei Erwachsenen, Citrullinämie Typ 2 im Erwachsenenalter, Arzneimittelnebenwirkungen, psychotischen affektiven Störungen, altersbedingter Makuladegeneration, altersbedingten Gedächtnisstörungen, akinetisch-rigider Variante der Huntington-Krankheit, okulärem Albinismus, Alkoholmissbrauch, Alkoholempfindlichkeit, Alkoholkonsumstörung, Anfälle durch Alkoholentzug, durch Alkoholentzug induzierten schweren motorischen Anfall, Alkoholintoxikation, chronischer Alkoholintoxikation, Allan-Herndon-Dudley-Syndrom (AHDS), Allodynie, Alloxandiabetes, alternierender Hemiplegie im Kindesalter, früh einsetzender Alzheimer-Krankheit, spät einsetzender Alzheimer-Krankheit, Alzheimer-Krankheit, fokaler Alzheimer-Krankheit, Amelogenesis imperfecta, Amelogenesis imperfecta vom Hypomaturationstyp, Amelogenesis imperfecta Hypomaturation vom Typ IIa5, Amelogenesis imperfecta vom Typ III, angeborenen Störungen des Aminosäurestoffwechsels, vererbten Störungen des Aminosäurestoffwechsels, Amphetaminmissbrauch, Amphetaminabhängigkeit, Amphetamin-bedingten Störungen, Amyotropher Lateralsklerose, Amyotropher Lateralsklerose mit Demenz, Guam-Form der Amyotrophen Lateralsklerose, anaplastischem Karzinom, Anasarka, Anämie, hämolytischer Anämie, erworbener hämolytischer Anämie, idiopathischer erworbener hämolytischer Anämie, hereditärer sphärozytärer hämolytischer Anämie, hypochromer mikrozytärer Anämie mit Eisenüberladung, hypochromer mikrozytärer Anämie mit Eisenüberladung 1, mikroangiopathischer Anämie, Pyridoxin-refraktärer sideroblastischer Anämie 2, Pyridoxin-refraktärer autosomal-rezessiver sideroblastischer Anämie, Anhedonie, Mamma-Neoplasien bei Tieren, Angststörungen, Angstneurose (Befund), neurotischen Angstzuständen, thorakalem Aortenaneurysma, Aortenklappenverkalkung, Apnoe, Aprosodie, arterieller Tortuosität, Arterial-Tortuosity-Syndrom, Arthrogrypose, Arthrogrypose, geistiger Behinderung und Krampfanfällen, Alsob-Persönlichkeit, Asperger-Syndrom, Ataxie, appendikulärer Ataxie, motorischer Ataxie, sensorischer Ataxie, Rumpfataxie, hereditären Ataxien, Atelosteogenesis Typ 2, Atherogenese, Atherosklerose, atonischer Absence-Epilepsie, atrophischer, Aufmerksamkeitsdefizitstörung, Aufmerksamkeitsdefizit-/Hyperaktivitätsstörung, Aufmerksamkeitsdefizit-/Hyperaktivitätsstörung (ADHS), atypischer duktaler Brusthyperplasie, Aura, Autismus-Spektrum-Störungen, autistischer Störung, autoimmunhämolytischer Anämie, autosomal-dominanter juveniler Parkinson-Krankheit, autosomal-dominantem Parkinsonismus, autosomal-rezessivem Parkinsonismus, autosomal-rezessiver polyzystischer Nierenerkrankung, autosomal-rezessiver primärer Mikrozephalie, autosomal-rezessiver sideroblastischer Anämie, ängstlich-vermeidender Persönlichkeitsstörung, Aufwachepilepsie, nichtobstruktiver Azoospermie, Barrett-Epithel, Barrett-Ösophagus, Bartter-Syndrom, pränatalem Bartter-Syndrom Typ 1, Basalzellkarzinom, idiopathischer Basalganglienverkalkung 6, Biotinresponsiver Basalganglienerkrankung, Basalganglienerkrankungen, gutartiger Neoplasie, beidseitiger Taubheit, Gallensäure-CoA-Ligase-Mangel und Amidierungsdefekt, primärer Gallensäuremalabsorption, biliärer Zirrhose, sekundärer biliärer Zirrhose, bipolarer Störung, bipolarer Störung I, Birk-Landau-Perez-Syndrom, Blasen-Neoplasie, Blastozystenzerfall, Body-Mass-Index-Quantitative-Trait-Locus 11, Body-Mass-Index-Quantitative-Trait-Locus 4 (Störung), Knochenerkrankungen, entwicklungsbedingten Knochenerkrankungen, Hirnerkrankungen, vesikulärer Dopamin-Serotonin-Transportkrankheit im Gehirn, Gehirnblutung, Gehirnverletzungen, fokalen Gehirnverletzungen, Gehirnischämie, Hirnläsionen, Brustkrebs, familiärem Brustkrebs, Brustkarzinom, Brittle-Diabetes, bronchioloalveolärem Adenokarzinom, braunem okulokutanem Albinismus, Brown-Vialetto-van-Laere-Syndrom, Brown-Vialetto-van-Laere-Syndrom 2, Brucellose, Lungenbrucellose, Bulbärparalyse, verbrennungsbedingter Multiorganverletzung, Buruli-Ulkus, Kalzinose, Kalziumpyrophosphat-Arthropathie, Kalziumpyrophosphat-Ablagerungskrankheit, embryonalem Krebs, embryonalem und gemischtem Krebs, Cannabiskonsum, Kohlenhydratmangel-Glykoprotein-Syndrom Typ 2k, Karzinom, Lungenkarzinom, Spindelzellkarzinom, Karzinomatose, Herzrhythmusstörungen, Herzhypertrophie, kardialer ischämischer Reperfusion, Kardiomegalie, dilatativer Kardiomyopathie, familiärer idiopathischer Kardiomyopathie, kardiovaskulären Erkrankungen, Karditis, Carnitin-Acylcarnitin-Translokase-Mangel, Katarakt, Katarakt 47, Katarakt und Kardiomyopathie, juvenilem Katarakt mit Mikrokornea und Glucosurie, Zöliakie, zentraler Hypothyreose, Gehirnverletzung, zerebraler Ischämie, zerebrovaskulärem Unfall, zerebrovaskulären Erkrankungen, neuronaler Ceroid-Lipofuszinose 7, neuronaler Ceroid-Lipofuszinose vom Parry-Typ, Coeruloplasminmangel, Gebärmutterhalskrebs, Charcot-Marie-Tooth-Krankheit, Charcot-Marie-Tooth-Krankheit Typ IA (Störung), Charcot-Marie-Tooth-Krankheit Typ IB, durch Chemikalien und Medikamente verursachter Leberschädigung, chemisch induzierter Lebertoxizität, Entwicklungsstörungen im Kindesalter, spezifischen Entwicklungsstörungen im Kindesalter, akuter lymphatischer Leukämie im Kindesalter, progressiver Bulbärparalyse im Kindesalter, Tic-Störungen im Kindesalter, Cholangiokarzinom, Choledochuszyste, Choledochuszyste Typ I, Choledochuszyste Typ II, Choledochuszyste Typ III, Choledochuszyste Typ IV, Choledochuszyste Typ V, Cholestase, Cholestase bei Neugeborenen, episodischer Choreoathetose/Spastik, chromophobem Nierenzellkarzinom, chronischer Depression, chronischer motorischer oder vokaler Tic-Störung, Zirrhose, Citrullinämie Typ II im Neugeborenenalter, Gaumenspalte, isolierter Gaumenspalte, Oberlippenspalte, Poltern (Redeflussstörung), Kokainmissbrauch, Kokainabhängigkeit, kokaininduzierter affektiver Störung, kokainbedingten Störungen, Kognitionsstörungen, kognitiver Beeinträchtigung, Kältehämagglutininkrankheit, Ductus-Bellini-Karzinom der Niere, Kollodiumfötus, Dickdarmkrebs, Dickdarm-Neoplasien, kolorektalem Krebs, kolorektalem Karzinom, kolorektalen Neoplasien, kombinierter D-2- und L-2-Hydroxyglutarazidurie, kombinierter oxidativer Phosphorylierungsdefizienz 18, kombinierter oxidativer Phosphorylierungsdefizienz 28, vollständigem Hörverlust, komplexen partiellen Anfällen, komplexem partiellem Status epilepticus, präneoplastischer Erkrankung, Reizleitungsstörung des Herzens, angeborener nichtprogressiver Zapfen-Stäbchen-Synapsenstörung, angeborener Anomalie, angeborener Anämie, angeborenem Aneurysma der aufsteigenden Aorta, angeborenem Katarakt, Hörverlust und Neurodegeneration, angeborener Chlorid-Diarrhoe, angeborener Glykosylierungsstörung Typ 2c, angeborener Glykosylierungsstörung Typ Ilf, angeborener Glykosylierungsstörung Typ Ilm, angeborener Glykosylierungsstörung Typ Iln, angeborenen Glykosylierungsstörungen, angeborener Glucose-Galactose-Malabsorption, angeborenen Herzfehlern, angeborener Hypothyreose, angeborener Ichthyose, angeborenem postsynaptischem myasthenem Syndrom, angeborenem präsynaptischem myasthenischem Syndrom, angeborener Myopathie (Störung), angeborener nichtbullöser ichthyosiformer Erythrodermie, angeborener sekretorischer Diarrhö vom Natriumtyp (Störung), kongestiver Herzinsuffizienz, Bindegewebserkrankungen, Verstopfung, Reizdarmsyndrom mit vorherrschender Verstopfung, Kontaktdermatitis, Kontaktüberempfindlichkeit, konventionellem (klarzelligem) Nierenzellkarzinom, Krämpfen, Krampfanfällen, Hornhautdystrophie und perzeptiver Schwerhörigkeit, Fuchsendothelialer Hornhautdystrophie 4, Hornhautendotheldystrophie 2, Hornhauttrübung, Corpus-callosum-Agenesie-Neuronopathie, kortikaler Dysplasie, Coxsackievirus-Infektionen, kraniofazialen Anomalien, autosomal-dominanter kraniometaphysärer Dysplasie, Kranioosteoarthropathie, Kraniosynostose, X-chromosomalem Kreatinmangel, Morbus Crohn, Morbus Crohn des Dickdarms, Morbus Crohn des Ileums, Stomatin-defizienter Kryohydrocytose mit geistiger Behinderung, Anfällen, Katarakten und massiver Hepatosplenomegalie, Mukoviszidose, Mukoviszidose-Modifikator 1, Zystinurie, Zystinurie Typ A, Zystinurie Typ A-B, Zystinurie Typ B, Zytopenie, De-la-Chapelle-Dysplasie, De-Toni-Debre-Fanconi-Syndrom, Taubstummheit, Taubheit, erworbener Taubheit, autosomal-dominanter 25 Taubheit (Störung), autosomal-dominanter 72 Taubheit, autosomal-rezessiver 4 Taubheit mit vergrößertem Aquaeductus vestibularis, autosomal-rezessiver 61 Taubheit, Mangel an Glutamatdecarboxylase, degenerativer Polyarthritis, Delirium, Demenz, depressiver Verstimmung, Depression, bipolarer Depression, neurotischer Depression, postpartaler Depression, depressiver Störung, behandlungsresistenter depressiver Störung, depressiver Symptome, depressivem Syndrom, allergischer Kontaktdermatitis, atopischer Dermatitis, Entwicklungsstörungen in der Schul- und Berufsbildung, Entwicklungsstörungen, Diabetes mellitus, experimentellem Diabetes mellitus, insulinabhängigem Diabetes mellitus, Ketose-anfälligem Diabetes mellitus, nichtinsulinabhängigem Diabetes mellitus, Diabetes mellitus Typ 2, Autoimmundiabetes, diabetischen Kardiomyopathien, diabetischem Katarakt, Diarrhö, diastrophischer Dysplasie, Dicarbonsäuren-Aminoazidurie, Krankheitsverschlechterung, distaler renaler tubulärer Azidose, Down-Syndrom, Down Syndrom mit partieller Trisomie 21, Drogenmissbrauch, Drogenabhängigkeit, Drogengewöhnung, Drogentoxizität, Drogengebrauchsstörungen, Drogenentzugssymptomen, drogeninduzierter akuter Leberschädigung, drogeninduzierten depressiven Zuständen, drogeninduzierter Lebererkrankung, duktalem Karzinom, Dysarthrie, schlaffer Dysarthrie, gutturaler Dysarthrie, gemischter Dysarthrie, Scanning-Dysarthrie, spastischer Dysarthrie, Dysglossie, Dyslalie, Dysosteosklerose, dysphorischer Stimmung, Dysthymie, Dystonie, Dystonie 18 (Störung), diurnaler Dystonie, Gliedmaßendystonie, paroxysmaler Dystonie, Ohrenerkrankungen, frühinfantiler epileptischer Enzephalopathie mit Burst-Suppression, früher myoklonischer Enzephalopathie, infantilem Ekzem, Ödem, Ehlers-Danlos-Syndrom, Ehlers-Danlos-Syndrom Typ IV, Elliptozytose 4, hereditärer Elliptozytose, Embryozerfall, Embryoverlust, embryonaler Neoplasie, embryonalem Rhabdomyosarkom, enzephaloklastischer proliferativer Vaskulopathie, Enzephalopathien, endogener Depression, Endometriom, Endometriose, Enterovirusinfektionen, Epilepsie, anteriorer frontopolarer Epilepsie, benigner psychomotorischer Epilepsie im Kindesalter, Absenceepilepsie im Kindesalter, cingulärer Epilepsie, kryptogener Epilepsie, Frontallappenepilepsie, Anfälligkeit für idiopathische generalisierte Epilepsie 12, Anfälligkeit für idiopathische generalisierte Epilepsie 14, lateraler temporaler Epilepsie, operkulärer Epilepsie, orbitofrontaler Epilepsie, Pyridoxin-abhängiger Epilepsie, supplementär-motorischer Epilepsie, Temporallappenepilepsie, epileptischer Sturzattacke, epileptischer Enzephalopathie, frühinfantiler epileptischer Enzephalopathie 25, frühinfantiler epileptischer Enzephalopathie 34, frühinfantiler epileptischer Enzephalopathie 41, epileptischen Anfällen, multipler epiphysärer Dysplasie 4, episodischer Ataxie Typ 6 (Störung), Erythrocytenlaktattransporterdefekt, Erythrocytose, Ösophagus-Neoplasien, Etat marbré, Riboflavin-responsiver Belastungsintoleranz, experimentellem Hepatom, externer Ophthalmoplegie, extrahepatischem Cholangiokarzinom, extrapyramidalen Störungen, extravaskulärer Hämolyse, Fahr-Syndrom (Störung), familiärer Alzheimer-Krankheit (FAD), familiärem Apoceruloplasminmangel, familiärer Demenz, familiärem juvenilem Parkinsonismus, familiärer Menière-Krankheit, familiärer renaler Glukosurie, Fanconi-Syndrom, Fanconi-Bickel-Syndrom, Fettleber, Fazio-Londe-Syndrom, Fibrose, Leberfibrose, schlaffem Muskeltonus, schlaffen Muskeln, hereditärer Folat-Malabsorption, follikulärem Adenom, Nahrungsmittelallergie, Foveahypoplasie 2, Foveahypoplasie und Vorderabschnitts-Dysgenesie, Fowler-Syndrom, Fraktur, Spiralfraktur, benigner Frontalepilepsie im Kindesalter, Fuchs-Endotheldystrophie, Magenkrebs, gastrointestinalen Funktionsstörungen, gastrointestinalen Neoplasien, gastrointestinalem Stromasarkom, gastrointestinalen Stromatumoren, generalisierten Absencen, generalisierter Psoriasis pustulosa, generalisierten Anfällen, Keimzellkrebs, Keimzelltumor, Gianotti-Crosti-Syndrom, Gitelman-Syndrom, Glaukom, Gliom, glomerulärer Filtrationsrate, Glomerulosklerose, Glucose-6-phosphat-Transportdefekt, Glut1-Defizienz-Syndrom, autosomal-rezessivem Glut1-Definzienz-Syndrom 1, Glycinenzephalopathie mit normalem Serumglycin, Glykogenspeicherkrankheit Typ Ic, Glykogenspeicherkrankheit Typ Id, renaler Glykosurie, Struma, Gorlin-Chaudhry-Moss-Syndrom, Gicht, Gichtanfälligkeit 2, Grand-Mal-Status epilepticus, gustatorischem Anfall, Harlekin-Fötus, Hartnup-Krankheit, Kopf-Hals-Plattenepithelkarzinom, Hörbehinderung, extremem Hörverlust, Herzdekompensation, Herzerkrankungen, Herzinsuffizienz, Rechtsherzinsuffizienz, hämatologischer Erkrankung, Hämochromatose, Hämochromatose Typ 4, Hämolyse (Störung), nichtalkoholischer Fettlebererkrankung (Steatose), Hepatitis B, drogeninduzierter Hepatitis, toxischer Hepatitis, Morris-Hepatom, Novikoff-Hepatom, Hepatomegalie, hereditärem klarzelligem Nierenzellkarzinom, hereditärem Trommelschlägelfinger, hereditärem diffusem Magenkrebs, hereditärer Hämochromatose, hereditärer Hyperekplexie, hereditärer motorischer und sensorischer Neuropathie Typ I, hereditärer motorischer und sensorischer Neuropathie mit Optikusatrophie (Störung), hereditärer motorischer und sensorischer Neuropathie Typ II, hereditärer Sphärocytose, Herpesenzephalitis, akuter nekrotisierender Herpesenzephalitis, Herpes-Meningoenzephalitis, HHH-Syndrom, Histiocytose, Histiocytose mit Gelenkkontrakturen und sensorineuraler Taubheit, HIV-Co-Infektion, HIV-Infektionen, Huntington-Krankheit, spät einsetzender Huntington-Krankheit, Hydrocephalus, Hydrops fetalis, hyperaktivem Verhalten, Hyperalgesie, primärer Hyperalgesie, sekundärer Hyperalgesie, thermischer Hyperalgesie, Hyperammonämie, Hyperammonämie, Hyperkalzämie, idiopathischer Hyperkalzämie im Kindesalter, infantiler Hyperkalzämie, infantiler Hyperkalzämie 1, infantiler Hyperkalzämie 2, Hyperkalziurie, familiärer Hypercholesterinämie, Hyperekplexie 3, Hyperglycinurie (Störung), Hyperhomocysteinämie, familiärer hyperinsulinämischer Hypoglykämie 7, Hyperinsulinismus durch Entkopplungsprotein-2-Defizienz, generalisierter Hyperkinesie, Hypermanganämie mit Dystonie 2, Hypermanganämie mit Dystonie, Polycythämie und Zirrhose, Hyperoxalurie, Hyperphosphatämie, Hyperpigmentierung, hypertensiver Erkrankung, Hypertrichose, hypertropher Kardiomyopathie, primärer autosomal-dominanter hypertropher Osteoarthropathie, primärer autosomal-rezessiver hypertropher Osteoarthropathie 2, Hyperurikämie, Hypogonadismus, isoliertem hypogonadotropem Hypogonadismus, hypogonadotropem Hypogonadismus, globaler zerebraler Hypomyelinisierung, Hypophosphatämie, hereditärer hypophosphatämischer Rachitis mit Hyperkalziurie, Hypotonie, Hypothyreose, Hypotonie-Cystinurie-Syndrom, renaler Hypourikämie 2, Ichthyosis congenita II, Ichthyose-Prämaturitäts-Syndrom, kongenitaler autosomal-rezessiver Ichthyose 6, idiopathischer autoimmunhämolytischer Anämie, idiopathischer generalisierter Epilepsie, idiopathischem hypogonadotropem Hypogonadismus, IgA-Glomerulonephritis, Ileokolitis, Iminoglycinurie, Immersionsepilepsie, gestörter Glukosetoleranz, impulsiver Persönlichkeit, inadäquater Persönlichkeit, angeborenen Stoffwechselstörungen, infantiler freier Sialinsäure-Speicherkrankheit, infantiler neuronaler Ceroid-Lipofuszinose, infantilem Nystagmus, infantiler Sialinsäurespeicherkrankheit, Entzündung, entzündlicher Darmerkrankung, entzündlichen Darmerkrankungen, Entzündungsreaktionen bei zerebraler Ischämie und Schädel-Hirn-Trauma, Grippe, akuten Traumata des Gehirns, Insulinresistenz, Anfälligkeit für Insulinresistenz, Insulinsensitivität, geistiger Entwicklungsstörung mit neuropsychiatrischen Merkmalen, geistiger Behinderung, innerer Ophthalmoplegie, Darmverschluss, intrakraniellen Blutungen, intrakranieller Hypertension, intrahepatischem Cholangiokarzinom, intravaskulärer Hämolyse, Involutionsdepression, Involutionsparaphrenie, Eisenmangelanämie, Eisenüberladung, irritablem Herz, Jackson-Anfall, juveniler Arthritis, juveniler Huntington-Krankheit, juveniler neuronaler Ceroid-Lipofuszinose, juveniler Psoriasis-Arthritis, juvenilem Still-Syndrom, ketotischer Hypoglykämie, Nierensteinen, Nierenerkrankungen, akutem Nierenversagen, Nierenverletzung, Nieren-Neoplasie, Kohlschütter-Tönz-Syndrom, Kyphose-Deformität der Wirbelsäule, durch Laktatazidose induzierter Pulmonalvenen-Arrhythmogenese, durch Laktatazidose induzierter Sepsis, spätinfantiler neuronaler Ceroid-Lipofuszinose, Lernbeeinträchtigungen, Lernstörungen, Lernschwächen, Linksherzinsuffizienz, viszeraler Leishmaniose, Linsentrübungen, lentikulostriatalen Erkrankungen, Lepra, Leukämie, akuter lymphatischer Leukämie L2, Leukodystrophie, Libman-Sacks-Krankheit, Lichtenstein-Knorr-Syndrom, Lipoidose, Leberkrebs, Leberkarzinom, Leberzirrhose, experimenteller Leberzirrhose, Lebererkrankungen, Leberfunktionsstörungen, Leber-Neoplasien, experimentellen Leber-Neoplasien, Langschläfer-Syndrom, Niederdruckglaukom, Lungenkrebs, Lungen-Neoplasien, systemischem Lupus erythematodes, lysinurischer Proteinintoleranz, Makuladystrophie mit Beteiligung zentraler Zapfen, schwerer depressiver Störung, Malabsorptionssyndrom, Malaria, Fehlbildungen der kortikalen Entwicklung, bösartigem Gliom, bösartigem Mesotheliom, bösartiger Neoplasie der Brust, bösartiger Neoplasie der Speiseröhre, bösartiger Neoplasie der Niere, bösartiger Neoplasie der Leber, bösartiger Neoplasie der Lunge, bösartiger Neoplasie des Eierstocks, bösartiger Neoplasie der Bauchspeicheldrüse, bösartiger Neoplasie der Prostata, bösartiger Neoplasie der Speicheldrüsen, bösartiger Neoplasie des Magens, bösartiger Neoplasie der Harnblase, bösartigen Neoplasien, bösartigem Tumor des Dickdarms, Mammakarzinom bei Tieren, Mamma-Neoplasien, experimentellen Mamma-Neoplasien, Mamma-Neoplasien bei Menschen, Manganvergiftung, Manie, manischer Störung, Marfan-Syndrom, Marihuanamissbrauch, mechanischer Allodynie, Anfälligkeit für Mekoniumileus bei Mukoviszidose, Megakolon, Melancholie, Melanom, semantischer Gedächtnisstörung, räumlicher Gedächtnisstörung, Gedächtnisstörungen, Gedächtnisbeeinträchtigungen, Gedächtnisverlust, Meningokokken-Meningitis der Serogruppe A, Meningokokken-Meningitis der Serogruppe B, Meningokokken-Meningitis der Serogruppe C, Meningokokken-Meningitis Serogruppe W-135, Meningokokken-Meningitis der Serogruppe Y, Meningokokken-Meningitis, geistiger Behinderung, geistiger Depression, südafrikanischem Typ der X-chromosomalen geistigen Behinderung, autosomal-rezessiver geistiger Behinderung 48, autosomal-rezessiver geistiger Behinderung Typ 7, psychosozialer geistiger Behinderung, X-chromosomaler geistiger Behinderung, X-chromosomaler syndromaler geistiger Behinderung vom Christianson-Typ, Mesotheliom, metabolischer Azidose, metabolischer Knochenstörung, mikroangiopathischer hämolytischer Anämie, Mikrokalzifikation, Mikrozephalie, primärer autosomal-rezessiver Mikrozephalie 15, Mikrozephalie vom Amish-Typ (Störung), Mikrolissenzephalie, Milch-Alkali-Syndrom, minimaler Hirnfunktionsstörung, mitochondrialen Erkrankungen, autosomal-dominantem mitochondrialem DNA-Depletionssyndrom 12a (kardiomyopathischer Typ), autosomal-rezessivem mitochondrialem DNA-Depletionssyndrom 12b (kardiomyopathischer Typ), Mangel an mitochondrialem Phosphatträger, Mangel an mitochondrialem Pyruvatträger, Mitralklappenprolaps-Syndrom, gemischten Gliomen, Monocarboxylat-Transporter-1-Mangel, Stimmungsstörungen, motorischen Tic-Störungen, Bewegungsstörungen, multipler epiphysärer Dysplasie, multipler Sklerose, akuter fulminant verlaufender multipler Sklerose, Muskelhypotonie, atonischem Muskeltonus, präsynaptischem kongenitalem myasthenem Syndrom 20, präsynaptischem kongenitalem myasthenischem Syndrom 21, kongenitalen myasthenischen Syndromen, kongenitalen langsamen Kanalmyasthenie-Syndromen, Schutz vor Mycobacterium tuberculosis, Anfälligkeit für Mycobacterium tuberculosis (Befund), Myokardversagen, Myokardinfarkt, Myokardischämie, Myokard-Reperfusionsschaden, Myokarditis, myoklonisch-astatischer Epilepsie, myoklonischen Anfällen, myoklonisch-atonischer Epilepsie, Myopathie, autosomal-dominanter Myopie 24, na, N-Acetylneuraminsäure-Speicherkrankheit, narzisstischer Persönlichkeitsstörung, Nekrose, neonataler Hypotonie, Neoplasie-Invasivität, Neoplasie-Metastasierung, Neoplasien, embryonalen und gemischten Neoplasien, Keimzell- und embryonalen Neoplasien, neoplastischer Zelltransformation, Nephrokalzinose, Calciumoxalat-Nephrolithiasis, hypophosphatämischer Nephrolithiasis-Osteoporose 1, Nervendegeneration, Störung des Nervensystems, neurozirkulatorischer Asthenie, neurologischen Entwicklungsstörungen, neuronalen Ceroid-Lipofuszinosen, neuropathischen Schmerzen, distaler hereditärer motorischer Neuropathie Typ Vlla, Neuropathie hereditärer motorischer und sensorischer Typ VIb, Niemann-Pick-Krankheit Typ Nova Scotia, Niemann-Pick-Krankheit Typ C, Niemann-Pick-Krankheit Typ C1, Niemann-Pick-Krankheit Typ D, angeborener stationärer Nachtblindheit, angeborener stationärer Nachtblindheit Typ 1, angeborener stationärer Nachtblindheit Typ 1a, angeborener stationärer Nachtblindheit Typ 1b, angeborener stationärer Nachtblindheit Typ 2a, angeborener stationärer Nachtblindheit Typ 2b (Störung), nicht-konvulsivem Status epilepticus, nicht-epileptischen Krämpfen, nicht-epileptischen Anfällen, nicht-infiltrierendem intraduktalem Karzinom, nicht-organischer Psychose, nicht-kleinzelligem Lungenkarzinom, Fettleibigkeit, Zwangsstörung, okulokutanem Albinismus Typ 2, okulokutanem Albinismus Typ 6, okulokutanem Albinismus Typ IV, Odontom, olfaktorischem Krampfanfall, Ophthalmoparese, Ophthalmoplegie, Optikusatrophie, substanzinduzierten organischen psychischen Störungen, orthostatischer Intoleranz, primärer hypertropher Osteoarthropathie, Osteoarthrose deformans, Osteogenesis imperfecta, Osteopenie, Ovalozytose vom malaysisch-melanesisch-philippinischen Typ, Eierstockkrebs, Eierstock-Neoplasie, Oxalose, Schmerzen, brennendem Schmerz, drückendem Schmerz, wanderndem Schmerz, spaltendem Schmerz, Bauchspeicheldrüsenkrebs, Bauchspeicheldrüsen-Neoplasie, Panzytopenie, papillärem Adenom, papillärem Nierenzellkarzinom, Paranoia, Parkinson-Krankheit, autosomal-rezessiver juveniler Parkinson-Krankheit 2, Parkinson-Erkrankungen, experimentellem Parkinsonismus, juvenilem Parkinsonismus, infantiler Parkinsonismus-Dystonie, pädiatrischer Autoimmunerkrankung, Pendred-Syndrom, peripherer diabetischer Neuropathie, peripherer Neuropathie, Peritoneal-Neoplasie, Malignome des Peritoneums, Persönlichkeitsstörungen, Petit-Mal-Status, Petty-Laxova-Wiedemann-Syndrom, physiologischer Wundheilung, pigmentiertem Basalzellkarzinom, Pitt-Rogers-Danks-Syndrom, Vergiftung, polyzystischer Nierenerkrankung, Polycythämie, disseminierter oberflächlicher aktinischer Porokeratose Typ 8, disseminierter oberflächlicher aktinischer Porokeratose, Hinterstrangataxie mit Retinitis pigmentosa, Blutung in der hinteren Schädelgrube (posterior fossa), posttraumatischer Tic-Störung, präkanzerösen Zuständen, Vorläuferzell-lymphoblastischer Leukämie (Lymphom), prälingualer Taubheit, pränatalen Verletzungen, Missbrauch von verschreibungspflichtigen Medikamenten, präseniler Demenz, primärer biliärer Zirrhose, primärem Hypogonadismus, primärer Hypothyreose, primärer Mikrozephalie, schwerer geistiger Behinderung, progressiver Bulbärparalyse, autosomal-dominanter progressiver externer Ophthalmoplegie mit mitochondrialen DNA-Deletionen 1, autosomal-dominanter progressiver externer Ophthalmoplegie mit mitochondrialen DNA-Deletionen 2, Prostatakrebs, Prostata-Neoplasien, Pseudoaphakie, Pseudohyperkaliämie (Cardiff), Drogenpsychosen, substanzinduzierten Psychosen, Involutionspsychose, psychotischen Störungen, pulmonaler alveolärer Mikrolithiasis, pulmonaler Hypertonie, ausstrahlenden Schmerzen, Ramsay-Hunt-Lähmungssyndrom, rezidivierender Depression, regionaler Enteritis, renalem Carnitin-Transporter-Defekt, Nierenzellkarzinom, renaler Hypourikämie, renaler tubulärer Azidose, distaler renaler tubulärer Azidose mit hämolytischer Anämie (Störung), distaler renaler tubulärer Azidose mit normaler Erythrocytenmorphologie, proximaler renaler tubulärer Azidose mit Augenanomalien und geistiger Behinderung, renaler tubulärer Azidose Typ II, Reperfusionsschäden, Atemdepression, Adultem Atemnotsyndrom, Atemversagen, respiratorischer Überempfindlichkeit, respiratorischer Insuffizienz, Netzhautdegeneration, Retinitis pigmentosa, Retinitis pigmentosa 68, RH-Mangel-Syndrom, Rhabdomyolyse, Rhabdomyosarkom, Rhabdomyosarkom 1, rheumatoider Arthritis, Rhinolalie, RH-Null vom Regulator-Typ, Riboflavinmangel, Rotor-Syndrom, Roussy-Lévy-Syndrom (Störung), Speicheldrüsen-Neoplasien, Sarkoidose, sarkomatoidem Nierenzellkarzinom, Schizophrenie, katatoner Schizophrenie, Schneckenbecken-Dysplasie, Schwartz-Lelek-Syndrom, saisonal-affektiver Störung, sekundärer Hypothyreose, Krampfanfällen, auditiven epileptischen Anfällen, klonischen Anfällen, fokalen Anfällen, sensorischen Anfällen, somatosensorischen Anfällen, seniler paranoider Demenz, sensorineuralem Hörverlust (Störung), sensorischem Hörverlust, schwerer angeborener Mikrozephalie, schwerer Depression, schwerer Major Depression mit psychotischen Merkmalen, hämorrhagischem Schock, Kurzschläfer-Syndrom, Sialinsäure-Speicherkrankheit vom finnischen Typ (Störung), Sialurie, sideroblastischer Anämie, einfachem partiellem Status epilepticus, einzelnem epileptischem Anfall, Sinushistiozytose, Variation der Haut-Haar-Augen-Pigmentierung 4, Schlafstörungen, Schlafqualität, Schlaf-Wach-Störungen, schlafbezogene neurogene Tachypnoe, südostasiatischer Ovalozytose, autosomal-dominanter spastischer Paraplegie 42, autosomal-dominanter spastischer Paraplegie 6 (Störung), hereditärer spastischer Paraplegie, spastischer Tetraplegie mit dünnem Corpus callosum und progressiver Mikrozephalie, Sprachstörungen, Sphärozytose Typ 4, autosomal-rezessiver spinozerebellärer Ataxie 3, Ehlers-Danlos-Syndrom-ähnlicher Spondylocheirodysplasie, Plattenepithelkarzinom, Plattenepithelkarzinom der Speiseröhre, Status epilepticus, durch Alkoholentzug induziertem Status epilepticus, subklinischem Status epilepticus, Steatohepatitis, Magenkarzinom, Magen-Neoplasien, Stomatocytose I, Streptozotocin-Diabetes, Schlaganfall, Substanzmissbrauchsproblematik, Substanzabhängigkeit, Substanzgebrauchsstörungen, Substanzentzugssyndrom, substanzbedingte Störungen, Subvigilanz-Syndrom, plötzlichem Kindstod, körperlichem Leiden, taktiler Allodynie, testikulärer Mikrolithiasis, Thiamin-Stoffwechselstörungs-Syndrom 4 (Typ bilaterale striatale Degeneration und progressive Polyneuropathie), Thiamin-responsive megaloblastische Anämie-Syndrom, Magerkeit, Schilddrüsenagenesie, Schilddrüsenkarzinom, Schilddrüsen-Dyshormonogenese 1, follikulärem Schilddrüsenadenom, Schilddrüsenhormonresistenzsyndrom, Schilddrüsenhypoplasie, Schilddrüsen-Neoplasie, Tic-Störung, vokalen Tic-Störungen, Gewebefibrose, Anfälligkeit für Tabaksucht (Befund), tonisch-klonischen Anfällen, tonischen Anfällen, Zahnanomalien, vorübergehender Tic-Störung, rubralem Tremor, meiotischer Nondisjunktions-Trisomie 21, mitotischer Non-Disjunctions-Trisomie 21, Tuberkulose, Tumorkalzinose, Colitis ulcerosa, Epilepsie uncinatus, undifferenziertem Karzinom, einseitiger Hypotonie, unipolarer Depression, Harnsäurekonzentration im Serum-Quantitative-Trait-Locus 2, Harnsäurekonzentration im Serum-Quantitative-Trait-Locus 4, Urolithiasis, Gefäßerkrankungen, Gefäßhypertrophie, Störungen der verbalen Fluenz, Schwindelanfall, visuellem Anfall, Entzugserscheinungen, Wolf-Hirschhorn-Syndrom, Xerozytose, X-chromosomalem CSNB; X-chromosomalem Immundefekt mit Magnesiummangel, Epstein-Barr-Virusinfektion und Neoplasie; und neonatalem Zinkmangel aufgrund von niedrigem Zinkgehalt in der Muttermilch.

## Revendications

1. Composé ayant la formule (I) suivante : 
Fraction de liaison de
protéine
transmembranaire
(TMPBM)
Lieur (L)
Fraction de liaison de la
ligase E3 (EBM)
 dans lequel
TMPBM est une fraction qui se lie à une protéine transmembranaire,
L est une fraction de liaison ; et
EBM est une fraction modifiant la fonction de la ligase E3 et/ou se liant à au moins un membre du complexe de ligase E3,
dans lequel la TMPBM est une fraction ayant la formule partielle (II) suivante ou un stéréo-isomère, un tautomère, un sel pharmaceutiquement acceptable ou un solvate : 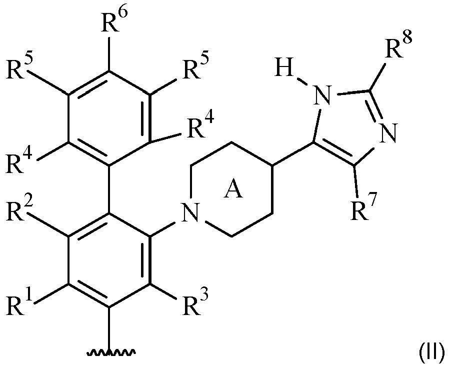 dans lequel
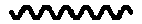 indique la position de l'attachement de la formule partielle (II) au lieur (L),
R¹, R², R³, R⁴, R⁵, R⁶, R⁷ et R⁸ sont chacun indépendamment choisis parmi -H, - halogène, - NO₂, -CN, alkyle en -C₁₋₃, -OH, -O-alkyle en C₁₋₂, NH₂, -NH(_{ALKYLE EN} C₁₋₂), -N(_{ALKYLE EN} C₁₋₂)₂, -CHO, -CO(alkyle en C₁₋₂), COOH, COO(alkyle en C₁₋₂), CONH₂, CONH(_{ALKYLE EN} C₁₋₂), et CON(-_{ALKYLE EN} C₁₋₂)₂, dans lequel chaque alkyle en C₁₋₂est facultativement substitué par un ou plusieurs F,
et le cycle A est facultativement substitué par un ou plusieurs F.

2. Composé selon la revendication 1, dans lequel les définitions suivantes s'appliquent à la fraction de formule partielle (II) :
R¹, R², R³ et chaque R⁴ sont chacun un hydrogène,
R⁷ est un méthyle,
R⁸ est H ou NH₂,
et le cycle A n'est substitué par aucun F.

3. Composé selon la revendication 1 ou 2, dans lequel les définitions suivantes s'appliquent à la fraction de formule partielle (II) :
un R⁵ est choisi parmi H, F, Cl, Me, CF₃, CF₂H et CH₂F,
l'autre R⁵ est H, et
R⁶ est F ou Cl.

4. Composé selon l'une quelconque des revendications 1 à 3, dans lequel la fraction de formule partielle (II) a la formule (IIa) suivante :

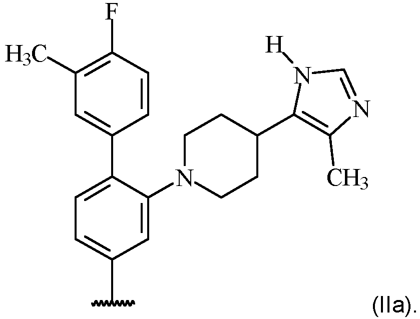

5. Composé selon l'une quelconque des revendications 1 à 4, dans lequel L est choisi parmi un alkylène en C₁₄₋₂₄, un alcénylène en C₁₄₋₂₄, et un alcynylène en C₁₄₋₂₄, dans lequel ledit alkylène, ledit alcénylène et ledit alcynylène sont chacun facultativement substitués par un ou plusieurs groupes choisis indépendamment parmi l'halogène, un haloalkyle en C₁₋₅, -O(_{HALOALKYLE EN} C₁₋₅), -CN, -OR²¹, -NR²¹R²¹, -NR²¹OR²¹, -COR²¹, - COOR²¹, -OCOR²¹, -CONR²¹R²¹, -NR²¹COR²¹, -NR²¹COOR²¹, -OCONR²¹R²¹, -SR²¹, - SOR²¹, -SO₂R²¹, -SO₂NR²¹R²¹, -NR²¹SO₂R²¹, -SO3R²¹ et -NO₂ et, en outre, dans lequel une ou plusieurs unités -CH₂- comprises dans ledit alkylène, ledit alcénylène ou ledit alcynylène sont chacune facultativement remplacées par un groupe choisi indépendamment parmi -O-, -NR²¹-, -CO-, -S-, -SO- et -SO₂- ;
chaque R²¹ est indépendamment choisi parmi l'hydrogène, un alkyle en C₁₋₅, un alcényle en C₂₋₅, un alcynyle en C₂₋₅, un carbocyclyle et un hétérocyclyle, dans lequel ledit alkyle, ledit alcényle et ledit alcynyle sont chacun facultativement substitués par un ou plusieurs groupes R^{Alk}, et dans lequel en outre ledit carbocyclyle et ledit hétérocyclyle sont chacun facultativement substitués par un ou plusieurs groupes R^{Cyc} ;
deux R²¹ quelconques sont facultativement liés pour former un cycle ;
chaque R^{Alk} est indépendamment choisi parmi -OH, -O(alkyle en C₁₋₅), -O(alkylène en C₁₋₅)-OH, -O(alkylène en C₁₋₅)-O(alkyle en C₁₋₅), -SH, -S(alkyle en C₁₋₅), -S(alkylène en C₁₋₅)-SH, -S(alkylène en C₁₋₅)-S(alkyle en C₁₋₅), -NH₂, -NH(_{ALKYLE EN} C₁₋₅), -N(_{ALKYLE EN} C₁₋₅)(alkyle en C₁₋₅), -NH-OH, -N(_{ALKYLE EN} C₁₋₅)-OH, -NH-O(alkyle en C₁₋₅), -N(alkyle en C₁₋₅)-O(alkyle en C₁₋₅), un halogène, un haloalkyle en C₁₋₅, -O(haloalkyle en C₁₋₅), - CN, -NO₂, -CHO, -CO(alkyle en C₁₋₅), -COOH, -COO(alkyle en C₁₋₅), -O-CO(alkyle en C₁₋₅), -CO-NH₂, -CO-NH(_{ALKYLE EN} C₁₋₅), -CO-N(_{ALKYLE EN} C₁₋₅)(alkyle en C₁₋₅), -NH-CO(_{ALKYLE EN} C₁₋₅), -N(_{ALKYLE EN} C₁₋₅)-CO(alkyle en C₁₋₅), -NH-COO(_{ALKYLE EN} C₁₋₅), - N(_{ALKYLE EN} C₁₋₅)-COO(alkyle en C₁₋₅), -O-CO-NH(_{ALKYLE EN} C₁₋₅), -O-CO-N(_{ALKYLE EN} C₁₋₅)(alkyle en C₁₋₅), -SO₂-NH₂, -SO₂-NH(_{ALKYLE EN} C₁₋₅), -SO₂-N(_{ALKYLE EN} C₁₋₅)(alkyle en C₁₋₅), -NH-SO₂-(_{ALKYLE EN} C₁₋₅), -N(_{ALKYLE EN} C₁₋₅)-SO₂-(alkyle en C₁₋₅), -SO₂-(alkyle en C₁₋₅), -SO-(alkyle en C₁₋₅), un aryle, un hétéroaryle, un cycloalkyle et un hétérocycloalkyle, dans lequel ledit aryle, ledit hétéroaryle, ledit cycloalkyle et ledit hétérocycloalkyle sont chacun facultativement substitués par un ou plusieurs groupes choisis indépendamment parmi un alkyle en C₁₋₅, un alcényle en C₂₋₅, un alcynyle en C₂₋₅, un halogène, un haloalkyle en C₁₋₅, -CN, -OH, -O(alkyle en C₁₋₅), -SH, -S(alkyle en C₁₋₅), -NH₂, -NH(_{ALKYLE EN} C₁₋₅), et -N(alkyle en C₁₋₅)(alkyle en C₁₋₅) ;
chaque R^{Cyc} est indépendamment choisi parmi un alkyle en C₁₋₅, un alcényle en C₂₋₅, un alcynyle en C₂₋₅, -OH, -O(alkyle en _{C1-5}), -O(alkylène en _{C1-5})-OH, -O(alkylène en _{C1-5})-O(alkyle en _{C1-5}), -SH, -S(alkyle en _{C1-5}), -S(alkylène en _{C1-5})-SH, -S(alkylène en _{C1-5})-S(alkyle en _{C1-5}), -NH₂, -NH(alkyle en _{C1-5}), -N(alkyle en _{C1-5})(alkyle en _{C1-5}), -NH-OH, - N(_{ALKYLE EN C1-5})-OH, - NH-O(_{ALKYLE EN} C₁₋₅), -N(_{ALKYLE EN} C₁₋₅)-O(alkyle en C₁₋₅), un halogène, un haloalkyle en C₁₋₅, -O(haloalkyle en C₁₋₅), -CN, -NO₂, -CHO, -CO(alkyle en C₁₋₅), -COOH, -COO(alkyle en C₁₋₅), -O-CO(alkyle C₁₋₅), -CO-NH₂, -CO-NH(_{ALKYLE EN} C₁₋₅), -CO-N(_{ALKYLE EN} C₁₋₅)(alkyle en C₁₋₅), -NH-CO(_{ALKYLE EN} C₁₋₅), -N(_{ALKYLE EN} C₁₋₅)-CO(alkyle en C₁₋₅), -NH-COO(_{ALKYLE EN} C₁₋₅), -N(_{ALKYLE EN} C₁₋₅)-COO(alkyle en C₁₋₅), -O-CO-NH(_{ALKYLE EN} C₁₋₅), -O-CO-N(_{ALKYLE EN} C₁₋₅)(alkyle en C₁₋₅), -SO₂-NH₂, - SO₂-NH(_{ALKYLE EN} C₁₋₅), -SO₂-N(_{ALKYLE EN} C₁₋₅)(alkyle en C₁₋₅), -NH-SO₂-(_{ALKYLE EN} C₁₋₅), -N(_{ALKYLE EN} C₁₋₅)-SO₂-(alkyle en C₁₋₅), -SO₂-(alkyle en C₁₋₅), -SO-(alkyle en C₁₋₅), un aryle, un hétéroaryle, un cycloalkyle et un hétérocycloalkyle, dans lequel ledit aryle, ledit hétéroaryle, ledit cycloalkyle et ledit hétérocycloalkyle sont chacun facultativement substitués par un ou plusieurs groupes choisis indépendamment parmi un alkyle en C₁₋₅, un alcényle en C₂₋₅, un alcynyle en C₂₋₅, un halogène, un haloalkyle en C₁₋₅, -CN, -OH, -O(alkyle en C₁₋₅), -SH, -S(alkyle en C₁₋₅), -NH₂, -NH(_{ALKYLE EN} C₁₋₅), et -N(alkyle en C₁₋₅)(alkyle en C₁₋₅).

6. Composé selon la revendication 5, dans lequel
(i) l'alkylène en C₁₄₋₂₄, l'alcénylène en C₁₄₋₂₄, et l'alcynylène en C₁₄₋₂₄ sont l'alkylène en C₁₅₋₂₂, l'alcénylène en C₁₅₋₂₂, et l'alcynylène en C₁₅₋₂₂, respectivement, de préférence l'alkylène en C₁₆₋₂₀, l'alcénylène en C₁₆₋₂₀, et l'alcynylène en C₁₆₋₂₀, respectivement, dans lequel chacun des alkylènes, alcénylènes et alcynylènes peuvent être modifiés comme indiqué dans la revendication 5 ;
(ii) L est sélectionné parmi un alkylène en C₁₄₋₂₄ et un alcénylène en C₁₄₋₂₄, plus préférentiellement un alkylène en C₁₅₋₂₂, un alcénylène en C₁₅₋₂₂, encore plus préférentiellement un alkylène en C₁₆₋₂₀, encore plus préférentiellement un alkylène en C₁₇₋₁₉, plus préférentiellement un alkylène en C₁₇, dans lequel chacun des alkylènes et alcénylènes peut être modifié comme indiqué dans la revendication 5 ;
(iii) L possède l'une des structures suivantes :
-O-L¹-NH-C(=O)- et -NH-L¹-NH-C(=O)-
dans lequel L¹ est choisi parmi un alkylène en C₁₃₋₁₇ et un alcénylène en C₁₃₋₁₇, dans lequel ledit alkylène et ledit alcénylène sont chacun facultativement substitués par un ou plusieurs groupes choisis indépendamment parmi un halogène, un alkyle en C₁₋₃ et un haloalkyle en C₁₋₃, et en outre dans lequel une ou plusieurs unités -CH₂- comprises dans ledit alkylène ou ledit alcénylène est/ sont remplacées par un groupe choisi indépendamment parmi -O-, -NR²¹-, -CO-, -S-, -SO-, et -SO₂- ; dans lequel il est préférable que le -O- ou -NH- du côté gauche se lie à l'EBM tel que défini dans l'un quelconque des points précédents et que le NH-C(=O)- du côté droit se lie à la TMPBM tel que définie dans l'un quelconque des points précédents ; ou
(iv) L a l'une des structures suivantes :
-O-L¹-NH-C(=O)- et -NH-L¹-NH-C(=O)-
dans lequel L¹ est choisi parmi un alkylène en C₁₃₋₁₇, dans lequel ledit alkylène est facultativement substitué par un ou plusieurs groupes choisis indépendamment parmi un halogène, un haloalkyle en C₁₋₃ et un haloalkyle en C₁₋₃, et en outre dans lequel une ou plusieurs unités -CH₂- comprises dans ledit alkylène ou ledit alcénylène est/sont remplacée(s) par un groupe choisi indépendamment parmi -O-, -NR²¹ et -CO- ; dans lequel il est préférable que le -O- ou -NH- du côté gauche se lie à l'EBM tel que défini dans l'un quelconque des points précédents et que le NH-C(=O)- du côté droit se lie à la TMPBM telle que définie dans l'un quelconque des points précédents.

7. Composé de formule (I) selon l'une quelconque des revendications précédentes, dans lequel l'EBM est une fraction de formule partielle (III) : 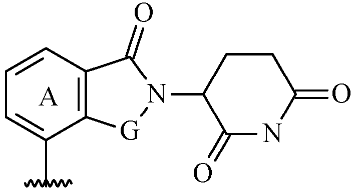 dans lequel
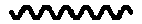 indique la position de l'attachement de la formule partielle (III) au lieur (L),
G est choisi parmi -C(=O)- et CH₂,
et le cycle A est facultativement substitué par un ou plusieurs F,
ou l'un quelconque de ses stéréo-isomères.

8. Composé selon la revendication 7, dans lequel, dans la formule partielle (III), G est - C(=O)- et le cycle A n'est pas substitué par un F.

9. Composition comprenant un composé selon l'une quelconque des revendications 1 à 8.

10. Composé selon l'une quelconque des revendications 1 à 8 ou composition selon la revendication 9 comprenant en outre un diluant, un excipient ou un support pharmaceutiquement acceptable.

11. Composé selon l'une quelconque des revendications 1 à 8 ou 10 ou composition selon la revendication 9 ou 10 pour une utilisation en tant que médicament.

12. Composé selon l'une quelconque des revendications 1 à 8, 10 ou 11, ou composition selon l'une quelconque des revendications 9 à 11, pour une utilisation dans le traitement ou la prévention du cancer, en particulier la leucémie telle que la leucémie myéloïde chronique.

13. Composé selon l'une quelconque des revendications 1 à 8, 10 ou 11, ou composition selon l'une quelconque des revendications 9 à 11 pour l'utilisation dans le traitement ou la prévention d'une maladie choisie parmi : le syndrome de microdélétion 2p21, la coordination anormale, l'anomalie de la cornée, les crises d'absence, la douleur, l'achondrogenèse de type ib (trouble), l'acidose, l'acrodermatite, l'acrodermatite entéropathique, les accidents vasculaires cérébraux aigus, la démence sénile confusionnelle aiguë, les lésions rénales aiguës, l'insuffisance rénale aiguë, les lésions pulmonaires aiguës, l'adénocarcinome du poumon (trouble), le carcinome adénoïde kystique, l'adénome, l'adénome basocellulaire, l'adénome microkystique, l'adénome monomorphe, l'adénome trabéculaire, le syndrome de Fanconi de l'adulte, les troubles de l'apprentissage chez l'adulte, la céroïde lipofuscinose neuronale de l'adulte, la citrullinémie de type 2 de l'adulte, la réaction indésirable au médicament, les troubles affectifs psychotiques, la dégénérescence maculaire liée à l'âge, les troubles de la mémoire liés à l'âge, la variante akinétique-rigide de la maladie de Huntington, l'albinisme oculaire, l'abus d'alcool, la sensibilité à l'alcool, les troubles liés à la consommation d'alcool, les crises liées au sevrage de l'alcool, la crise motrice majeure induite par le sevrage de l'alcool, l'intoxication alcoolique, l'intoxication alcoolique chronique, le syndrome d'Allan-Herndon-Dudley (ahds), l'allodynie, le diabète alloxanique, l'hémiplégie alternante de l'enfance, la maladie d'Alzheimer précoce, l'apparition tardive de la maladie d'Alzheimer, la maladie d'Alzheimer, la maladie d'Alzheimer à début focal, l'amélogenèse imparfaite, l'amélogenèse imparfaite de type hypomature, l'amélogenèse imparfaite de type iia5 hypomature, l'amélogenèse imparfaite de type iii, les erreurs innées du métabolisme des acides aminés, les troubles héréditaires du métabolisme des acides aminés, l'abus d'amphétamines, la dépendance aux amphétamines, les troubles liés aux amphétamines, la sclérose latérale amyotrophique, la sclérose latérale amyotrophique avec démence, la sclérose latérale amyotrophique de forme Guam, le carcinome anaplasique, l'anasarque, l'anémie, l'anémie hémolytique, l'anémie hémolytique acquise, l'anémie hémolytique acquise idiopathique, l'anémie hémolytique sphérocytaire héréditaire, l'anémie microcytaire hypochrome avec surcharge en fer, l'anémie microcytaire hypochrome avec surcharge en fer 1, l'anémie microangiopathique, l'anémie sidéroblastique réfractaire à la pyridoxine de type 2, l'anémie sidéroblastique autosomique récessive réfractaire à la pyridoxine, l'anhédonie, les néoplasmes mammaires chez l'animal, les troubles anxieux, la névrose d'angoisse (constat), les états d'anxiété névrotique, l'anévrisme de l'aorte thoracique, la calcification de la valve aortique, l'apnée, l'aprosodie, la tortuosité artérielle, le syndrome de tortuosité artérielle, l'arthrogrypose, l'arthrogrypose, le retard mental et les crises d'épilepsie, la personnalité « as if », le syndrome d'Asperger, l'ataxie, l'ataxie appendiculaire, l'ataxie motrice, l'ataxie sensorielle, l'ataxie tronculaire, les ataxies héréditaires, l'atélostéogenèse de type 2, l'athérogenèse, l'athérosclérose, les crises d'absence atoniques, l'atrophie, le trouble déficitaire de l'attention, le trouble déficitaire de l'attention avec hyperactivité, le trouble déficitaire de l'attention avec hyperactivité (TDAH), l'hyperplasie canalaire atypique du sein, l'aura, les troubles du spectre autistique, le trouble autistique, l'anémie hémolytique auto-immune, la maladie de Parkinson juvénile autosomique dominante, le syndrome parkinsonien autosomique dominant, le syndrome parkinsonien autosomique récessif, la polykystose rénale autosomique récessive, la microcéphalie primaire autosomique récessive, l'anémie sidéroblastique autosomique récessive, le trouble de la personnalité évitante, l'épilepsie du réveil, l'azoospermie non obstructive, l'épithélium de Barrett, l'œsophage de Barrett, la maladie de Bartter, le syndrome de Bartter de type 1 anténatal, le carcinome basocellulaire, la calcification idiopathique des ganglions de la base de type 6, la maladie des ganglions de la base répondant à la biotine, les maladies des ganglions de la base, le néoplasme bénin, la surdité bilatérale, l'insuffisance en acides biliaires-CoA ligase et le défaut d'amidation, la malabsorption primaire des acides biliaires, la cirrhose biliaire, la cirrhose biliaire secondaire, le trouble bipolaire, le trouble bipolaire de type i, le syndrome de Birk-Landau-Perez, le néoplasme de la vessie, la désintégration du blastocyste, le locus de caractères quantitatifs 11 de l'indice de masse corporelle, le locus de caractères quantitatifs 4 de l'indice de masse corporelle (trouble), les maladies osseuses, les maladies osseuses liées au développement, les maladies du cerveau, la maladie du transport vésiculaire de la dopamine et de la sérotonine dans le cerveau, l'hémorragie cérébrale, les lésions cérébrales, les lésions cérébrales focales, l'ischémie cérébrale, les lacérations cérébrales, le cancer du sein, le cancer du sein familial, le carcinome du sein, le diabète instable, l'adénocarcinome bronchioloalvéolaire, l'albinisme oculocutané brun, le syndrome de Brown-Vialetto-van Laere, le syndrome de Brown-Vialetto-van Laere 2, la brucellose, la brucellose pulmonaire, la paralysie bulbaire, les lésions multiples des organes induites par les brûlures, l'ulcère de Buruli, la calcinose, l'arthropathie au pyrophosphate de calcium, la maladie des dépôts de pyrophosphate de calcium, le cancer embryonnaire, le cancer embryonnaire et mixte, la consommation de cannabis, le syndrome des glycoprotéines déficientes en hydrates de carbone de type 2k, le carcinome, le carcinome du poumon, le carcinome à cellules fusiformes, la carcinomatose, l'arythmie cardiaque, l'hypertrophie cardiaque, la reperfusion ischémique cardiaque, la cardiomégalie, la cardiomyopathie dilatée, la cardiomyopathie idiopathique familiale, les maladies cardiovasculaires, la cardite, le déficit en carnitine-acylcarnitine translocase, la cataracte, la cataracte 47, la cataracte et la cardiomyopathie, la cataracte juvénile avec micro-cornée et glucosurie, la maladie coeliaque, l'hypothyroïdie centrale, les lésions cérébrales, l'ischémie cérébrale, l'accident vasculaire cérébral, les troubles cérébrovasculaires, la céroïde lipofuscinose neuronale 7, la céroïde lipofuscinose neuronale de type Parry, le déficit en céruloplasmine, le cancer du col de l'utérus, la maladie de Charcot-Marie-Tooth, la maladie de Charcot-Marie-Tooth de type ia (trouble), la maladie de Charcot-Marie-Tooth de type ib, les lésions hépatiques induites par des produits chimiques et des médicaments, la toxicité hépatique induite par des produits chimiques, les écarts en termes de développement de l'enfant, les troubles spécifiques du développement de l'enfant, la leucémie lymphoblastique aiguë de l'enfant, la paralysie bulbaire progressive de l'enfant, les troubles du tic chez l'enfant, le cholangiocarcinome, le kyste du cholédoque, le kyste du cholédoque de type i, le kyste du cholédoque de type ii, le kyste du cholédoque de type iii, le kyste du cholédoque de type iv, le kyste du cholédoque de type v, la cholestase, la cholestase chez le nouveau-né, la choréo-athétose/spasticité épisodique, le carcinome rénal chromophobe, la dépression chronique, le trouble chronique des tics moteurs ou vocaux, la cirrhose, la citrullinémie de type ii d'apparition néonatale, la fente palatine, la fente palatine isolée, la fente labiale supérieure, le bredouillement, l'abus de cocaïne, la dépendance à la cocaïne, les troubles de l'humeur induits par la cocaïne, les troubles liés à la cocaïne, les troubles de la cognition, les troubles cognitifs, la maladie de l'hémagglutinine froide, le carcinome du canal collecteur du rein, le fœtus collodion, le cancer du côlon, les néoplasmes coliques, le cancer colorectal, le carcinome colorectal, les néoplasmes colorectaux, l'acidurie combinée d-2- et l-2-hydroxyglutarique, le déficit combiné de la phosphorylation oxydative de type 18, le déficit combiné de la phosphorylation oxydative de type 28, la perte auditive complète, les crises partielles complexes, l'état de mal épileptique partiel complexe, l'état prénéoplasique, le trouble de la conduction cardiaque, le trouble synaptique congénital non progressif des cônes et des bâtonnets, l'anomalie congénitale, l'anémie congénitale, l'anévrisme congénital de l'aorte ascendante, les cataractes congénitales, la perte d'audition et la neurodégénérescence, la diarrhée congénitale au chlorure, le trouble congénital de la glycosylation de type 2c, le trouble congénital de la glycosylation de type iif, le trouble congénital de la glycosylation de type iim, le trouble congénital de la glycosylation de type iin, les troubles congénitaux de la glycosylation, la malabsorption congénitale du glucose et du galactose, les malformations cardiaques congénitales, l'hypothyroïdie congénitale, l'ichtyose congénitale, les syndromes myasthéniques congénitaux postsynaptiques, les syndromes myasthéniques congénitaux présynaptiques, la myopathie congénitale (trouble), l'érythrodermie ichtyosiforme congénitale non bulleuse, la diarrhée sécrétoire congénitale de type sodique (trouble), l'insuffisance cardiaque congestive, les maladies du tissu conjonctif, la constipation, le syndrome du côlon irritable à prédominance de constipation, la dermatite de contact, l'hypersensibilité de contact, le carcinome rénal conventionnel (à cellules claires), les convulsions, les crises convulsives, la dystrophie cornéenne et la surdité de perception, la dystrophie cornéenne endothéliale de Fuchs de stade 4, la dystrophie endothéliale cornéenne de stade 2, l'opacité cornéenne, la neuronopathie due à l'agénésie du corps calleux, la dysplasie corticale, les infections à virus Coxsackie, les anomalies craniofaciales, la dysplasie craniométaphysaire autosomique dominante, la cranio-ostéoarthropathie, la craniosynostose, la déficience en créatine liée au chromosome X, la maladie de Crohn, la maladie de Crohn du gros intestin, la maladie de Crohn de l'iléon, la cryohydrocytose déficiente en stomatine avec retard mental, les crises d'épilepsie, les cataractes, et l'hépatosplénomégalie massive, la mucoviscidose, le modificateur de la mucoviscidose 1, la cystinurie, la cystinurie de type a, la cystinurie de type a-b, la cystinurie de type b, la cytopénie, la dysplasie type de la Chapelle, le syndrome de Toni-Debre-Fanconi, le mutisme sourd, la surdité, la surdité acquise, la surdité autosomique dominante 25 (trouble), la surdité autosomique dominante 72, la surdité autosomique récessive 4 avec aqueduc vestibulaire élargi, la surdité autosomique récessive 61, le déficit en glutamate décarboxylase, la polyarthrite dégénérative, le délire, la démence, l'humeur dépressive, la dépression, la dépression bipolaire, la dépression névrotique, la dépression post-partum, le trouble dépressif, le trouble dépressif résistant au traitement, les symptômes dépressifs, le syndrome dépressif, la dermatite allergique de contact, la dermatite atopique, le trouble du développement scolaire, les troubles du développement, le diabète sucré, le diabète sucré expérimental, le diabète sucré insulinodépendant, le diabète sucré à tendance cétosique, le diabète sucré non insulinodépendant, le diabète sucré de type 2, le diabète auto-immun, les cardiomyopathies diabétiques, la cataracte diabétique, la diarrhée, la dysplasie diastrophique, l'aminoacidurie dicarboxylique, l'exacerbation de la maladie, l'acidose tubulaire rénale distale, le syndrome de Down, le syndrome de Down de trisomie 21 partielle, l'abus de drogues, la toxicomanie, l'accoutumance aux drogues, la toxicité des médicaments, les troubles liés à la consommation de drogues, les symptômes de sevrage de la drogue, les lésions hépatiques aiguës induites par les médicaments, l'état dépressif induit par les médicaments, les maladies du foie induites par les médicaments, le carcinome canalaire, la dysarthrie, la dysarthrie flasque, la dysarthrie gutturale, la dysarthrie mixte, la dysarthrie scandée, la dysarthrie spastique, la dysglossie, la dyslalie, la dysostéosclérose, l'humeur dysphorique, le trouble dysthymique, la dystonie, la dystonie 18 (trouble), la dystonie diurne, la dystonie des membres, la dystonie paroxystique, les maladies de l'oreille, l'encéphalopathie épileptique infantile précoce avec salves de suppression, l'encéphalopathie myoclonique précoce, l'eczéma infantile, l'œdème, le syndrome d'Ehlers-Danlos, le syndrome d'Ehlers-Danlos de type iv, l'elliptocytose 4, l'elliptocytose héréditaire, la désintégration de l'embryon, la perte d'embryons, le néoplasme embryonnaire, le rhabdomyosarcome embryonnaire, la vasculopathie proliférative encéphaloclastique, les encéphalopathies, la dépression endogène, l'endométriome, l'endométriose, les infections à entérovirus, l'épilepsie, l'épilepsie fronto-polaire antérieure, l'épilepsie bénigne psychomotrice de l'enfant, l'absence épileptique de l'enfant, l'épilepsie cingulaire, l'épilepsie cryptogénique, l'épilepsie du lobe frontal, la susceptibilité à l'épilepsie généralisée idiopathique 12, la susceptibilité à l'épilepsie généralisée idiopathique 14, l'épilepsie temporale latérale, l'épilepsie operculaire, l'épilepsie orbito-frontale, l'épilepsie dépendante de la pyridoxine, l'épilepsie motrice supplémentaire, l'épilepsie du lobe temporal, la crise de goutte épileptique, l'encéphalopathie épileptique, l'encéphalopathie épileptique infantile précoce 25, l'encéphalopathie épileptique infantile précoce 34, l'encéphalopathie épileptique infantile précoce 41, les crises d'épilepsie, la dysplasie épiphysaire multiple 4, l'ataxie épisodique de type 6 (trouble), le défaut du transporteur de lactate érythrocytaire, l'érythrocytose, les néoplasmes de l'oesophage, l'état marbré, l'intolérance à l'effort répondant à la riboflavine, l'hépatome expérimental, l'ophtalmoplégie externe, le cholangiocarcinome extra-hépatique, les troubles extrapyramidaux, l'hémolyse extravasculaire, le syndrome de Fahr (trouble), la maladie d'Alzheimer familiale (MAF), le déficit familial en apocéruloplasmine, la démence familiale, le syndrome parkinsonien juvénile familial, la maladie de Ménière familiale, la glycosurie rénale familiale, le syndrome de Fanconi, le syndrome de Fanconi-Bickel, la stéatose hépatique, le syndrome de Fazio-Londe, la fibrose, la fibrose du foie, le tonus musculaire flasque, les muscles flasques, la malabsorption héréditaire des folates, l'adénome folliculaire, l'allergie alimentaire, l'hypoplasie fovéale 2, l'hypoplasie fovéale et la dysgénésie du segment antérieur, le syndrome de Fowler, la fracture, la fracture en spirale, l'épilepsie frontale bénigne de l'enfant, la dystrophie endothéliale de Fuchs, le cancer de l'estomac, le dysfonctionnement gastro-intestinal, les néoplasmes gastro-intestinaux, le sarcome stromal gastro-intestinal, les tumeurs stromales gastro-intestinales, les crises d'absence généralisées, le psoriasis pustuleux généralisé, les crises d'épilepsie généralisées, le cancer germinal, la tumeur germinale, le syndrome de Gianotti-Crosti, le syndrome de Gitelman, le glaucome, le gliome, le taux de filtration glomérulaire, la glomérulosclérose, le défaut de transport du glucose-6-phosphate, le syndrome du déficit en glut1, le syndrome autosomique récessif de déficit en glut1, l'encéphalopathie glycinique avec un taux de glycine sérique normal, le maladie de stockage du glycogène ic, la maladie de stockage du glycogène de type id, la glycosurie rénale, le goitre, le syndrome de Gorlin-Chaudhry-Moss, la goutte, la susceptibilité à la goutte 2, la crise épileptique convulsive généralisée continue la crise gustative, le fœtus arlequin, la maladie de Hartnup, le carcinome épidermoïde de la tête et du cou, la déficience auditive, la perte auditive extrême, la décompensation cardiaque, les maladies cardiaques, l'insuffisance cardiaque, l'insuffisance cardiaque droite, la maladie hématologique, l'hémochromatose, l'hémochromatose de type 4, l'hémolyse (trouble), la stéatose hépatique non alcoolique, l'hépatite b, l'hépatite induite par les médicaments, l'hépatite toxique, l'hépatome de Morris, l'hépatome de Novikoff, l'hépatomégalie, le carcinome rénal héréditaire à cellules claires, l'hippocratisme héréditaire, le cancer gastrique diffus héréditaire, l'hémochromatose héréditaire, l'hyperexplexie héréditaire, la neuropathie motrice et sensorielle héréditaire de type i, la neuropathie motrice et sensorielle héréditaire avec atrophie optique (trouble), la neuropathie motrice et sensorielle héréditaire de type ii, la sphérocytose héréditaire, l'encéphalite herpétique, l'encéphalite herpétique nécrosante aiguë, la méningo-encéphalite herpétique, le syndrome HHH, l'histiocytose, l'histiocytose avec contractures articulaires et surdité neurosensorielle, la co-infection par le vih, les infections par le vih, la maladie de Huntington, la maladie de Huntington à début tardif, l'hydrocéphalie, l'anasarque fœto-placentaire, le comportement hyperactif, l'hyperalgésie, l'hyperalgésie primaire, l'hyperalgésie secondaire, l'hyperalgésie thermique, l'hyperammoniémie, l'hyperammonémie, l'hypercalcémie, l'hypercalcémie idiopathique de l'enfance, l'hypercalcémie infantile, l'hypercalcémie infantile 1, l'hypercalcémie infantile 2, l'hypercalciurie, l'hypercholestérolémie familiale, l'hyperekplexie 3, l'hyperglycinurie (trouble), l'hyperhomocystéinémie, l'hypoglycémie hyperinsulinémique familiale 7, l'hyperinsulinisme dû à un déficit en protéine découplante 2, l'hyperkinésie généralisée, l'hypermanganésémie avec dystonie 2, l'hypermanganésémie avec dystonie polycythémie et cirrhose, l'hyperoxalurie, l'hyperphosphatémie, l'hyperpigmentation, l'hypertension, l'hypertrichose, la cardiomyopathie hypertrophique, l'ostéoarthropathie hypertrophique primaire autosomique dominante, l'ostéoarthropathie hypertrophique primaire autosomique récessive 2, l'hyperuricémie, l'hypogonadisme, l'hypogonadisme hypogonadotrope isolé, l'hypogonadisme hypogonadotrope, l'hypomyélinisation cérébrale globale, l'hypophosphatémie, le rachitisme hypophosphatémique héréditaire avec hypercalciurie, l'hypotension, l'hypothyroïdie, le syndrome d'hypotonie-cystinurie, l'hypouricémie rénale 2, l'ichtyose congénitale ii, le syndrome ichtyose-prématurité, l'ichtyose congénitale autosomique récessive 6, l'anémie hémolytique auto-immune idiopathique, l'épilepsie généralisée idiopathique, l'hypogonadisme hypogonadotrope idiopathique, la glomérulonéphrite à IgA, l'iléocolite, l'iminoglycinurie, l'épilepsie liée à l'immersion, l'intolérance au glucose, la personnalité impulsive, la personnalité inadéquate, les erreurs innées du métabolisme, la maladie de stockage de l'acide sialique libre infantile, la céroïde lipofuscinose neuronale infantile, le nystagmus infantile, la maladie infantile de stockage de l'acide sialique, l'inflammation, la maladie inflammatoire de l'intestin, les maladies inflammatoires de l'intestin, les réponses inflammatoires dans l'ischémie cérébrale et les lésions cérébrales traumatiques, la grippe, les lésions cérébrales aiguës, la résistance à l'insuline, la susceptibilité à la résistance à l'insuline, la sensibilité à l'insuline, le trouble du développement intellectuel avec caractéristiques neuropsychiatriques, la déficience intellectuelle, l'ophtalmoplégie interne, l'obstruction intestinale, les hémorragies intracrâniennes, l'hypertension intracrânienne, le cholangiocarcinome intrahépatique, l'hémolyse intravasculaire, la dépression involutive, la paraphrénie involutive, l'anémie ferriprive, la surcharge en fer, le coeur irritable, la crise jacksonienne, l'arthrite juvénile, la maladie de Huntington juvénile, la céroïde lipofuscinose neuronale juvénile, l'arthrite psoriasique juvénile, la maladie de Still à début juvénile, l'hypoglycémie cétosique, les calculs rénaux, les maladies rénales, l'insuffisance rénale aiguë, les lésions rénales, le néoplasme rénal, le syndrome de Kohlschutter Tonz, la déformation de la colonne vertébrale en cyphose, l'arythmogénèse des veines pulmonaires induite par l'acidose lactique, la septicémie induite par l'acidose lactique, la céroïde lipfuscinose neuronale infantile tardive, les troubles de l'apprentissage, les troubles d'apprentissage, le trouble des apprentissages, l'insuffisance cardiaque gauche, la leishmaniose viscérale, les opacités du cristallin, les troubles lenticulo-striés, la lèpre, la leucémie, leucémie lymphocytaire aiguë l2, la leucodystrophie, la maladie de Libman-Sacks, le syndrome de Lichtenstein-Knorr, la lipoïdose, le cancer du foie, le carcinome du foie, la cirrhose du foie, la cirrhose du foie expérimentale, les maladies du foie, le dysfonctionnement du foie, les néoplasmes du foie, les néoplasmes hépatiques expérimentaux, le syndrome du gros dormeur, le glaucome à basse tension, le cancer du poumon les néoplasmes pulmonaires, le lupus érythémateux disséminé, l'intolérance aux protéines avec lysinurie, la dystrophie maculaire avec atteinte du cône central, le trouble dépressif majeur, le syndrome de malabsorption, le paludisme, les malformations du développement cortical, le gliome malin, le mésothéliome malin, le néoplasme malin du sein, le néoplasme malin de l'oesophage, le néoplasme malin du rein, le néoplasme malin du foie, le néoplasme malin du poumon, le néoplasme malin de l'ovaire, le néoplasme malin du pancréas, le néoplasme malin de la prostate, le néoplasme malin de la glande salivaire, le néoplasme malin de l'estomac, le néoplasme malin de la vessie, les néoplasmes malins, la tumeur maligne du côlon, le carcinome mammaire chez l'animal, les néoplasmes mammaires, les néoplasmes mammaires expérimentaux, les néoplasmes mammaires humains, l'empoisonnement au manganèse, la manie, le trouble maniaque, le syndrome de Marfan, l'abus de marijuana, l'allodynie mécanique, la susceptibilité à l'iléus méconial dans la mucoviscidose, le mégacôlon, la mélancolie, le mélanome, le trouble de la mémoire sémantique, le trouble de la mémoire spatiale, les troubles de la mémoire, les troubles mnésiques, la perte de mémoire, la méningite à méningocoques du sérogroupe a, la méningite à méningocoques du sérogroupe b, la méningite à méningocoques du sérogroupe c, la méningite à méningocoques du sérogroupe w-135, la méningite à méningocoques du sérogroupe y, la méningite à méningocoques, la déficience mentale, la dépression mentale, le retard mental de type sud-africain lié au chromosome X, le retard mental autosomique récessif 48, le retard mental autosomique récessif 7, le retard mental psychosocial, le retard mental lié au chromosome X, le retard mental syndromique de type Christianson lié au chromosome X, le mésothéliome, l'acidose métabolique, le trouble métabolique des os, l'anémie hémolytique microangiopathique, la microcalcification, la microcéphalie, la microcéphalie primaire autosomique récessive 15, la microcéphalie de type amish (trouble), la microlissencéphalie, le syndrome du lait et des alcalins, le dysfonctionnement cérébral minime, les maladies mitochondriales, le syndrome autosomique dominant de déplétion de l'adn mitochondrial 12a (type cardiomyopathique), le syndrome autosomique dominant de déplétion de l'adn mitochondrial 12b (type cardiomyopathique), le déficit en transporteur mitochondrial du phosphate, le déficit en transporteur mitochondrial du pyruvate, le syndrome du prolapsus de la valve mitrale, les gliomes mixtes, le déficit du transporteur du monocarboxylate 1, les troubles de l'humeur, les troubles du tic moteur, les troubles du mouvement, la dysplasie épiphysaire multiple, la sclérose en plaques, la sclérose en plaques aiguë foudroyante, l'hypotonie musculaire, le tonus musculaire atonique, le syndrome myasthénique congénital présynaptique 20, le syndrome myasthénique congénital présynaptique 21, les syndromes myasthéniques congénitaux, les syndromes myasthéniques congénitaux à canal lent, la protection contre mycobacterium tuberculosis, la sensibilité à (découverte de) mycobacterium tuberculosis, l'insuffisance myocardique, l'infarctus du myocarde, l'ischémie myocardique, les lésions de reperfusion du myocarde, la myocardite, l'épilepsie myoclono-astatique, les crises myocloniques, l'épilepsie myoclono-atonique, la myopathie, la myopie autosomique dominante 24, na, la maladie de stockage de l'acide n-acétylneuraminique, le trouble de la personnalité narcissique, la nécrose, l'hypotonie néonatale, la capacité d'envahissement des néoplasmes, les métastases des néoplasmes, les néoplasmes, les néoplasmes embryonnaires et mixtes, les néoplasmes germinaux et embryonnaires, la transformation des cellules néoplasiques, la néphrocalcinose, la néphrolithiase d'oxalate de calcium, la néphrolithiase hypophosphatémique-ostéoporose 1, la dégénérescence nerveuse, le trouble du système nerveux, l'asthénie neurocirculatoire, les troubles du développement neurologique, les céroïdes-lipofuscinoses neuronales, la douleur neuropathique, la neuropathie motrice héréditaire distale de type viia, la neuropathie motrice et sensorielle héréditaire de type vib, la maladie de Niemann-Pick de la Nouvelle-Ecosse, la maladie de Niemann-Pick de type c, la maladie de Niemann-Pick de type c1, la maladie de Niemann-Pick de type d, la cécité nocturne stationnaire congénitale, la cécité nocturne stationnaire congénitale de type 1, la cécité nocturne stationnaire congénitale de type 1a, la cécité nocturne stationnaire congénitale de type 1b, la cécité nocturne stationnaire congénitale de type 2a, la cécité nocturne stationnaire congénitale de type 2b (trouble), l'épilepsie non convulsive, la convulsion non épileptique, les crises non épileptiques, le carcinome intra-canalaire non infiltrant, la psychose non organique, le carcinome pulmonaire non à petites cellules, l'obésité, les troubles obsessionnels compulsifs, l'albinisme oculocutané de type 2, l'albinisme oculocutané de type 6, l'albinisme oculocutané de type iv, l'odontome, la crise olfactive, l'ophtalmoparésie, l'ophtalmoplégie, l'atrophie optique, les troubles mentaux organiques induits par une substance, l'intolérance orthostatique, l'ostéoarthropathie hypertrophique primaire, l'arthrose déformante, l'ostéogenèse imparfaite, l'ostéopénie, l'ovalocytose de Malaisie-Mélanésie-Philippines, le cancer de l'ovaire, le néoplasme de l'ovaire, l'oxalose, la douleur, la douleur cuisante, la douleur écrasante, la douleur migratoire, la douleur foudroyante, le cancer du pancréas, le néoplasme du pancréas, la pancytopénie, l'adénome papillaire, le carcinome papillaire à cellules rénales, la paranoïa, la maladie de Parkinson, la maladie de parkinson juvénile autosomique récessive 2, les troubles parkinsoniens, le syndrome parkinsonien expérimental, le syndrome parkinsonien juvénile, le syndrome parkinsonien infantile-dystonie, les maladies auto-immunes pédiatriques, le syndrome de Pendred, la neuropathie diabétique périphérique, la neuropathie périphérique, les néoplasmes péritonéaux, la tumeur maligne de la surface péritonéale, les troubles de la personnalité, l'état d'absence, le syndrome de Petty Laxova Wiedemann, la cicatrisation physiologique, le carcinome basocellulaire pigmenté, le syndrome de Pitt-Rogers-Danks, l'empoisonnement, la polykystose rénale, la polyglobulie, la porokératose actinique disséminée superficielle 8, la porokératose actinique disséminée superficielle, l'ataxie de la colonne postérieure avec rétinite pigmentaire, l'hémorragie de la fosse postérieure, le trouble du tic post-traumatique, les affections précancéreuses, le lymphome leucémique lymphoblastique à précurseurs, la surdité prélinguistique, les lésions prénatales, l'abus de médicaments sur ordonnance, la démence présénile, la cirrhose biliaire primitive, l'hypogonadisme primaire, l'hypothyroïdie primaire, la microcéphalie primaire, le retard mental profond, la paralysie bulbaire progressive, l'ophtalmoplégie externe progressive autosomique dominante avec délétions de l'ADN mitochondrial 1, l'ophtalmoplégie externe progressive autosomique dominante avec délétions de l'ADN mitochondrial 2, le cancer de la prostate, les néoplasmes prostatiques, la pseudo-aphakie, la pseudo-hyperkaliémie Cardiff, les psychoses médicamenteuses, les psychoses induites par une substance, la psychose involutive, les troubles psychotiques, la microlithiase alvéolaire pulmonaire, l'hypertension pulmonaire, la douleur irradiante, le syndrome de paralysie de Ramsay Hunt, la dépression récurrente, l'entérite régionale, le défaut de transport rénal de la carnitine, le carcinome à cellules rénales, l'hypouricémie rénale, l'acidose tubulaire rénale, l'acidose tubulaire rénale distale avec anémie hémolytique (trouble), l'acidose tubulaire rénale distale avec morphologie normale des globules rouges, l'acidose tubulaire rénale proximale avec anomalies oculaires et retard mental, l'acidose tubulaire rénale de type ii, les lésions de reperfusion, la dépression respiratoire, le syndrome de détresse respiratoire de l'adulte, l'insuffisance respiratoire, l'hypersensibilité respiratoire, l'insuffisance respiratoire, la dégénérescence rétinienne, la rétinite pigmentaire, la rétinite pigmentaire 68, le syndrome de déficit Rh, la rhabdomyolyse, le rhabdomyosarcome, le rhabdomyosarcome 1, la polyarthrite rhumatoïde, la rhinolalie, le Rh-null de type régulateur, la carence en riboflavine, le syndrome de Rotor, le syndrome de Roussy-Levy (trouble), les néoplasmes des glandes salivaires, la sarcoïdose, le carcinome rénal sarcomatoïde, la schizophrénie, la schizophrénie catatonique, la dysplasie de Schneckenbecken, le syndrome de Schwartz-Lelek, le trouble affectif saisonnier, l'hypothyroïdie secondaire, les crises d'épilepsie, les crises auditives, les crises cloniques, les crises focales, les crises sensorielles, les crises somatosensorielles, la démence sénile et paranoïaque, la surdité de perception (trouble), perte auditive sensorielle, la microcéphalie congénitale sévère, la dépression sévère, la dépression majeure sévère avec caractéristiques psychotiques, le choc hémorragique, le syndrome du petit dormeur, la maladie de stockage de l'acide sialique de type finlandais (trouble), la sialurie, l'anémie sidéroblastique, l'état de mal épileptique partiel simple, la crise épileptique unique, l'histiocytose sinusale, la variation de la pigmentation de la peau, des cheveux et des yeux de type 4, les troubles du sommeil, la qualité du sommeil, les troubles du sommeil et de la veille, la tachypnée neurogène liée au sommeil, l'ovalocytose de l'Asie du Sud-Est, la paraplégie spastique autosomique dominante 42, la paraplégie spastique autosomique dominante (trouble) 6, la paraplégie spastique héréditaire, la tétraplégie spastique du corps calleux fin et microcéphalie progressive, les troubles de la parole, la sphérocytose de type 4, l'ataxie spinocérébelleuse autosomique récessive 3, la spondylochéirodysplasie de type syndrome d'Ehlers-Danlos, le carcinome épidermoïde, carcinome épidermoïde de l'oesophage, l'état de mal épileptique, l'état de mal épileptique induit par le sevrage de l'alcool, l'état épileptique subclinique, la stéatose hépatique, le carcinome de l'estomac, les néoplasmes de l'estomac, la stomatocytose i, le diabète induit par la streptozotocine, l'AVC, le problème de toxicomanie, la dépendance aux drogues, les troubles liés à l'utilisation de drogues, le syndrome de sevrage d'une drogue, les troubles liés aux drogues, la somnolence, le syndrome de la mort subite du nourrisson, la souffrance physique, l'allodynie tactile, la microlithiase testiculaire, le syndrome de dysfonctionnement du métabolisme de la thiamine de type 4 (dégénérescence striatale bilatérale et polyneuropathie progressive), le syndrome d'anémie mégaloblastique répondant à la thiamine, la minceur, l'agénésie de la thyroïde, le carcinome thyroïdien, la dyshormonogenèse thyroïdienne 1, l'adénome folliculaire de la glande thyroïde, le syndrome de résistance aux hormones thyroïdiennes, l'hypoplasie de la thyroïde, le néoplasme de la thyroïde, le trouble des tics, les troubles du tic vocal, la fibrose des tissus, la susceptibilité à (découverte de) la dépendance au tabac, les crises tonico-cloniques, les crises toniques, les anomalies dentaires, le trouble du tic transitoire, le tremblement rurale, la trisomie 21 de non-disjonction méiotique, la trisomie 21 de non-disjonction mitotique, la tuberculose, la calcinose tumorale, la colite ulcéreuse, l'épilepsie uncinée, le carcinome indifférencié, l'hypotonie unilatérale, la dépression unipolaire, locus de caractères quantitatifs du sérum de la concentration en acide urique 2, locus de caractères quantitatifs du sérum de la concentration en acide urique 4, l'urolithiase, les maladies vasculaires, l'hypertrophie vasculaire, les troubles de la fluidité verbale, la crise vertigineuse, la crise visuelle, les symptômes de sevrage, le syndrome de Wolf-Hirschhorn, la xérocytose, la CNSC liée au chromosome X, l'immunodéficience liée à l'X avec infection et néoplasie liées au virus d'Epstein-Barr et carence en magnésium et carence néonatale en zinc due à une faible teneur en zinc du lait maternel.
